(19) Europäisches Patentamt
European Patent Office
Office européen des brevets

(11) **EP 2 997 154 B1**

(12) **EUROPEAN PATENT SPECIFICATION**

(45) Date of publication and mention of the grant of the patent:
**11.07.2018 Bulletin 2018/28**

(21) Application number: **14797735.9**

(22) Date of filing: **15.05.2014**

(51) Int Cl.:
***C12P 19/02*** *(2006.01)*

(86) International application number:
**PCT/US2014/038184**

(87) International publication number:
**WO 2014/186567 (20.11.2014 Gazette 2014/47)**

(54) **ENHANCING ENZYMATIC HYDROLYSIS BY ENZYMATIC PRECONDITIONING**

ERHÖHUNG DER ENZYMATISCHEN HYDROLYSE DURCH ENZYMATISCHE VORKONDITIONIERUNG

AMELIORATION DE L'HYDROLYSE ENZYMATIQUE PAR PRECONDITIONNEMENT ENZYMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2013 US 201361824082 P**

(43) Date of publication of application:
**23.03.2016 Bulletin 2016/12**

(73) Proprietor: **Novozymes A/S**
**2880 Bagsvaerd (DK)**

(72) Inventors:
- **LI, Xin**
  **Raleigh, North Carolina 27614 (US)**
- **PUTNAM, Lorraine**
  **Youngsville, North Carolina 27596 (US)**
- **EMME, Brandon**
  **Kansas City, MO 64155-3218 (US)**
- **SMITH, Mads Torry**
  **Raleigh, North Carolina 27615 (US)**

(74) Representative: **NZ EPO Representatives**
**Krogshoejvej 36**
**2880 Bagsvaerd (DK)**

(56) References cited:
**WO-A1-2008/134259**
**US-A1- 2010 159 509**
**US-A1- 2012 094 358**
**US-A1- 2012 159 839**
**US-B2- 8 163 976**

- **XIMENES E ET AL: "Inhibition of cellulases by phenols", ENZYME AND MICROBIAL TECHNOLOGY, STONEHAM, MA, US, vol. 46, no. 3-4, 5 March 2010 (2010-03-05), pages 170-176, XP026862189, ISSN: 0141-0229 [retrieved on 2009-11-11]**
- **STERNBERG D ET AL: "beta-Glucosidase: microbial production and effect on enzymatic hydrolysis of cellulose", CANADIAN JOURNAL OF MICROBIOLOGY, NRC RESEARCH PRESS, CA, vol. 23, no. 2, 1 February 1977 (1977-02-01), pages 139-147, XP002564234, ISSN: 0008-4166, DOI: 10.1139/M77-020**

EP 2 997 154 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Novozymes A/S Li, Xin Lorraine, Putnam Emme, Brandon Cory Smith, Mads Torry

<120> ENHANCING ENZYMATIC HYDROLYSIS OF DILUTE ACID PRETREATED CORN FIBER BY ENZYMATIC PRECONDITIONING

<130> 12599-WO-PCT

<150> US 61/824,082
<151> 2013-05-16

<160> 9

<170> PatentIn version 3.5

<210> 1
<211> 253
<212> PRT
<213> Penicillium emersonii

<400> 1

```
Met Leu Ser Ser Thr Thr Arg Thr Leu Ala Phe Thr Gly Leu Ala Gly
1               5                   10                  15

Leu Leu Ser Ala Pro Leu Val Lys Ala His Gly Phe Val Gln Gly Ile
            20                  25                  30

Val Ile Gly Asp Gln Phe Tyr Ser Gly Tyr Ile Val Asn Ser Phe Pro
        35                  40                  45

Tyr Glu Ser Asn Pro Pro Pro Val Ile Gly Trp Ala Thr Thr Ala Thr
    50                  55                  60

Asp Leu Gly Phe Val Asp Gly Thr Gly Tyr Gln Gly Pro Asp Ile Ile
65                  70                  75                  80

Cys His Arg Asn Ala Thr Pro Ala Pro Leu Thr Ala Pro Val Ala Ala
                85                  90                  95

Gly Gly Thr Val Glu Leu Gln Trp Thr Pro Trp Pro Asp Ser His His
            100                 105                 110

Gly Pro Val Ile Thr Tyr Leu Ala Pro Cys Asn Gly Asn Cys Ser Thr
        115                 120                 125

Val Asp Lys Thr Thr Leu Glu Phe Phe Lys Ile Asp Gln Gln Gly Leu
    130                 135                 140
```

Ile Asp Asp Thr Ser Pro Pro Gly Thr Trp Ala Ser Asp Asn Leu Ile
145 150 155 160

Ala Asn Asn Asn Ser Trp Thr Val Thr Ile Pro Asn Ser Val Ala Pro
165 170 175

Gly Asn Tyr Val Leu Arg His Glu Ile Ile Ala Leu His Ser Ala Asn
180 185 190

Asn Lys Asp Gly Ala Gln Asn Tyr Pro Gln Cys Ile Asn Ile Glu Val
195 200 205

Thr Gly Gly Gly Ser Asp Ala Pro Glu Gly Thr Leu Gly Glu Asp Leu
210 215 220

Tyr His Asp Thr Asp Pro Gly Ile Leu Val Asp Ile Tyr Glu Pro Ile
225 230 235 240

Ala Thr Tyr Thr Ile Pro Gly Pro Pro Glu Pro Thr Phe
245 250

<210> 2
<211> 863
<212> PRT
<213> Aspergillus fumigatus

<400> 2

Met Arg Phe Gly Trp Leu Glu Val Ala Ala Leu Thr Ala Ala Ser Val
1 5 10 15

Ala Asn Ala Gln Glu Leu Ala Phe Ser Pro Pro Phe Tyr Pro Ser Pro
20 25 30

Trp Ala Asp Gly Gln Gly Glu Trp Ala Asp Ala His Arg Arg Ala Val
35 40 45

Glu Ile Val Ser Gln Met Thr Leu Ala Glu Lys Val Asn Leu Thr Thr
50 55 60

Gly Thr Gly Trp Glu Met Asp Arg Cys Val Gly Gln Thr Gly Ser Val
65 70 75 80

Pro Arg Leu Gly Ile Asn Trp Gly Leu Cys Gly Gln Asp Ser Pro Leu
85 90 95

Gly Ile Arg Phe Ser Asp Leu Asn Ser Ala Phe Pro Ala Gly Thr Asn
100 105 110

```
Val Ala Ala Thr Trp Asp Lys Thr Leu Ala Tyr Leu Arg Gly Lys Ala
        115                 120                 125

Met Gly Glu Glu Phe Asn Asp Lys Gly Val Asp Ile Leu Leu Gly Pro
    130                 135                 140

Ala Ala Gly Pro Leu Gly Lys Tyr Pro Asp Gly Gly Arg Ile Trp Glu
145                 150                 155                 160

Gly Phe Ser Pro Asp Pro Val Leu Thr Gly Val Leu Phe Ala Glu Thr
                165                 170                 175

Ile Lys Gly Ile Gln Asp Ala Gly Val Ile Ala Thr Ala Lys His Tyr
            180                 185                 190

Ile Leu Asn Glu Gln Glu His Phe Arg Gln Val Gly Glu Ala Gln Gly
        195                 200                 205

Tyr Gly Tyr Asn Ile Thr Glu Thr Ile Ser Ser Asn Val Asp Asp Lys
    210                 215                 220

Thr Met His Glu Leu Tyr Leu Trp Pro Phe Ala Asp Ala Val Arg Ala
225                 230                 235                 240

Gly Val Gly Ala Val Met Cys Ser Tyr Asn Gln Ile Asn Asn Ser Tyr
                245                 250                 255

Gly Cys Gln Asn Ser Gln Thr Leu Asn Lys Leu Leu Lys Ala Glu Leu
            260                 265                 270

Gly Phe Gln Gly Phe Val Met Ser Asp Trp Ser Ala His His Ser Gly
        275                 280                 285

Val Gly Ala Ala Leu Ala Gly Leu Asp Met Ser Met Pro Gly Asp Ile
    290                 295                 300

Ser Phe Asp Asp Gly Leu Ser Phe Trp Gly Thr Asn Leu Thr Val Ser
305                 310                 315                 320

Val Leu Asn Gly Thr Val Pro Ala Trp Arg Val Asp Asp Met Ala Val
                325                 330                 335

Arg Ile Met Thr Ala Tyr Tyr Lys Val Gly Arg Asp Arg Leu Arg Ile
            340                 345                 350

Pro Pro Asn Phe Ser Ser Trp Thr Arg Asp Glu Tyr Gly Trp Glu His
        355                 360                 365
```

```
Ser Ala Val Ser Glu Gly Ala Trp Thr Lys Val Asn Asp Phe Val Asn
    370                 375                 380

Val Gln Arg Ser His Ser Gln Ile Ile Arg Glu Ile Gly Ala Ala Ser
385                 390                 395                 400

Thr Val Leu Leu Lys Asn Thr Gly Ala Leu Pro Leu Thr Gly Lys Glu
                405                 410                 415

Val Lys Val Gly Val Leu Gly Glu Asp Ala Gly Ser Asn Pro Trp Gly
            420                 425                 430

Ala Asn Gly Cys Pro Asp Arg Gly Cys Asp Asn Gly Thr Leu Ala Met
        435                 440                 445

Ala Trp Gly Ser Gly Thr Ala Asn Phe Pro Tyr Leu Val Thr Pro Glu
    450                 455                 460

Gln Ala Ile Gln Arg Glu Val Ile Ser Asn Gly Gly Asn Val Phe Ala
465                 470                 475                 480

Val Thr Asp Asn Gly Ala Leu Ser Gln Met Ala Asp Val Ala Ser Gln
                485                 490                 495

Ser Ser Val Ser Leu Val Phe Val Asn Ala Asp Ser Gly Glu Gly Phe
            500                 505                 510

Ile Ser Val Asp Gly Asn Glu Gly Asp Arg Lys Asn Leu Thr Leu Trp
        515                 520                 525

Lys Asn Gly Glu Ala Val Ile Asp Thr Val Val Ser His Cys Asn Asn
    530                 535                 540

Thr Ile Val Val Ile His Ser Val Gly Pro Val Leu Ile Asp Arg Trp
545                 550                 555                 560

Tyr Asp Asn Pro Asn Val Thr Ala Ile Ile Trp Ala Gly Leu Pro Gly
                565                 570                 575

Gln Glu Ser Gly Asn Ser Leu Val Asp Val Leu Tyr Gly Arg Val Asn
            580                 585                 590

Pro Ser Ala Lys Thr Pro Phe Thr Trp Gly Lys Thr Arg Glu Ser Tyr
        595                 600                 605

Gly Ala Pro Leu Leu Thr Glu Pro Asn Asn Gly Asn Gly Ala Pro Gln
    610                 615                 620
```

```
Asp Asp Phe Asn Glu Gly Val Phe Ile Asp Tyr Arg His Phe Asp Lys
625                 630                 635                 640

Arg Asn Glu Thr Pro Ile Tyr Glu Phe Gly His Gly Leu Ser Tyr Thr
                645                 650                 655

Thr Phe Gly Tyr Ser His Leu Arg Val Gln Ala Leu Asn Ser Ser Ser
            660                 665                 670

Ser Ala Tyr Val Pro Thr Ser Gly Glu Thr Lys Pro Ala Pro Thr Tyr
        675                 680                 685

Gly Glu Ile Gly Ser Ala Ala Asp Tyr Leu Tyr Pro Glu Gly Leu Lys
    690                 695                 700

Arg Ile Thr Lys Phe Ile Tyr Pro Trp Leu Asn Ser Thr Asp Leu Glu
705                 710                 715                 720

Asp Ser Ser Asp Asp Pro Asn Tyr Gly Trp Glu Asp Ser Glu Tyr Ile
                725                 730                 735

Pro Glu Gly Ala Arg Asp Gly Ser Pro Gln Pro Leu Leu Lys Ala Gly
            740                 745                 750

Gly Ala Pro Gly Gly Asn Pro Thr Leu Tyr Gln Asp Leu Val Arg Val
        755                 760                 765

Ser Ala Thr Ile Thr Asn Thr Gly Asn Val Ala Gly Tyr Glu Val Pro
    770                 775                 780

Gln Leu Tyr Val Ser Leu Gly Gly Pro Asn Glu Pro Arg Val Val Leu
785                 790                 795                 800

Arg Lys Phe Asp Arg Ile Phe Leu Ala Pro Gly Glu Gln Lys Val Trp
                805                 810                 815

Thr Thr Thr Leu Asn Arg Arg Asp Leu Ala Asn Trp Asp Val Glu Ala
            820                 825                 830

Gln Asp Trp Val Ile Thr Lys Tyr Pro Lys Lys Val His Val Gly Ser
        835                 840                 845

Ser Ser Arg Lys Leu Pro Leu Arg Ala Pro Leu Pro Arg Val Tyr
    850                 855                 860
```

<210> 3
<211> 253

<212> PRT
<213> Penicillium emersonii

<400> 3

```
Met Leu Ser Ser Thr Thr Arg Thr Leu Ala Phe Thr Gly Leu Ala Gly
1               5                   10                  15

Leu Leu Ser Ala Pro Leu Val Lys Ala His Gly Phe Val Gln Gly Ile
            20                  25                  30

Val Ile Gly Asp Gln Phe Tyr Ser Gly Tyr Ile Val Asn Ser Phe Pro
        35                  40                  45

Tyr Glu Ser Asn Pro Pro Pro Val Ile Gly Trp Ala Thr Thr Ala Thr
    50                  55                  60

Asp Leu Gly Phe Val Asp Gly Thr Gly Tyr Gln Gly Pro Asp Ile Ile
65                  70                  75                  80

Cys His Arg Asn Ala Thr Pro Ala Pro Leu Thr Ala Pro Val Ala Ala
                85                  90                  95

Gly Gly Thr Val Glu Leu Gln Trp Thr Pro Trp Pro Asp Ser His His
            100                 105                 110

Gly Pro Val Ile Thr Tyr Leu Ala Pro Cys Asn Gly Asn Cys Ser Thr
        115                 120                 125

Val Asp Lys Thr Thr Leu Glu Phe Phe Lys Ile Asp Gln Gln Gly Leu
    130                 135                 140

Ile Asp Asp Thr Ser Pro Pro Gly Thr Trp Ala Ser Asp Asn Leu Ile
145                 150                 155                 160

Ala Asn Asn Asn Ser Trp Thr Val Thr Ile Pro Asn Ser Val Ala Pro
                165                 170                 175

Gly Asn Tyr Val Leu Arg His Glu Ile Ile Ala Leu His Ser Ala Asn
            180                 185                 190

Asn Lys Asp Gly Ala Gln Asn Tyr Pro Gln Cys Ile Asn Ile Glu Val
        195                 200                 205

Thr Gly Gly Gly Ser Asp Ala Pro Glu Gly Thr Leu Gly Glu Asp Leu
    210                 215                 220

Tyr His Asp Thr Asp Pro Gly Ile Leu Val Asp Ile Tyr Glu Pro Ile
```

225 230 235 240

Ala Thr Tyr Thr Ile Pro Gly Pro Pro Glu Pro Thr Phe
245 250

<210> 4
<211> 454
<212> PRT
<213> Aspergillus fumigatus

<400> 4

```
Met Lys His Leu Ala Ser Ser Ile Ala Leu Thr Leu Leu Leu Pro Ala
1               5                   10                  15

Val Gln Ala Gln Gln Thr Val Trp Gly Gln Cys Gly Gly Gln Gly Trp
            20                  25                  30

Ser Gly Pro Thr Ser Cys Val Ala Gly Ala Ala Cys Ser Thr Leu Asn
        35                  40                  45

Pro Tyr Tyr Ala Gln Cys Ile Pro Gly Ala Thr Ala Thr Ser Thr Thr
    50                  55                  60

Leu Thr Thr Thr Thr Ala Ala Thr Thr Thr Ser Gln Thr Thr Thr Lys
65                  70                  75                  80

Pro Thr Thr Thr Gly Pro Thr Thr Ser Ala Pro Thr Val Thr Ala Ser
                85                  90                  95

Gly Asn Pro Phe Ser Gly Tyr Gln Leu Tyr Ala Asn Pro Tyr Tyr Ser
            100                 105                 110

Ser Glu Val His Thr Leu Ala Met Pro Ser Leu Pro Ser Ser Leu Gln
        115                 120                 125

Pro Lys Ala Ser Ala Val Ala Glu Val Pro Ser Phe Val Trp Leu Asp
    130                 135                 140

Val Ala Ala Lys Val Pro Thr Met Gly Thr Tyr Leu Ala Asp Ile Gln
145                 150                 155                 160

Ala Lys Asn Lys Ala Gly Ala Asn Pro Pro Ile Ala Gly Ile Phe Val
                165                 170                 175

Val Tyr Asp Leu Pro Asp Arg Asp Cys Ala Ala Leu Ala Ser Asn Gly
            180                 185                 190

Glu Tyr Ser Ile Ala Asn Asn Gly Val Ala Asn Tyr Lys Ala Tyr Ile
```

195 200 205

Asp Ala Ile Arg Ala Gln Leu Val Lys Tyr Ser Asp Val His Thr Ile
210 215 220

Leu Val Ile Glu Pro Asp Ser Leu Ala Asn Leu Val Thr Asn Leu Asn
225 230 235 240

Val Ala Lys Cys Ala Asn Ala Gln Ser Ala Tyr Leu Glu Cys Val Asp
245 250 255

Tyr Ala Leu Lys Gln Leu Asn Leu Pro Asn Val Ala Met Tyr Leu Asp
260 265 270

Ala Gly His Ala Gly Trp Leu Gly Trp Pro Ala Asn Leu Gly Pro Ala
275 280 285

Ala Thr Leu Phe Ala Lys Val Tyr Thr Asp Ala Gly Ser Pro Ala Ala
290 295 300

Val Arg Gly Leu Ala Thr Asn Val Ala Asn Tyr Asn Ala Trp Ser Leu
305 310 315 320

Ser Thr Cys Pro Ser Tyr Thr Gln Gly Asp Pro Asn Cys Asp Glu Lys
325 330 335

Lys Tyr Ile Asn Ala Met Ala Pro Leu Leu Lys Glu Ala Gly Phe Asp
340 345 350

Ala His Phe Ile Met Asp Thr Ser Arg Asn Gly Val Gln Pro Thr Lys
355 360 365

Gln Asn Ala Trp Gly Asp Trp Cys Asn Val Ile Gly Thr Gly Phe Gly
370 375 380

Val Arg Pro Ser Thr Asn Thr Gly Asp Pro Leu Gln Asp Ala Phe Val
385 390 395 400

Trp Ile Lys Pro Gly Gly Glu Ser Asp Gly Thr Ser Asn Ser Thr Ser
405 410 415

Pro Arg Tyr Asp Ala His Cys Gly Tyr Ser Asp Ala Leu Gln Pro Ala
420 425 430

Pro Glu Ala Gly Thr Trp Phe Gln Ala Tyr Phe Glu Gln Leu Leu Thr
435 440 445

Asn Ala Asn Pro Ser Phe
450

<210> 5
<211> 454
<212> PRT
<213> Aspergillus fumigatus

<400> 5

```
Met Lys His Leu Ala Ser Ser Ile Ala Leu Thr Leu Leu Leu Pro Ala
1               5                   10                  15

Val Gln Ala Gln Gln Thr Val Trp Gly Gln Cys Gly Gly Gln Gly Trp
            20                  25                  30

Ser Gly Pro Thr Ser Cys Val Ala Gly Ala Ala Cys Ser Thr Leu Asn
        35                  40                  45

Pro Tyr Tyr Ala Gln Cys Ile Pro Gly Ala Thr Ala Thr Ser Thr Thr
    50                  55                  60

Leu Thr Thr Thr Thr Ala Ala Thr Thr Thr Ser Gln Thr Thr Thr Lys
65                  70                  75                  80

Pro Thr Thr Thr Gly Pro Thr Thr Ser Ala Pro Thr Val Thr Ala Ser
                85                  90                  95

Gly Asn Pro Phe Ser Gly Tyr Gln Leu Tyr Ala Asn Pro Tyr Tyr Ser
            100                 105                 110

Ser Glu Val His Thr Leu Ala Met Pro Ser Leu Pro Ser Ser Leu Gln
        115                 120                 125

Pro Lys Ala Ser Ala Val Ala Glu Val Pro Ser Phe Val Trp Leu Asp
    130                 135                 140

Val Ala Ala Lys Val Pro Thr Met Gly Thr Tyr Leu Ala Asp Ile Gln
145                 150                 155                 160

Ala Lys Asn Lys Ala Gly Ala Asn Pro Pro Ile Ala Gly Ile Phe Val
                165                 170                 175

Val Tyr Asp Leu Pro Asp Arg Asp Cys Ala Ala Leu Ala Ser Asn Gly
            180                 185                 190

Glu Tyr Ser Ile Ala Asn Asn Gly Val Ala Asn Tyr Lys Ala Tyr Ile
        195                 200                 205
```

```
Asp Ala Ile Arg Ala Gln Leu Val Lys Tyr Ser Asp Val His Thr Ile
    210                 215                 220

Leu Val Ile Glu Pro Asp Ser Leu Ala Asn Leu Val Thr Asn Leu Asn
225                 230                 235                 240

Val Ala Lys Cys Ala Asn Ala Gln Ser Ala Tyr Leu Glu Cys Val Asp
                245                 250                 255

Tyr Ala Leu Lys Gln Leu Asn Leu Pro Asn Val Ala Met Tyr Leu Asp
            260                 265                 270

Ala Gly His Ala Gly Trp Leu Gly Trp Pro Ala Asn Leu Gly Pro Ala
        275                 280                 285

Ala Thr Leu Phe Ala Lys Val Tyr Thr Asp Ala Gly Ser Pro Ala Ala
    290                 295                 300

Val Arg Gly Leu Ala Thr Asn Val Ala Asn Tyr Asn Ala Trp Ser Leu
305                 310                 315                 320

Ser Thr Cys Pro Ser Tyr Thr Gln Gly Asp Pro Asn Cys Asp Glu Lys
                325                 330                 335

Lys Tyr Ile Asn Ala Met Ala Pro Leu Leu Lys Glu Ala Gly Phe Asp
            340                 345                 350

Ala His Phe Ile Met Asp Thr Ser Arg Asn Gly Val Gln Pro Thr Lys
        355                 360                 365

Gln Asn Ala Trp Gly Asp Trp Cys Asn Val Ile Gly Thr Gly Phe Gly
    370                 375                 380

Val Arg Pro Ser Thr Asn Thr Gly Asp Pro Leu Gln Asp Ala Phe Val
385                 390                 395                 400

Trp Ile Lys Pro Gly Gly Glu Ser Asp Gly Thr Ser Asn Ser Thr Ser
                405                 410                 415

Pro Arg Tyr Asp Ala His Cys Gly Tyr Ser Asp Ala Leu Gln Pro Ala
            420                 425                 430

Pro Glu Ala Gly Thr Trp Phe Gln Ala Tyr Phe Glu Gln Leu Leu Thr
        435                 440                 445

Asn Ala Asn Pro Ser Phe
    450
```

<210> 6
<211> 397
<212> PRT
<213> Aspergillus fumigatus

<400> 6

```
Met Val His Leu Ser Ser Leu Ala Ala Ala Leu Ala Ala Leu Pro Leu
1               5                   10                  15

Val Tyr Gly Ala Gly Leu Asn Thr Ala Ala Lys Ala Lys Gly Leu Lys
            20                  25                  30

Tyr Phe Gly Ser Ala Thr Asp Asn Pro Glu Leu Thr Asp Ser Ala Tyr
        35                  40                  45

Val Ala Gln Leu Ser Asn Thr Asp Asp Phe Gly Gln Ile Thr Pro Gly
    50                  55                  60

Asn Ser Met Lys Trp Asp Ala Thr Glu Pro Ser Gln Asn Ser Phe Ser
65                  70                  75                  80

Phe Ala Asn Gly Asp Ala Val Val Asn Leu Ala Asn Lys Asn Gly Gln
                85                  90                  95

Leu Met Arg Cys His Thr Leu Val Trp His Ser Gln Leu Pro Asn Trp
            100                 105                 110

Val Ser Ser Gly Ser Trp Thr Asn Ala Thr Leu Leu Ala Ala Met Lys
        115                 120                 125

Asn His Ile Thr Asn Val Val Thr His Tyr Lys Gly Lys Cys Tyr Ala
    130                 135                 140

Trp Asp Val Val Asn Glu Ala Leu Asn Glu Asp Gly Thr Phe Arg Asn
145                 150                 155                 160

Ser Val Phe Tyr Gln Ile Ile Gly Pro Ala Tyr Ile Pro Ile Ala Phe
                165                 170                 175

Ala Thr Ala Ala Ala Ala Asp Pro Asp Val Lys Leu Tyr Tyr Asn Asp
            180                 185                 190

Tyr Asn Ile Glu Tyr Ser Gly Ala Lys Ala Thr Ala Ala Gln Asn Ile
        195                 200                 205

Val Lys Met Ile Lys Ala Tyr Gly Ala Lys Ile Asp Gly Val Gly Leu
    210                 215                 220
```

Gln Ala His Phe Ile Val Gly Ser Thr Pro Ser Gln Ser Asp Leu Thr
225 230 235 240

Thr Val Leu Lys Gly Tyr Thr Ala Leu Gly Val Glu Val Ala Tyr Thr
245 250 255

Glu Leu Asp Ile Arg Met Gln Leu Pro Ser Thr Ala Ala Lys Leu Ala
260 265 270

Gln Gln Ser Thr Asp Phe Gln Gly Val Ala Ala Ala Cys Val Ser Thr
275 280 285

Thr Gly Cys Val Gly Val Thr Ile Trp Asp Trp Thr Asp Lys Tyr Ser
290 295 300

Trp Val Pro Ser Val Phe Gln Gly Tyr Gly Ala Pro Leu Pro Trp Asp
305 310 315 320

Glu Asn Tyr Val Lys Lys Pro Ala Tyr Asp Gly Leu Met Ala Gly Leu
325 330 335

Gly Ala Ser Gly Ser Gly Thr Thr Thr Thr Thr Thr Thr Thr Ser Thr
340 345 350

Thr Thr Gly Gly Thr Asp Pro Thr Gly Val Ala Gln Lys Trp Gly Gln
355 360 365

Cys Gly Gly Ile Gly Trp Thr Gly Pro Thr Thr Cys Val Ser Gly Thr
370 375 380

Thr Cys Gln Lys Leu Asn Asp Trp Tyr Ser Gln Cys Leu
385 390 395

<210> 7
<211> 792
<212> PRT
<213> Aspergillus fumigatus

<400> 7

Met Ala Val Ala Lys Ser Ile Ala Ala Val Leu Val Ala Leu Leu Pro
1 5 10 15

Gly Ala Leu Ala Gln Ala Asn Thr Ser Tyr Val Asp Tyr Asn Val Glu
20 25 30

Ala Asn Pro Asp Leu Thr Pro Gln Ser Val Ala Thr Ile Asp Leu Ser
35 40 45

```
Phe Pro Asp Cys Glu Asn Gly Pro Leu Ser Lys Thr Leu Val Cys Asp
    50                  55                  60

Thr Ser Ala Arg Pro His Asp Arg Ala Ala Ala Leu Val Ser Met Phe
65                  70                  75                  80

Thr Phe Glu Glu Leu Val Asn Asn Thr Gly Asn Thr Ser Pro Gly Val
                85                  90                  95

Pro Arg Leu Gly Leu Pro Pro Tyr Gln Val Trp Ser Glu Ala Leu His
            100                 105                 110

Gly Leu Asp Arg Ala Asn Phe Thr Asn Glu Gly Glu Tyr Ser Trp Ala
        115                 120                 125

Thr Ser Phe Pro Met Pro Ile Leu Thr Met Ser Ala Leu Asn Arg Thr
    130                 135                 140

Leu Ile Asn Gln Ile Ala Thr Ile Ile Ala Thr Gln Gly Arg Ala Phe
145                 150                 155                 160

Asn Asn Val Gly Arg Tyr Gly Leu Asp Val Tyr Ala Pro Asn Ile Asn
                165                 170                 175

Ala Phe Arg Ser Ala Met Trp Gly Arg Gly Gln Glu Thr Pro Gly Glu
            180                 185                 190

Asp Ala Tyr Cys Leu Ala Ser Ala Tyr Ala Tyr Glu Tyr Ile Thr Gly
        195                 200                 205

Ile Gln Gly Gly Val Asp Pro Glu His Leu Lys Leu Val Ala Thr Ala
    210                 215                 220

Lys His Tyr Ala Gly Tyr Asp Leu Glu Asn Trp Asp Gly His Ser Arg
225                 230                 235                 240

Leu Gly Asn Asp Met Asn Ile Thr Gln Gln Glu Leu Ser Glu Tyr Tyr
                245                 250                 255

Thr Pro Gln Phe Leu Val Ala Ala Arg Asp Ala Lys Val His Ser Val
            260                 265                 270

Met Cys Ser Tyr Asn Ala Val Asn Gly Val Pro Ser Cys Ala Asn Ser
        275                 280                 285

Phe Phe Leu Gln Thr Leu Leu Arg Asp Thr Phe Gly Phe Val Glu Asp
    290                 295                 300
```

Gly Tyr Val Ser Ser Asp Cys Asp Ser Ala Tyr Asn Val Trp Asn Pro
305 310 315 320

His Glu Phe Ala Ala Asn Ile Thr Gly Ala Ala Ala Asp Ser Ile Arg
325 330 335

Ala Gly Thr Asp Ile Asp Cys Gly Thr Thr Tyr Gln Tyr Tyr Phe Gly
340 345 350

Glu Ala Phe Asp Glu Gln Glu Val Thr Arg Ala Glu Ile Glu Arg Gly
355 360 365

Val Ile Arg Leu Tyr Ser Asn Leu Val Arg Leu Gly Tyr Phe Asp Gly
370 375 380

Asn Gly Ser Val Tyr Arg Asp Leu Thr Trp Asn Asp Val Val Thr Thr
385 390 395 400

Asp Ala Trp Asn Ile Ser Tyr Glu Ala Ala Val Glu Gly Ile Val Leu
405 410 415

Leu Lys Asn Asp Gly Thr Leu Pro Leu Ala Lys Ser Val Arg Ser Val
420 425 430

Ala Leu Ile Gly Pro Trp Met Asn Val Thr Thr Gln Leu Gln Gly Asn
435 440 445

Tyr Phe Gly Pro Ala Pro Tyr Leu Ile Ser Pro Leu Asn Ala Phe Gln
450 455 460

Asn Ser Asp Phe Asp Val Asn Tyr Ala Phe Gly Thr Asn Ile Ser Ser
465 470 475 480

His Ser Thr Asp Gly Phe Ser Glu Ala Leu Ser Ala Ala Lys Lys Ser
485 490 495

Asp Val Ile Ile Phe Ala Gly Gly Ile Asp Asn Thr Leu Glu Ala Glu
500 505 510

Ala Met Asp Arg Met Asn Ile Thr Trp Pro Gly Asn Gln Leu Gln Leu
515 520 525

Ile Asp Gln Leu Ser Gln Leu Gly Lys Pro Leu Ile Val Leu Gln Met
530 535 540

Gly Gly Gly Gln Val Asp Ser Ser Ser Leu Lys Ser Asn Lys Asn Val

545 550 555 560

Asn Ser Leu Ile Trp Gly Gly Tyr Pro Gly Gln Ser Gly Gly Gln Ala
565 570 575

Leu Leu Asp Ile Ile Thr Gly Lys Arg Ala Pro Ala Gly Arg Leu Val
580 585 590

Val Thr Gln Tyr Pro Ala Glu Tyr Ala Thr Gln Phe Pro Ala Thr Asp
595 600 605

Met Ser Leu Arg Pro His Gly Asn Asn Pro Gly Gln Thr Tyr Met Trp
610 615 620

Tyr Thr Gly Thr Pro Val Tyr Glu Phe Gly His Gly Leu Phe Tyr Thr
625 630 635 640

Thr Phe His Ala Ser Leu Pro Gly Thr Gly Lys Asp Lys Thr Ser Phe
645 650 655

Asn Ile Gln Asp Leu Leu Thr Gln Pro His Pro Gly Phe Ala Asn Val
660 665 670

Glu Gln Met Pro Leu Leu Asn Phe Thr Val Thr Ile Thr Asn Thr Gly
675 680 685

Lys Val Ala Ser Asp Tyr Thr Ala Met Leu Phe Ala Asn Thr Thr Ala
690 695 700

Gly Pro Ala Pro Tyr Pro Asn Lys Trp Leu Val Gly Phe Asp Arg Leu
705 710 715 720

Ala Ser Leu Glu Pro His Arg Ser Gln Thr Met Thr Ile Pro Val Thr
725 730 735

Ile Asp Ser Val Ala Arg Thr Asp Glu Ala Gly Asn Arg Val Leu Tyr
740 745 750

Pro Gly Lys Tyr Glu Leu Ala Leu Asn Asn Glu Arg Ser Val Val Leu
755 760 765

Gln Phe Val Leu Thr Gly Arg Glu Ala Val Ile Phe Lys Trp Pro Val
770 775 780

Glu Gln Gln Gln Ile Ser Ser Ala
785 790

<210> 8
<211> 618
<212> PRT
<213> Talaromyces emersonii

<400> 8

```
Met Ala Ser Leu Val Ala Gly Ala Leu Cys Ile Leu Gly Leu Thr Pro
1               5                   10                  15

Ala Ala Phe Ala Arg Ala Pro Val Ala Ala Arg Ala Thr Gly Ser Leu
            20                  25                  30

Asp Ser Phe Leu Ala Thr Glu Thr Pro Ile Ala Leu Gln Gly Val Leu
        35                  40                  45

Asn Asn Ile Gly Pro Asn Gly Ala Asp Val Ala Gly Ala Ser Ala Gly
    50                  55                  60

Ile Val Val Ala Ser Pro Ser Arg Ser Asp Pro Asn Tyr Phe Tyr Ser
65                  70                  75                  80

Trp Thr Arg Asp Ala Ala Leu Thr Ala Lys Tyr Leu Val Asp Ala Phe
                85                  90                  95

Ile Ala Gly Asn Lys Asp Leu Glu Gln Thr Ile Gln Gln Tyr Ile Ser
            100                 105                 110

Ala Gln Ala Lys Val Gln Thr Ile Ser Asn Pro Ser Gly Asp Leu Ser
        115                 120                 125

Thr Gly Gly Leu Gly Glu Pro Lys Phe Asn Val Asn Glu Thr Ala Phe
    130                 135                 140

Thr Gly Pro Trp Gly Arg Pro Gln Arg Asp Gly Pro Ala Leu Arg Ala
145                 150                 155                 160

Thr Ala Leu Ile Ala Tyr Ala Asn Tyr Leu Ile Asp Asn Gly Glu Ala
                165                 170                 175

Ser Thr Ala Asp Glu Ile Ile Trp Pro Ile Val Gln Asn Asp Leu Ser
            180                 185                 190

Tyr Ile Thr Gln Tyr Trp Asn Ser Ser Thr Phe Asp Leu Trp Glu Glu
        195                 200                 205

Val Glu Gly Ser Ser Phe Phe Thr Thr Ala Val Gln His Arg Ala Leu
    210                 215                 220
```

```
Val Glu Gly Asn Ala Leu Ala Thr Arg Leu Asn His Thr Cys Ser Asn
225                 230                 235                 240

Cys Val Ser Gln Ala Pro Gln Val Leu Cys Phe Leu Gln Ser Tyr Trp
                245                 250                 255

Thr Gly Ser Tyr Val Leu Ala Asn Phe Gly Gly Ser Gly Arg Ser Gly
            260                 265                 270

Lys Asp Val Asn Ser Ile Leu Gly Ser Ile His Thr Phe Asp Pro Ala
        275                 280                 285

Gly Gly Cys Asp Asp Ser Thr Phe Gln Pro Cys Ser Ala Arg Ala Leu
    290                 295                 300

Ala Asn His Lys Val Val Thr Asp Ser Phe Arg Ser Ile Tyr Ala Ile
305                 310                 315                 320

Asn Ser Gly Ile Ala Glu Gly Ser Ala Val Ala Val Gly Arg Tyr Pro
                325                 330                 335

Glu Asp Val Tyr Gln Gly Gly Asn Pro Trp Tyr Leu Ala Thr Ala Ala
            340                 345                 350

Ala Ala Glu Gln Leu Tyr Asp Ala Ile Tyr Gln Trp Lys Lys Ile Gly
        355                 360                 365

Ser Ile Ser Ile Thr Asp Val Ser Leu Pro Phe Phe Gln Asp Ile Tyr
    370                 375                 380

Pro Ser Ala Ala Val Gly Thr Tyr Asn Ser Gly Ser Thr Thr Phe Asn
385                 390                 395                 400

Asp Ile Ile Ser Ala Val Gln Thr Tyr Gly Asp Gly Tyr Leu Ser Ile
                405                 410                 415

Val Glu Lys Tyr Thr Pro Ser Asp Gly Ser Leu Thr Glu Gln Phe Ser
            420                 425                 430

Arg Thr Asp Gly Thr Pro Leu Ser Ala Ser Ala Leu Thr Trp Ser Tyr
        435                 440                 445

Ala Ser Leu Leu Thr Ala Ser Ala Arg Arg Gln Ser Val Val Pro Ala
    450                 455                 460

Ser Trp Gly Glu Ser Ser Ala Ser Ser Val Pro Ala Val Cys Ser Ala
465                 470                 475                 480
```

```
Thr Ser Ala Thr Gly Pro Tyr Ser Thr Ala Thr Asn Thr Val Trp Pro
                485                 490                 495

Ser Ser Gly Ser Gly Ser Ser Thr Thr Thr Ser Ser Ala Pro Cys Thr
            500                 505                 510

Thr Pro Thr Ser Val Ala Val Thr Phe Asp Glu Ile Val Ser Thr Ser
        515                 520                 525

Tyr Gly Glu Thr Ile Tyr Leu Ala Gly Ser Ile Pro Glu Leu Gly Asn
    530                 535                 540

Trp Ser Thr Ala Ser Ala Ile Pro Leu Arg Ala Asp Ala Tyr Thr Asn
545                 550                 555                 560

Ser Asn Pro Leu Trp Tyr Val Thr Val Asn Leu Pro Pro Gly Thr Ser
                565                 570                 575

Phe Glu Tyr Lys Phe Phe Lys Asn Gln Thr Asp Gly Thr Ile Val Trp
            580                 585                 590

Glu Asp Asp Pro Asn Arg Ser Tyr Thr Val Pro Ala Tyr Cys Gly Gln
        595                 600                 605

Thr Thr Ala Ile Leu Asp Asp Ser Trp Gln
    610                 615
```

<210> 9
<211> 574
<212> PRT
<213> Trametes cingulata

<400> 9

```
Met Arg Phe Thr Leu Leu Thr Ser Leu Leu Gly Leu Ala Leu Gly Ala
1               5                   10                  15

Phe Ala Gln Ser Ser Ala Ala Asp Ala Tyr Val Ala Ser Glu Ser Pro
            20                  25                  30

Ile Ala Lys Ala Gly Val Leu Ala Asn Ile Gly Pro Ser Gly Ser Lys
        35                  40                  45

Ser Asn Gly Ala Lys Ala Gly Ile Val Ile Ala Ser Pro Ser Thr Ser
    50                  55                  60

Asn Pro Asn Tyr Leu Tyr Thr Trp Thr Arg Asp Ser Ser Leu Val Phe
65                  70                  75                  80
```

Lys Ala Leu Ile Asp Gln Phe Thr Thr Gly Glu Asp Thr Ser Leu Arg
85 90 95

Thr Leu Ile Asp Glu Phe Thr Ser Ala Glu Ala Ile Leu Gln Gln Val
100 105 110

Pro Asn Pro Ser Gly Thr Val Ser Thr Gly Gly Leu Gly Glu Pro Lys
115 120 125

Phe Asn Ile Asp Glu Thr Ala Phe Thr Asp Ala Trp Gly Arg Pro Gln
130 135 140

Arg Asp Gly Pro Ala Leu Arg Ala Thr Ala Ile Ile Thr Tyr Ala Asn
145 150 155 160

Trp Leu Leu Asp Asn Lys Asn Thr Thr Tyr Val Thr Asn Thr Leu Trp
165 170 175

Pro Ile Ile Lys Leu Asp Leu Asp Tyr Val Ala Ser Asn Trp Asn Gln
180 185 190

Ser Thr Phe Asp Leu Trp Glu Glu Ile Asn Ser Ser Ser Phe Phe Thr
195 200 205

Thr Ala Val Gln His Arg Ala Leu Arg Glu Gly Ala Thr Phe Ala Asn
210 215 220

Arg Ile Gly Gln Thr Ser Val Val Ser Gly Tyr Thr Thr Gln Ala Asn
225 230 235 240

Asn Leu Leu Cys Phe Leu Gln Ser Tyr Trp Asn Pro Thr Gly Gly Tyr
245 250 255

Ile Thr Ala Asn Thr Gly Gly Gly Arg Ser Gly Lys Asp Ala Asn Thr
260 265 270

Val Leu Thr Ser Ile His Thr Phe Asp Pro Ala Ala Gly Cys Asp Ala
275 280 285

Val Thr Phe Gln Pro Cys Ser Asp Lys Ala Leu Ser Asn Leu Lys Val
290 295 300

Tyr Val Asp Ala Phe Arg Ser Ile Tyr Ser Ile Asn Ser Gly Ile Ala
305 310 315 320

Ser Asn Ala Ala Val Ala Thr Gly Arg Tyr Pro Glu Asp Ser Tyr Met
325 330 335

Gly Gly Asn Pro Trp Tyr Leu Thr Thr Ser Ala Val Ala Glu Gln Leu
340 345 350

Tyr Asp Ala Leu Ile Val Trp Asn Lys Leu Gly Ala Leu Asn Val Thr
355 360 365

Ser Thr Ser Leu Pro Phe Phe Gln Gln Phe Ser Ser Gly Val Thr Val
370 375 380

Gly Thr Tyr Ala Ser Ser Ser Ser Thr Phe Lys Thr Leu Thr Ser Ala
385 390 395 400

Ile Lys Thr Phe Ala Asp Gly Phe Leu Ala Val Asn Ala Lys Tyr Thr
405 410 415

Pro Ser Asn Gly Gly Leu Ala Glu Gln Tyr Ser Arg Ser Asn Gly Ser
420 425 430

Pro Val Ser Ala Val Asp Leu Thr Trp Ser Tyr Ala Ala Ala Leu Thr
435 440 445

Ser Phe Ala Ala Arg Ser Gly Lys Thr Tyr Ala Ser Trp Gly Ala Ala
450 455 460

Gly Leu Thr Val Pro Thr Thr Cys Ser Gly Ser Gly Gly Ala Gly Thr
465 470 475 480

Val Ala Val Thr Phe Asn Val Gln Ala Thr Thr Val Phe Gly Glu Asn
485 490 495

Ile Tyr Ile Thr Gly Ser Val Pro Ala Leu Gln Asn Trp Ser Pro Asp
500 505 510

Asn Ala Leu Ile Leu Ser Ala Ala Asn Tyr Pro Thr Trp Ser Ser Thr
515 520 525

Val Asn Leu Pro Ala Ser Thr Thr Ile Glu Tyr Lys Tyr Ile Arg Lys
530 535 540

Phe Asn Gly Ala Val Thr Trp Glu Ser Asp Pro Asn Asn Ser Ile Thr
545 550 555 560

Thr Pro Ala Ser Gly Thr Phe Thr Gln Asn Asp Thr Trp Arg
565 570

## Description

This patent contains a Sequence Listing.

## FIELD OF THE INVENTION

The invention relates to processes for obtaining hydrolysis products and fermentation products from pretreated lignocellulose-containing material.

## DESCRIPTION OF RELATED ART

Renewable energy sources provide an alternative to current fossil fuel dependence. Production of ethanol as an energy source includes the basic steps of hydrolysis and fermentation. These steps are integrated within larger processes to obtain ethanol from various source materials. Processes have been developed using sources including starch-based feedstocks, sugars and biomass, including cellulosic feedstocks.

When the selected feedstock is starch-based, the sugars are readily available and/or accessible for hydrolysis and fermentation. Where biomass is used for production of ethanol, the biomass is generally pretreated to reduce resistance to hydrolysis and fermentation processes. The pretreatment may be performed prior to or subsequent with the hydrolysis and/or fermentation steps, in order to increase the availability of the cellulosic material for those steps. Without this pretreatment, the structural complexity of the biomass will hinder conversion of the cellulosic material to ethanol.

Current pretreatment processes include physical pretreatment, chemical pretreatment and biological pretreatment, but each of these processes has inherent limitations.

Pretreatment of biomass can also result in release of byproducts that are undesirable to the process as a whole. Different types of biomass will contain varying amounts of cellulose, hemicellulose, lignin and other components. Pretreatment of the biomass, which is necessary to access the sugars from the cellulose and hemicellulose may also result in production of byproducts that may interfere in the hydrolysis and fermentation steps, resulting in reduced overall process efficiency and ethanol production. Such byproducts may include sugar based oligomers and lignin based inhibitors.

While laccase has been observed as useful in the detoxification of pretreated corn stover (WO2008134259), additional detoxification processes would be useful to the industry.

Thus, there remains a need in the art to develop processes for treatment of biomass feedstocks that result in increased availability of cellulosic material for conversion to ethanol and decreased production of undesirable byproducts. The present invention provides such processes.

## SUMMARY OF THE INVENTION

Described herein are processes for preconditioning pretreated lignocellulosic material to improve availability for enzymatic hydrolysis (saccharification). The invention relates to processes for producing a hydrolysis product and/or a fermentation product from preconditioned lignocellulosic material. Compositions suitable for use in methods and/or processes of the invention are also described.

The present invention is based on the surprising discovery that preconditioning of pretreated biomass with a combination of laccase and beta-glucosidase prior to hydrolysis reduces DP2 concentration and lignin-based inhibitors, thereby improving enzyme hydrolysis and increasing glucose yield.

Disclosed are processes for preconditioning pretreated lignocellulose-containing material, the process comprising incubating the pretreated lignocellulose-containing material with laccase and beta-glucosidase prior to hydrolysis.

In the first aspect, the invention relates to processes for producing a hydrolysis product from pretreated lignocellulose-containing material, the process comprising:

(i) preconditioning a pretreated lignocellulose-containing material, the preconditioning comprising incubating the pretreated lignocellulose-containing material with laccase and beta-glucosidase; and

(ii) hydrolyzing the preconditioned lignocellulose-containing material with a cellulolytic enzyme composition to obtain a hydrolysis product, wherein preconditioning and hydrolysis are carried out sequentially.

In an additional aspect, the invention relates to processes for producing a fermentation product from pretreated lignocellulose-containing material, the process comprising:
preconditioning a pretreated lignocellulose-containing material, the preconditioning comprising incubating the pretreated

lignocellulose-containing material with laccase and beta-glucosidase;

(i) hydrolyzing the preconditioned lignocellulose-containing material with a cellulolytic enzyme composition to obtain a hydrolysis product; and

(ii) fermenting the hydrolysis product to obtain a fermentation product, wherein preconditioning and hydrolysis are carried out sequentially.

Disclosed is an enzyme preconditioning composition for the preconditioning of unwashed pretreated fractionated corn fiber, the composition comprising laccase and beta-glucosidase.

## DEFINITIONS

**Beta-glucosidase:** The term "beta-glucosidase" means a beta-D-glucoside glucohydrolase (E.C. 3.2.1.21) that catalyzes the hydrolysis of terminal non-reducing beta-D-glucosyl residues with the release of beta-D-glucose.

Beta-glucosidase activity may be determined using p-nitrophenyl-beta-D-glucopyranoside as substrate according to the procedure of Venturi et al., 2002, Extracellular beta-D-glucosidase from Chaetomium thermophilum var. coprophilum: production, purification and some biochemical properties, J. Basic Microbiol. 42: 55-66. One unit of beta-glucosidase is defined as 1.0 μmole of p-nitrophenolate anion produced per minute at 25°C, pH 4.8 from 1 mM p-nitrophenyl-beta-D-glucopyranoside as substrate in 50 mM sodium citrate containing 0.01% TWEEN® 20 (polyoxyethylene sorbitan monolaurate).

**Cellobiohydrolase:** The term "cellobiohydrolase" ("CBH") generally means a 1,4-beta-D-glucan cellobiohydrolase (E.C. 3.2.1.91) that catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain (Teeri, 1997, Crystalline cellulose degradation: New insight into the function of cellobiohydrolases, Trends in Biotechnology 15: 160-167; Teeri et al., 1998, Trichoderma reesei cellobiohydrolases: why so efficient on crystalline cellulose?, Biochem. Soc. Trans. 26: 173-178).

Cellobiohydrolase activity is determined according to the procedures described by Lever et al., 1972, Anal. Biochem. 47: 273-279; van Tilbeurgh et al., 1982, FEBS Letters, 149: 152-156; van Tilbeurgh and Claeyssens, 1985, FEBS Letters, 187: 283-288; and Tomme et al., 1988, Eur. J. Biochem. 170: 575-581. In the present invention, the Tomme *et al.* method can be used to determine cellobiohydrolase activity.

**Cellulolytic enzyme preparation, cellulolytic composition, or cellulase:** The term "cellulolytic enzyme preparation", "cellulolytic composition", or "cellulase" means one or more (*e.g.,* several) enzymes that hydrolyze a cellulosic material. Such enzymes include endoglucanase(s), cellobiohydrolase(s), beta-glucosidase(s), or combinations thereof. The two basic approaches for measuring cellulolytic activity include: (1) measuring the total cellulolytic activity, and (2) measuring the individual cellulolytic activities (endoglucanases, cellobiohydrolases, and beta-glucosidases) as reviewed in Zhang et al., Outlook for cellulase improvement: Screening and selection strategies, 2006, Biotechnology Advances 24: 452-481. Total cellulolytic activity is usually measured using insoluble substrates, including Whatman №1 filter paper, microcrystalline cellulose, bacterial cellulose, algal cellulose, cotton, pretreated lignocellulose, etc. The most common total cellulolytic activity assay is the filter paper assay using Whatman №1 filter paper as the substrate. The assay was established by the International Union of Pure and Applied Chemistry (IUPAC) (Ghose, 1987, Measurement of cellulase activities, Pure Appl. Chem. 59: 257-68).

Cellulolytic enzyme activity is determined by measuring the increase in hydrolysis of a cellulosic material by cellulolytic enzyme(s) under the following conditions: 1-50 mg of cellulolytic enzyme protein/g of cellulose in Pretreated Corn Stover ("PCS") (or other pretreated cellulosic material) for 3-7 days at a suitable temperature, e.g., 50°C, 55°C, or 60°C, compared to a control hydrolysis without addition of cellulolytic enzyme protein. Typical conditions are 1 ml reactions, washed or unwashed PCS, 5% insoluble solids, 50 mM sodium acetate pH 5, 1 mM $MnSO_4$, 50°C, 55°C, or 60°C, 72 hours, sugar analysis by AMINEX® HPX-87H column (Bio-Rad Laboratories, Inc., Hercules, CA, USA).

**Hemicellulolytic enzyme, hemicellulolytic enzyme preparation or hemicellulase:** The term "hemicellulolytic enzyme", "hemicellulolytic enzyme preparation" or "hemicellulase" means one or more (e.g., several) enzymes that hydrolyze a hemicellulosic material. See, for example, Shallom and Shoham, 2003, Current Opinion In Microbiology 6(3): 219-228). Hemicellulases are key components in the degradation of plant biomass. Examples of hemicellulases include, but are not limited to, an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates for these enzymes, hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families. Some families, with an overall similar fold, can be further grouped into clans, marked alphabetically (*e.g.,* GH-A). A most informative and updated classification of these and other carbohydrate active enzymes is available in the Carbohydrate-Active Enzymes (CAZy) database. Hemicellulolytic enzyme activities can be measured according to Ghose and Bisaria, 1987, Pure & Appl. Chem. 59: 1739-1752, at a suitable temperature such as 40°C-80°C, e.g., 50°C, 55°C, 60°C, 65°C, or 70°C, and a suitable pH such as 4-9, *e.g.,* 5.0, 5.5, 6.0, 6.5, or 7.0.

**Laccase:** The term "laccase" means an oxidase family enzyme (E.C. 3.2.1.21) that oxidizes phenols and similar molecules.

**Mature polypeptide:** The term "mature polypeptide" means a polypeptide in its final form following translation and any post-translational modifications, such as N-terminal processing, C-terminal truncation, glycosylation, phosphorylation, etc. For instance, the mature polypeptide may be identified, using, e.g., the SignalP program (Nielsen et al., 1997, Protein Engineering 10: 1-6) that predicts a portion of the amino acid sequence as a signal peptide. As such, the mature polypeptide would be identified as the sequence lacking such redicted signal portion.

It is known in the art that a host cell may produce a mixture of two of more different mature polypeptides (*i.e.,* with a different C-terminal and/or N-terminal amino acid) expressed by the same polynucleotide.

**Mature polypeptide coding sequence:** The term "mature polypeptide coding sequence" means a polynucleotide that encodes a mature polypeptide having enzyme activity.

**Parent Enzyme:** The term "parent" means an enzyme to which an alteration is made to produce a variant. The parent may be a naturally occurring (wild-type) polypeptide or a variant thereof.

**Polypeptide having cellulolytic enhancing activity:** The term "polypeptide having cellulolytic enhancing activity" means a polypeptide that catalyzes the enhancement of the hydrolysis of a cellulosic material by enzyme having cellulolytic activity.

"Auxiliary Activity 9" or "AA9" means a polypeptide classified as a lytic polysaccharide monooxygenase (Quinlan et al., 2011, Proc. Natl. Acad. Sci. USA 208: 15079-15084; Phillips et al., 2011, ACS Chem. Biol. 6: 1399-1406; Lin et a/., 2012, Structure 20: 1051-1061). AA9 polypeptides were formerly classified into the glycoside hydrolase Family 61 (GH61) according to Henrissat, 1991, Biochem. J. 280: 309-316, and Henrissat and Bairoch, 1996, Biochem. J. 316: AA9 polypeptides enhance the hydrolysis of a cellulosic material by an enzyme having cellulolytic activity. Cellulolytic enhancing activity may be determined by measuring the increase in reducing sugars or the increase of the total of cellobiose and glucose from the hydrolysis of a cellulosic material by cellulolytic enzyme under the following conditions: 1-50 mg of total protein/g of cellulose in pretreated corn stover (PCS), wherein total protein is comprised of 50-99.5% w/w cellulolytic enzyme protein and 0.5-50% w/w protein of an AA9 polypeptide for 1-7 days at a suitable temperature, such as 40°C-80°C, e.g., 50°C, 55°C, 60°C, 65°C, or 70°C, and a suitable pH, such as 4-9, *e.g.,* 4.5, 5.0, 5.5, 6.0, 6.5, 7.0, 7.5, 8.0, or 8.5, compared to a control hydrolysis with equal total protein loading without cellulolytic enhancing activity (1-50 mg of cellulolytic protein/g of cellulose in PCS).

AA9 polypeptide enhancing activity can be determined using a mixture of CELLUCLAST® 1.5L (Novozymes A/S, Bagsværd, Denmark) in the presence of 2-3% of total protein weight *Aspergillus oryzae* beta-glucosidase (recombinantly produced in *Aspergillus oryzae* according to WO 02/095014) or 2-3% of total protein weight *Aspergillus fumigatus* beta-glucosidase (recombinantly produced in *Aspergillus oryzae* as described in WO 02/095014) of cellulase protein loading is used as the source of the cellulolytic activity.

AA9 polypeptide enhancing activity can also be determined by incubating an AA9 polypeptide with 0.5% phosphoric acid swollen cellulose (PASC), 100 mM sodium acetate pH 5, 1 mM $MnSO_4$, 0.1% gallic acid, 0.025 mg/ml of *Aspergillus fumigatus* beta-glucosidase, and 0.01% TRITON® X-100 (4-(1,1,3,3-tetramethylbutyl)phenyl-polyethylene glycol) for 24-96 hours at 40°C followed by determination of the glucose released from the PASC

AA9 polypeptide enhancing activity can also be determined according to WO 2013/028928 for high temperature compositions.

AA9 polypeptides enhance the hydrolysis of a cellulosic material catalyzed by enzyme having cellulolytic activity by reducing the amount of cellulolytic enzyme required to reach the same degree of hydrolysis preferably at least 1.01-fold, *e.g.,* at least 1.05-fold, at least 1.10-fold, at least 1.25-fold, at least 1.5-fold, at least 2-fold, at least 3-fold, at least 4-fold, at least 5-fold, at least 10-fold, or at least 20-fold.

**Variant:** The term "variant" means a polypeptide having enzyme or enzyme enhancing activity comprising an alteration, *i.e.,* a substitution, insertion, and/or deletion, at one or more (*e.g.,* several) positions. A substitution means replacement of the amino acid occupying a position with a different amino acid; a deletion means removal of the amino acid occupying a position; and an insertion means adding an amino acid adjacent to and immediately following the amino acid occupying a position.

**Wild-Type Enzyme:** The term "wild-type" enzyme means an enzyme expressed by a naturally occurring microorganism, such as a bacterium, yeast, or filamentous fungus found in nature.

**Allelic variant:** The term "allelic variant" means any of two or more (*e.g.,* several) alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Fragment:** The term "fragment" means a polypeptide having one or more (*e.g.,* several) amino acids absent from the amino and/or carboxyl terminus of a mature polypeptide main; wherein the fragment has enzyme activity. In one aspect, a fragment contains at least 85%, *e.g.,* at least 90% or at least 95% of the amino acid residues of the mature polypeptide of an enzyme.

**Mature polypeptide:** The term "mature polypeptide" means a polypeptide in its final form following translation and any post-translational modifications, such as N-terminal processing, C-terminal truncation, glycosylation, phosphorylation, etc. For instance, the mature polypeptide of an *A. fumigatus* cellobiohydrolase I is amino acids 27 to 532 of SEQ ID NO: 10 herein based on the SignalP program (Nielsen et al., 1997, Protein Engineering 10: 1-6) that predicts amino acids 1 to 26 of SEQ ID NO: 10 herein are a signal peptide. In another aspect, the mature polypeptide of an *A. fumigates* cellobiohydrolase II is amino acids 20 to 454 of SEQ ID NO: 11 herein based on the SignalP program that predicts amino acids 1 to 19 of SEQ ID NO: 11 herein are a signal peptide. In another aspect, the mature polypeptide of an *A. fumigatus* beta-glucosidase is amino acids 20 to 863 of SEQ ID NO: 5 herein based on the SignalP program that predicts amino acids 1 to 19 of SEQ ID NO: 5 herein are a signal peptide. In another aspect, the mature polypeptide of a *Penicillium* sp. GH61 polypeptide is amino acids 26 to 253 of SEQ ID NO: 7 herein based on the SignalP program that predicts amino acids 1 to 25 of SEQ ID NO: 7 herein are a signal peptide.

It is known in the art that a host cell may produce a mixture of two of more different mature polypeptides (i.e., with a different C-terminal and/or N-terminal amino acid) expressed by the same polynucleotide.

**Mature polypeptide coding sequence:** The term "mature polypeptide coding sequence" means a polynucleotide that encodes a mature polypeptide having enzyme activity.

**Parent Enzyme:** The term "parent" means an enzyme to which an alteration is made to produce a variant. The parent may be a naturally occurring (wild-type) polypeptide or a variant thereof.

**Pretreated corn stover:** The term "PCS" or "Pretreated Corn Stover" means a cellulosic material derived from corn stover by treatment with heat and dilute sulfuric acid, alkaline pretreatment, or neutral pretreatment.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice et al., 2000, *supra),* preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Deoxyribonucleotides} \times 100)/(\text{Length of Alignment} - \text{Total Number of Gaps in Alignment})$$

**Subsequence:** The term "subsequence" means a polynucleotide having one or more (e.g., several) nucleotides

absent from the 5' and/or 3' end of a mature polypeptide coding sequence; wherein the subsequence encodes a fragment having enzyme activity. In one aspect, a subsequence contains at least 85%, *e.g.,* at least 90% or at least 95% of the nucleotides of the mature polypeptide coding sequence of an enzyme.

Reference to "about" a value or parameter herein includes aspects that are directed to that value or parameter perse. For example, description referring to "about X" includes the aspect "X".

As used herein and in the appended claims, the singular forms "a," "or," and "the" include plural referents unless the context clearly dictates otherwise. It is understood that the aspects of the invention described herein include "consisting" and/or "consisting essentially of" aspects.

Unless defined otherwise or clearly indicated by context, all technical and scientific terms used herein have the same meaning as commonly understood by one of ordinary skill in the art to which this invention belongs.

## BRIEF DESCRIPTION OF THE DRAWINGS

FIG. 1 is a graph of glucose concentration after hydrolysis of preconditioned uwPCF under the conditions described in Example 1.

FIG. 2 is a graph of glucose concentration in response to varied laccase doses on low solids uwPCF under the conditions described in Example 5.

## DETAILED DESCRIPTION

Described herein are processes for preconditioning biomass to increase production of fermentable sugars and to decrease production of interfering byproducts, such as oligomers (e.g., DP2) and lignin-based inhibitors. Also described are preconditioning compositions suitable for use in the processes and/or methods described herein.

The present invention relates to processes of producing hydrolysis products using such preconditioned biomass and to processes of producing fermentation products using such preconditioned biomass.

The present inventors have surprisingly found that enzymatic preconditioning of lignocellulose-containing material with a combination of laccase and beta-glucosidase before saccharifying (i.e., hydrolyzing) with a cellulolytic enzyme preparation results in decreased inhibitors of saccharification and lignin based inhibitors. The preconditioning therefore results in improved saccharification, e.g. improved sugar concentrations, as compared to when no preconditioning is done. Processes of the invention result in increased yield of fermentable sugars and, ultimately, fermentation products, as compared to preconditioning with laccase without beta-glucosidase. The laccase mode of action is known to be through the creation of semi-stable free radicals. While not wishing to be bound by any particular theory, it is believed that a combination of laccase and beta-glucosidase work synergistically to create more accessible cellulose for subsequent hydrolysis. Because the free radicals can degrade proteins/enzymes, in a particular embodiment the methods described herein are provided as a preconditioning step, prior to hydrolysis.

Subsequently, the sugars may be converted into a number of products including fermentation products (*e.g.,* ethanol or butanol) or into syrups (*e.g.,* High Fructose Corn Syrup (HFCS) and lignocellulose-derived plastics including polyethylene, polystyrene, polypropylene). Other end products include lactic acid which can serve as a feedstock for production of polylactic acid (PLA) to replace petrochemical packaging materials such as PET.

### Cellulosic Material

The processes of the present invention are carried out using lignocellulosic material. The term "lignocellulosic material" means any material containing lignocellulose The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in the stems, leaves, hulls, husks, and cobs of plants or leaves, branches, and wood of trees. The cellulosic material may be, but is not limited to, agricultural residue, herbaceous material (including energy crops), municipal solid waste, pulp and paper mill residue, waste paper, and wood (including forestry residue) (see, for example, Wiselogel et al., 1995, in Handbook on Bioethanol (Charles E. Wyman, editor),

pp.105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp. 23-40, Springer-Verlag, New York). It is understood herein that the cellulose may be in the form of lignocellulose, a plant cell wall material containing lignin, cellulose, and hemicellulose in a mixed matrix. In a preferred embodiment, the cellulosic material is any biomass material. In another preferred embodiment, the cellulosic material is lignocellulose, which comprises cellulose, hemicelluloses, and lignin.

In an embodiment, the cellulosic material is agricultural residue, herbaceous material (including energy crops), municipal solid waste, pulp and paper mill residue, waste paper, orwood (including forestry residue).

In another embodiment, the cellulosic material is arundo, bagasse, bamboo, corn cob, corn fiber, corn stover, miscanthus, rice straw, switchgrass, or wheat straw.

Corn kernels are comprised of three main components: bran, endosperm, and germ. Corn bran holds the fiber, the hard outer layer of the kernel. The endosperm contains the majority of the starch, found on the interior of the kernel. The germ is at the center of the kernel by the bottom tip cap, containing an abundance of proteins and oils.

In one embodiment, the cellulosic material is fiber, such as corn fiber or wheat fiber. Fiber, such as corn or wheat fiber, may be obtained by fractionation. Fractionation technologies are well-known in the art. In one embodiment the cellulosic material is fiber obtained from dry fractionation processes. In one embodiment the cellulosic material is fiber obtained from wet fractionation processes.

In another embodiment, the cellulosic material is aspen, eucalyptus, fir, pine, poplar, spruce, or willow.

In another embodiment, the cellulosic material is algal cellulose, bacterial cellulose, cotton linter, filter paper, microcrystalline cellulose (*e.g.,* AVICEL®), or phosphoric-acid treated cellulose.

In another embodiment, the cellulosic material is an aquatic biomass. As used herein the term "aquatic biomass" means biomass produced in an aquatic environment by a photosynthesis process. The aquatic biomass may be algae, emergent plants, floating-leaf plants, or submerged plants.

The cellulosic material may be used as is or may be subjected to pretreatment, using conventional methods known in the art, as described more fully herein. In a preferred embodiment, the cellulosic material is pretreated.

**Pretreatment of Cellulosic Material**

In practicing the processes of the present invention, any pretreatment process known in the art may be used to disrupt plant cell wall components of the cellulosic material (Chandra et al., 2007, Adv. Biochem. Engin./Biotechnol. 108: 67-93; Galbe and Zacchi, 2007, Adv. Biochem. Engin./Biotechnol. 108: 41-65; Hendriks and Zeeman, 2009, Bioresource Technology 100: 10-18; Mosier et al., 2005, Bioresource Technology 96: 673-686; Taherzadeh and Karimi, 2008, Int. J. Mol. Sci. 9: 1621-1651; Yang and Wyman, 2008, Biofuels Bioproducts and Biorefining-Biofpr. 2: 26-40).

The cellulosic material may also be subjected to particle size reduction, sieving, presoaking, wetting, washing, and/or conditioning prior to or with additional pretreatment methods, using methods known in the art or as otherwise described herein.

Conventional pretreatments include, but are not limited to, steam pretreatment (with or without explosion), dilute acid pretreatment, hot water pretreatment, alkaline pretreatment, lime pretreatment, wet oxidation, wet explosion, ammonia fiber explosion, organosolv pretreatment, and biological pretreatment. Additional pretreatments include ammonia percolation, ultrasound, electroporation, microwave, supercritical $CO_2$, supercritical $H_2O$, ozone, ionic liquid, and gamma irradiation pretreatments. The cellulosic material is pretreated before hydrolysis and/or fermentation. Pretreatment is preferably performed prior to the hydrolysis. In a further embodiment preconditioning is performed after pretreatment and before hydrolysis and fermentation.

Alternatively, pretreatment may be carried out simultaneously with enzyme hydrolysis to release fermentable sugars, such as glucose, xylose, and/or cellobiose. In most cases the pretreatment step itself results in some conversion of biomass to fermentable sugars (even in absence of enzymes).

Steam Pretreatment. In steam pretreatment, the cellulosic material is heated to disrupt the plant cell wall components, including lignin, hemicellulose, and cellulose to make the cellulose and other fractions, e.g., hemicellulose, accessible to enzymes. The cellulosic material is passed to or through a reaction vessel where steam is injected to increase the temperature to the required temperature and pressure and is retained therein for the desired reaction time. Steam pretreatment is preferably performed at 140-250°C, *e.g.,* 160-200°C or 170-190°C, where the optimal temperature range depends on optional addition of a chemical catalyst. Residence time for the steam pretreatment is preferably 1-60 minutes, e.g., 1-30 minutes, 1-20 minutes, 3-12 minutes, or 4-10 minutes, where the optimal residence time depends on the temperature and optional addition of a chemical catalyst. Steam pretreatment allows for relatively high solids loadings, so that the cellulosic material is generally only moist during the pretreatment. The steam pretreatment is often combined with an explosive discharge of the material after the pretreatment, which is known as steam explosion, that is, rapid flashing to atmospheric pressure and turbulent flow of the material to increase the accessible surface area by

fragmentation (Duff and Murray, 1996, Bioresource Technology 855: 1-33; Galbe and Zacchi, 2002, Appl. Microbiol. Biotechnol. 59: 618-628; U.S. Patent Application No. 2002/0164730). During steam pretreatment, hemicellulose acetyl groups are cleaved and the resulting acid autocatalyzes partial hydrolysis of the hemicellulose to monosaccharides and oligosaccharides. Lignin is removed to only a limited extent.

Chemical Pretreatment: The term "chemical pretreatment" refers to any chemical pretreatment that promotes the separation and/or release of cellulose, hemicellulose, and/or lignin. Such a pretreatment may convert crystalline cellulose to amorphous cellulose. Examples of suitable chemical pretreatment processes include, for example, dilute acid pretreatment, lime pretreatment, wet oxidation, ammonia fiber/freeze expansion (AFEX), ammonia percolation (APR), ionic liquid, and organosolv pretreatments.

A chemical catalyst such as $H_2SO_4$ or $SO_2$ (typically 0.3 to 5% w/w) is sometimes added prior to steam pretreatment, which decreases the time and temperature, increases the recovery, and improves enzymatic hydrolysis (Ballesteros et al., 2006, Appl. Biochem. Biotechnol. 129-132: 496-508; Varga et al., 2004, Appl. Biochem. Biotechnol. 113-116: 509-523; Sassner et al., 2006, Enzyme Microb. Technol. 39: 756-762). In dilute acid pretreatment, the cellulosic material is mixed with dilute acid, typically $H_2SO_4$, and water to form a slurry, heated by steam to the desired temperature, and after a residence time flashed to atmospheric pressure. The dilute acid pretreatment may be performed with a number of reactor designs, *e.g.,* plug-flow reactors, counter-current reactors, or continuous counter-current shrinking bed reactors (Duff and Murray, 1996, *supra*; Schell et al., 2004, Bioresource Technology 91: 179-188; Lee et al., 1999, Adv. Biochem. Eng. Biotechnol. 65: 93-115).

Several methods of pretreatment under alkaline conditions may also be used. These alkaline pretreatments include, but are not limited to, sodium hydroxide, lime, wet oxidation, ammonia percolation (APR), and ammonia fiber/freeze expansion (AFEX) pretreatment.

Lime pretreatment is performed with calcium oxide or calcium hydroxide at temperatures of 85-150°C and residence times from 1 hour to several days (Wyman et al., 2005, Bioresource Technology 96: 1959-1966; Mosier *et al.,* 2005, *supra).* WO 2006/110891, WO 2006/110899, WO 2006/110900, and WO 2006/110901 disclose pretreatment methods using ammonia.

Wet oxidation is a thermal pretreatment performed typically at 180-200°C for 5-15 minutes with addition of an oxidative agent such as hydrogen peroxide or over-pressure of oxygen (Schmidt and Thomsen, 1998, Bioresource Technology 64: 139-151; Palonen et al., 2004, Appl. Biochem. Biotechnol. 117: 1-17; Varga et al., 2004, Biotechnol. Bioeng. 88: 567-574; Martin et al., 2006, J. Chem. Technol. Biotechnol. 81: 1669-1677). The pretreatment is performed preferably at 1-40% dry matter, *e.g.,* 2-30% dry matter or 5-20% dry matter, and often the initial pH is increased by the addition of alkali such as sodium carbonate.

A modification of the wet oxidation pretreatment method, known as wet explosion (combination of wet oxidation and steam explosion) can handle dry matter up to 30%. In wet explosion, the oxidizing agent is introduced during pretreatment after a certain residence time. The pretreatment is then ended by flashing to atmospheric pressure (WO 2006/032282).

Ammonia fiber expansion (AFEX) involves treating the cellulosic material with liquid or gaseous ammonia at moderate temperatures such as 90-150°C and high pressure such as 17-20 bar for 5-10 minutes, where the dry matter content can be as high as 60% (Gollapalli et al., 2002, Appl. Biochem. Biotechnol. 98: 23-35; Chundawat et al., 2007, Biotechnol. Bioeng. 96: 219-231; Alizadeh et al., 2005, Appl. Biochem. Biotechnol. 121: 1133-1141; Teymouri et al., 2005, Bioresource Technology 96: 2014-2018). During AFEX pretreatment cellulose and hemicelluloses remain relatively intact. Lignin-carbohydrate complexes are cleaved.

Organosolv pretreatment delignifies the cellulosic material by extraction using aqueous ethanol (40-60% ethanol) at 160-200°C for 30-60 minutes (Pan et a/., 2005, Biotechnol. Bioeng. 90: 473-481; Pan et al., 2006, Biotechnol. Bioeng. 94: 851-861; Kurabi et al., 2005, Appl. Biochem. Biotechnol. 121: 219-230). Sulphuric acid is usually added as a catalyst. In organosolv pretreatment, the majority of hemicellulose and lignin is removed.

Other examples of suitable pretreatment methods are described by Schell et al., 2003, Appl. Biochem. Biotechnol. 105-108: 69-85, and Mosier *et al.,* 2005, *supra,* and U.S. Published Application 2002/0164730.

In one embodiment, the chemical pretreatment is preferably carried out as a dilute acid treatment, and more preferably as a continuous dilute acid treatment. The acid is typically sulfuric acid, but other acids may also be used, such as acetic acid, citric acid, nitric acid, phosphoric acid, tartaric acid, succinic acid, hydrogen chloride, or mixtures thereof. Mild acid treatment is conducted in the pH range of preferably 1-5, *e.g.,* 1-4 or 1-2.5. In one embodiment, the acid concentration is in the range from preferably 0.01 to 10 wt. % acid, *e.g.,* 0.05 to 5 wt. % acid or 0.1 to 2 wt. % acid. The acid is contacted with the cellulosic material and held at a temperature in the range of preferably 140-200°C, *e.g.,* 165-190°C, for periods ranging from 1 to 60 minutes.

In another embodiment, pretreatment takes place in an aqueous slurry. In preferred embodiments, the cellulosic material is present during pretreatment in amounts preferably between 10-80 wt. %, e.g., 20-70 wt. % or 30-60 wt. %, such as around 40 wt. %. The pretreated cellulosic material may be unwashed or washed using any method known in the art, e.g., washed with water.

Mechanical Pretreatment or Physical Pretreatment: The term "mechanical pretreatment" or "physical pretreatment" refers to any pretreatment that promotes size reduction of particles. For example, such pretreatment may involve various types of grinding or milling (*e.g.,* dry milling, wet milling, or vibratory ball milling).

The cellulosic material may be pretreated both physically (mechanically) and chemically. Mechanical or physical pretreatment may be coupled with steaming/steam explosion, hydrothermolysis, dilute or mild acid treatment, high temperature, high pressure treatment, irradiation (*e.g.,* microwave irradiation), or combinations thereof. In one embodiment, high pressure means pressure in the range of preferably about 100 to about 400 psi, *e.g.,* about 150 to about 250 psi. In another embodiment, high temperature means temperature in the range of about 100 to about 300°C, *e.g.,* about 140 to about 200°C. In a preferred embodiment, mechanical or physical pretreatment is performed in a batch-process using a steam gun hydrolyzer system that uses high pressure and high temperature as defined above, *e.g.,* a Sunds Hydrolyzer available from Sunds Defibrator AB, Sweden. The physical and chemical pretreatments may be carried out sequentially or simultaneously, as desired.

Accordingly, in a preferred embodiment, the cellulosic material is subjected to physical (mechanical) or chemical pretreatment, or any combination thereof, to promote the separation and/or release of cellulose, hemicellulose, and/or lignin.

Biological Pretreatment: The term "biological pretreatment" refers to any biological pretreatment that promotes the separation and/or release of cellulose, hemicellulose, and/or lignin from the cellulosic material. Biological pretreatment techniques may involve applying lignin-solubilizing microorganisms and/or enzymes (see, for example, Hsu, T.-A., 1996, Pretreatment of biomass, in Handbook on Bioethanol: Production and Utilization, Wyman, C. E., ed., Taylor & Francis, Washington, DC, 179-212; Ghosh and Singh, 1993, Adv. Appl. Microbiol. 39: 295-333; McMillan, J. D., 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson and Hahn-Hagerdal, 1996, Enz. Microb. Tech. 18: 312-331; and Vallander and Eriksson, 1990, Adv. Biochem. Eng./Biotechnol. 42: 63-95).

**Processes for Preconditioning Pretreated Cellulosic Material**

When lignocellulose-containing material is pretreated, degradation products that may be inhibitory to enzymes or toxic to fermenting organisms may be produced. These degradation products severely decrease both hydrolysis and fermentation processes and therefore reduction of the degradation products is desired.

The inventors have found that preconditioning pretreated lignocellulose-containing material with a combination of a phenol oxidizing enzyme and a beta-glucosidase is effective to reduce degradation products from pretreatment of lignocellulose-containing material.

As used herein "preconditioning" refers to conditioning of a cellulosic material. "Preconditioning" may also be referred to as detoxification of pretreated lignocellulosic material or to a stage of pretreatment within a multi-stage pretreatment process, after a step of pretreatment to disrupt plant cell wall components of the cellulosic material has occurred.

As used herein, "degradation products" include lignin degradation products, cellulose degradation products and hemicellulose degradation products. The pretreated lignin degradation products may be phenolics in nature. The pretreated cellulose degradation products may comprise cellodextrins, e.g., cellobiose, cellotriose, etc.

Hemicellulose degradation products include furans from sugars (such as hexoses and/or pentoses), including xylose, mannose, galactose, rhamanose, and arabinose. Examples of hemicelluloses include xylan, galactoglucomannan, arabinogalactan, arabinoglucuronoxylan, glucuronoxylan, and derivatives and combinations thereof.

Examples of inhibitory compounds, i.e., pretreated lignocellulose degradation products, include 4-OH benzyl alcohol, 4-OH benzaldehyde, 4-OH benzoic acid, trimethyl benzaldehyde, 2-furoic acid, coumaric acid, ferulic acid, phenol, guaiacol, veratrole, pyrogallollol, pyrogallol mono methyl ether, vanillyl alcohol, vanillin, isovanillin, vanillic acid, isovanillic acid, homovanillic acid, veratryl alcohol, veratraldehyde, veratric acid, 2-O-methyl gallic acid, syringyl alcohol, syringaldehyde, syringic acid, trimethyl gallic acid, homocatechol, ethyl vanillin, creosol, p-methyl anisol, anisaldehyde, anisic acid, furfural, hydroxymethylfurfural, 5-hydroxymethylfurfural, formic acid, acetic acid, levulinic acid, cinnamic acid, coniferyl aldehyde, isoeugenol, hydroquinone, eugenol or combinations thereof. Other inhibitory compounds may be found in, e.g., Luo et al., 2002, Biomass and Bioenergy 22: 125-138.

Preconditioning processes described herein are preferably carried out at a pH that is suitable for the phenol oxidizing enzyme and glycosidase. In one embodiment the pH is between 2 and 7, preferably between about 3 and about 6, especially between about 4 and about 5.5, more particularly about 5.0, 5.1, 5.2 or 5.3. In a preferred embodiment the temperature during preconditioning is a temperature suitable for the phenol oxidizing enzyme and a glycosidase. In one embodiment the temperature during preconditioning is between 20°C and 70°C, preferably between about 40°C

and about 60°C, more preferably about 50°C. In an embodiment preconditioning incubating occurs for at least 30 minutes, *e.g.,* at least about 1 hour, at least about 2 hours, at least about 3 hours, at least about 4 hours, at least about 5 hours, at least about 8 hours, at least about 12 hours, or at least about 24 hours, such as from about 30 minutes to about 24 hours.

Suitable pHs, temperatures and other process conditions can easily be determined by one skilled in the art.

By preconditioning processes described herein, hydrolysis can be significantly improved at low hydrolysis enzyme loading, such that a similar sugar concentration can be obtained from less hydrolysis enzymes than would be used in hydrolysis of non-preconditioned material. Correspondingly, use of the same enzyme loading as would be used in hydrolysis of non-preconditioned material in hydrolysis of preconditioned material, would increase production of hydrolysis products.

Furthermore, by preconditioning processes described herein, fermentation time may be reduced, such that the total time from material to fermentation product is reduced. Additionally, preconditioning processes described herein may reduce the need for a washing step after pretreatment of the lignocellulose-containing material.

Described are processes for preconditioning pretreated lignocellulose-containing material, the processes comprising incubating pretreated lignocellulose-containing material with a combination of a phenol oxidizing enzyme and a glycosidase. In particular processes for preconditioning pretreated lignocellulose-containing materialcomprises incubating pretreated lignocellulose-containing material with laccase and beta-glucosidase. The preconditioning enzymes may be added in preconditioning processes simultaneously or separately. In one embodiment the laccase and the beta-glucosidase are added at or about the same time. In another embodiment the laccase is added prior to addition of the beta-glucosidase. In yet another embodiment the beta-glucosidase is added prior to addition of the laccase.

The processes of preconditioning are conducted prior to saccharification of the cellulosic material. In an embodiment the invention relates to processes for preconditioning pretreated lignocellulose-containing material, the processes comprising incubating pretreated lignocellulose-containing material with laccase and beta-glucosidase, prior to saccharification of the pretreated lignocellulose-containing material. As demonstrated in the examples, enzymatic preconditioning of dilute acid pretreated fractionated unwashed corn fiber with a combination of laccase and beta-glucosidase before saccharifying (i.e., hydrolyzing) with a cellulolytic enzyme preparation resulted in increased glucose concentrations (*See* Example 1, Kettles R4 and R5), as compared to no preconditioning being done (*See* Example 1, Kettle R1), preconditioning with laccase alone (See Example 1, Kettle R2, R6), and preconditioning with laccase and a small amount of a cellulolytic enzyme preparation or hemicellulolytic enzyme preparation (See Example 1, Kettles R3, R7 and R8).

Example 5 demonstrates that in an enzymatic preconditioning screening trial, dose responses of laccase (liquid and granular) were evaluated on low solids unwashed pretreated corn stover. Because of the high starch content of dry fractionated corn fiber, a low dose of cellulolytic enzyme preparation may be realized. Additionally evaluated was a blend of glucoamylase to show the benefit of addition of a cellulolytic enzyme preparation.

Therefore described are processes for preconditioning pretreated lignocellulose-containing material, the processes comprising incubating pretreated lignocellulose-containing material with laccase and beta-glucosidase or a beta-glucosidase containing enzyme preparation for a period of time sufficient to precondition the pretreated lignocellulose-containing material such that the hydrolysis of the preconditioned pretreated lignocellulose-containing material results in an increased yield compared to a pretreated lignocellulose-containing material that is not so preconditioned.

The lignocellulose-containing material used in processes of the invention may be pretreated. The pretreated lignocellulose-containing material may be pretreated using any suitable method. Suitable pretreatment methods are known in the art, such as those listed in the "Pretreatment"-section herein. In a preferred embodiment the pretreated lignocellulose-containing material has been dilute acid pretreated or auto-hydrolyzed before preconditioning. In one embodiment the lignocellulose-containing material may be pretreated corn fiber, pretreated corn stover (PCS), pretreated corn cob, pretreated wheat straw, pretreated rice straw or pretreated switch grass. In a preferred embodiment the lignocellulose-containing material is dilute acid pretreated corn fiber. Other examples of contemplated material are described in the "Cellulosic Materials"-section herein.

In another embodiment the pretreated lignocellulose-containing material is unwashed. In an embodiment the pretreated lignocellulose-containing material is un-detoxified. In an embodiment the lignocellulose-containing material is washed, undetoxified or unwashed pretreated corn fiber, corn stover (PCS), corn cob, wheat straw, rice straw and/or switch grass. In an embodiment the lignocellulose-containing material is fractionated. In an embodiment the lignocellulose-containing material may be pretreated dry fractionated corn fiber. In another embodiment the lignocellulose-containing material may be pretreated dry fractionated unwashed corn fiber. In an embodiment the lignocellulose-containing material may be pretreated wet fractionated corn fiber. In an embodiment preconditioning occurs at about 5-50% TS (Total Solids), such as about 10-40% TS, such as about 15-35% TS.

## Preconditioning Enzymes

The preconditioning enzymes may be of any origin, including of mammalian, plant and microbial origin, such as bacterial and fungal origin.

Phenol oxidizing enzymes may in preferred embodiments belong to any of the following EC classes including: Catechol oxidase (EC 1.10.3.1), Laccase (EC 1.10.3.2), o-Aminophenol oxidase (1.10.3.4); and Monophenol monooxygenase (1.14.18.1). The processes of the invention comprise addition of laccase as a preconditioning enzyme. The laccase may belong to the following EC class: Laccase (EC 1.10.3.2). In a preferred embodiment the laccase is a laccase such as one from *Myceliophthora thermophila* (MtL), such as the laccase set forth as as SEQ ID NO: 2 in WO 95/33836 (SEQ ID NO: 1 herein). In an embodiment the laccase has at least 60%, at least 70% at least 80%, at least 85%, at least 90%, at least 95%, at least 97%, at least 98%, at least 99% sequence identity to the *Myceliopthora thermophila* laccase disclosed as SEQ ID NO: 2 in WO 95/33836 (SEQ ID NO: 1 herein). Other suitable laccases are mentioned in the "Laccases"-section below. In an embodiment the laccase loading is between 1-500 μg, such as 5-100 μg EP/g cellulose. In an embodiment the laccase loading is between 0.005 and 20 mg Enzyme Protein (EP)/g cellulose, such as 0.1-1 mg EP/g cellulose. In various embodiments the laccase is in liquid form, granular form or powdered form.

The glycosidase may in preferred embodiments belong to any of the following EC classes including: glucosidases (E.C. 3.2.1), beta-glucosidase (E.C.3.2.1.21), glucoamylase (E.C. 3.2.1.3) and alpha-glucosidase (E.C. 3.2.1.20). The processes of the invention comprise addition of beta-glucosidase as a preconditioning enzyme. The beta-glucosidase may be derived from a strain of *Aspergillus,* such as *Aspergillus niger, Aspergillus fumigatus,* or *Aspergillus oryzae.* In an embodiment the beta-glucosidase loading is between 0.01 and 20 mg EP/g cellulose, such as 0.1-1 mg EP/g cellulose. In an embodiment the beta-glucosidase is comprised in a beta-glucosidase containing enzyme preparation. Other suitable beta-glucosidases and beta-glucosidase containing enzyme preparations are mentioned in the "beta-glucosidase"-section below.

In various embodiments, additional enzymes may be present or included during preconditioning processes, including, but not limited to, cellulases and hemicellulases.

In embodiments of processes of the invention, preconditioning enzymes may be added separately or simultaneously.

Described are preconditioning compositions comprising laccase and beta-glucosidase.

Additional information regarding preconditioning enzymes contemplated according to methods of the invention and examples thereof may be found in the "Enzymes" section below.

**Producing Hydrolysis Products from Preconditioned Pretreated Cellulosic Material**

The invention relates to processes for producing a hydrolysis product from pretreated lignocellulose-containing material, the process comprising:

(i) preconditioning a pretreated lignocellulose-containing material, the preconditioning comprising incubating the pretreated lignocellulose-containing material with laccase and beta-glucosidase; and
(ii) hydrolyzing the preconditioned lignocellulose-containing material with a cellulolytic enzyme composition to obtain a hydrolysis product, wherein preconditioning and hydrolysis are carried out sequentially.

Suitable lignocellulose-containing material and methods or processes of pretreating and preconditioning such materials according to embodiments of the invention are described more fully herein. In one embodiment the preconditioning comprises incubating the pretreated lignocellulose-containing material with a combination of a phenol oxidizing enzyme and a glycosidase. In a particular embodiment the phenol oxidizing enzyme is a laccase, such as one from *Myceliophthora thermophila* (MtL) (WO 95/33836 (SEQ ID NO: 1 herein)) and the glycosidase is a beta-glucosidase or a beta-glucosidase containing enzyme preparation. In an embodiment, preconditioning step (i) is carried out in accordance with preconditioning processes described herein.

In sequential processes, the preconditioning step (i) is completed prior to the initiation of the hydrolyzing step (ii). Completion of the preconditioning step may be determined by depletion of the preconditioning enzyme, use of a predetermined amount of preconditioning enzyme, preconditioning of all substrate, and/or preconditioning of a predetermined amount of substrate.

In an embodiment where the preconditioning step (i) of sequential processes contains enzymes with activity in the conversion of cellulosic material to fermentable sugars, e.g., cellulases and hemicellulases, such enzymes are present such that only a small portion of the material is converted and that all, or essentially all of the material remains unconverted until the hydrolysis step (ii) is performed. Further, in an embodiment where the preconditioning step (i) of sequential processes contains enzymes with activity in the conversion of cellulosic material to fermentable sugars, e.g., cellulases and hemicellulases, such enzymes are present in an amount sufficient to provide a synergistic effect with regard to generation of free radicals from lignin derivatives and to create more accessible cellulose as compared to a preconditioning step without addition of such enzymes.

**Hydrolysis (Saccharification)**

In the hydrolysis step (*i.e.,* saccharification step) the pretreated preconditioned lignocellulose-containing material is hydrolyzed to break down cellulose and/or hemicellulose to fermentable sugars, such as glucose, cellobiose, xylose, xylulose, arabinose, mannose, galactose, and/or soluble oligosaccharides. The saccharification is performed enzymatically using a cellulolytic enzyme preparation.

Enzymatic hydrolysis (*i.e.,* saccharification) may be carried out in a suitable aqueous environment under conditions that may be readily determined by one skilled in the art. In one embodiment, hydrolysis is performed under conditions suitable for the activity of the cellulolytic enzyme preparation, preferably optimal for the cellulolytic enzyme preparation. The hydrolysis may be carried out as a fed batch or continuous process where the preconditioned unwashed pretreated lignocellulose-containing material (substrate) is fed gradually to, for example, an enzyme containing hydrolysis solution.

The hydrolysis is generally performed in stirred-tank reactors or fermentors under controlled pH, temperature, and mixing conditions. Suitable process time, temperature and pH conditions may readily be determined by one skilled in the art. For example, the hydrolysis may last up to 200 hours, but is typically performed for preferably about 12 to about 120 hours, e.g., about 16 to about 72 hours or about 24 to about 48 hours. In an embodiment hydrolysis is performed for at least 5 days.

The temperature is in the range of preferably about 25°C to about 70°C, e.g., about 30°C to about 65°C, about 40°C to about 60°C, or about 50°C to about 55°C. The pH is in the range of preferably about 3 to about 8, e.g., about 3.5 to about 7, about 4 to about 6, or about 4.5 to about 5.5. The dry solids content is in the range of preferably about 5 to about 50 wt. %, e.g., about 10 to about 40 wt. % or about 20 to about 30 wt. %.

The enzymes used in hydrolysis may comprise any protein useful in degrading the cellulosic material.

In an embodiment, sugars obtained from hydrolysis step (ii), i.e., a "hydrolysis product," may be fermented.

In an embodiment, hydrolysis is carried out in the presence of one or more (e.g., several) proteins selected from the group consisting of a cellulase, an AA9 polypeptide, a hemicellulase, an esterase, an expansin, laccase, a ligninolytic enzyme, an oxidoreductase, a pectinase, a protease, and a swollenin. In another embodiment, the cellulase is preferably one or more (*e.g.,* several) enzymes selected from the group consisting of an endoglucanase, a cellobiohydrolase, and a beta-glucosidase. In another embodiment, the hemicellulase is preferably one or more (*e.g.,* several) enzymes selected from the group consisting of an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. In another embodiment, the oxidoreductase is preferably one or more (*e.g.,* several) enzymes selected from the group consisting of a catalase, a laccase, and a peroxidase. The compositions may also comprise one or more (*e.g.,* several) enzymes selected from the group consisting of a hydrolase, an isomerase, a ligase, a lyase, an oxidoreductase, or a transferase, *e.g.,* an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

In a further embodiment the hydrolysis is performed in the presence of a glucoamylase. The glucoamylase may be derived from *Talaromyces* (e.g., *Talaromyces emersonii*), *Trametes* (e.g., *Trametes cingulate*) or *Rhizopus* (e.g., *Rhizopus oryzae*).

In an embodiment the cellulolytic enzyme composition used in hydrolysis may be of fungal origin. In a preferred embodiment the cellulolytic enzyme composition is derived from *Trichoderma* (*e.g., Trichoderma reesei*). In a preferred embodiment hydrolysis (saccharification) is carried out in the presence of a cellulolytic enzyme preparation including enzyme activities selected from the group of cellulase, endoglucanase, cellobiohydrolase, and beta-glucosidase *(e.g., Aspergillus fumigatus* or *Aspergillus oryzae* beta-glucosidase). In a preferred embodiment hydrolysis is carried out using a polypeptide having cellulolytic enhancing activity (*e.g.,* a *Thermoascus aurantiacus* or *Penicillium emersonii* cellulolytic enhancing polypeptide).

In an embodiment the cellulolytic enzyme preparation used for hydrolysis is of fungal origin, such as derived from *Trichoderma* (*e.g., Trichoderma reesei*). A hemicellulase may also be present or added during hydrolysis. In an embodiment hydrolysis is carried out in the presence of a cellulolytic enzyme preparation including enzyme activities selected from the group of endoglucanase, cellobiohydrolase, and beta-glucosidase (*e.g., Aspergillus fumigatus* beta-glucosidase, such as the one shown in WO 2005/047499 or WO 2012/044915 (SEQ ID NO: 2 herein), or *Aspergillus oryzae* beta-glucosidase). In an embodiment hydrolysis is carried out in the presence of a polypeptide having cellulolytic enhancing activity, such as GH61 polypeptide, *e.g.,* a *Thermoascus aurantiacus* GH61 polypeptide, such as the one shown in WO 2005/074656 or *Penicillium emersonii* GH61 polypeptide, such as the one shown in WO 2011/041397 (SEQ ID NO: 3 herein).

In an embodiment the hemicellulase may be a xylanase (*e.g.,* an *Aspergillus aculeatus,* such as the one shown in WO 94/021785 or *Aspergillus fumigatus* xylanase, such as the one shown in WO 2006/078256 (SEQ ID NO: 6 herein)), or a xylosidase *(e.g., Aspergillus fumigatus* beta-xylosidase, such as the one shown in WO 2011/057140 (SEQ ID NO: 7 herein)).

In an embodiment, a hemicellulase, such as a hemicelluloytic enzyme preparation, is present or added during hydrolysis (and/or fermentation). In a preferred embodiment the hemicellulolytic enzyme preparation comprises a cellulolytic enzyme preparation from *Trichoderma reesei,* further comprising *Aspergillus fumigatus* xylanase (WO 2006/078256, SEQ ID NO: 6 herein) and *Aspergillus fumigatus* beta-xylosidase (WO 2011/057140, SEQ ID NO: 7 herein).

In a preferred embodiment, hydrolysis is carried out in the presence of a cellulolytic enzyme preparation derived from *Trichoderma* (*e.g., Trichoderma reesei*) including endoglucanase (EG) and cellobiohydrolase (CBH) further comprises a polypeptide having cellulolytic enhancing activity (*e.g.,* a *Thermoascus aurantiacus* or *Penicillium emersonii* cellulolytic enhancing polypeptide) and a beta-glucosidase *(e.g., Aspergillus fumigatus* or *Aspergillus oryzae* beta-glucosidase).

In a preferred embodiment the cellulolytic enzyme preparation, present or added during hydrolysis, is a cellulolytic enzyme preparation derived from *Trichoderma reesei* further comprising AA9 (GH61A) polypeptide having cellulolytic enhancing activity derived from a strain of *Penicillium emersonii* (SEQ ID NO: 2 in WO 2011/041397, SEQ ID NO: 3 herein), *Aspergillus fumigatus* beta-glucosidase (SEQ ID NO: 2 in WO 2005/047499, SEQ ID NO: 2 herein) variant F100D, S283G, N456E, F512Y) disclosed in WO 2012/044915; *Aspergillus fumigatus* Cel7A CBH1 disclosed as SEQ ID NO: 6 in WO2011/057140 (SEQ ID NO: 4 herein) and *Aspergillus fumigatus* CBH II disclosed as SEQ ID NO: 18 in WO 2011/057140 (SEQ ID NO: 5 herein). Further, the cellulolytic enzyme preparation may further be supplemented with 10% hemicellulolytic enzyme preparation comprising a cellulolytic enzyme preparation from *Trichoderma reesei* further comprising *Aspergillus fumigatus* xylanase (WO 2006/078256) (SEQ ID NO: 6 herein) and *Aspergillus fumigatus* beta-xylosidase (WO 2011/041397) (SEQ ID NO: 7 herein).

In the processes of the present invention, the enzyme(s) may be added prior to or during hydrolysis, hydrolysis and fermentation, or fermentation.

One or more (*e.g.,* several) components of the enzyme composition may be native proteins, recombinant proteins, or a combination of native proteins and recombinant proteins. For example, one or more (*e.g.,* several) components may be native proteins of a cell, which is used as a host cell to express recombinantly one or more (*e.g.,* several) other components of the enzyme composition. It is understood herein that the recombinant proteins may be heterologous (*e.g.,* foreign) and/or native to the host cell. One or more (*e.g.,* several) components of the enzyme composition may be produced as monocomponents, which are then combined to form the enzyme composition. The enzyme composition may be a combination of multicomponent and monocomponent protein preparations.

The enzymes used in the processes of the present invention may be in any form suitable for use, such as, for example, a fermentation broth formulation or a cell composition, a cell lysate with or without cellular debris, a semi-purified or purified enzyme preparation, or a host cell as a source of the enzymes. The enzyme composition may be a dry powder or granulate, a non-dusting granulate, a liquid, a stabilized liquid, or a stabilized protected enzyme. Liquid enzyme preparations may, for instance, be stabilized by adding stabilizers such as a sugar, a sugar alcohol or another polyol, and/or lactic acid or another organic acid according to established processes.

The optimum amounts of the enzymes and polypeptides depend on several factors including, but not limited to, the mixture of cellulolytic enzymes and/or hemicellulolytic enzymes, the cellulosic material, the concentration of cellulosic material, the pretreatment of the cellulosic material, temperature, time, pH, and inclusion of a fermenting organism (*e.g.,* for Simultaneous Saccharification and Fermentation).

In one embodiment, an effective amount of cellulolytic or hemicellulolytic enzyme to the cellulosic material is about 0.5 to about 50 mg, e.g., about 0.5 to about 40 mg, about 0.5 to about 25 mg, about 0.75 to about 20 mg, about 0.75 to about 15 mg, about 0.5 to about 10 mg, or about 2.5 to about 10 mg per g of the cellulosic material.

The polypeptides having cellulolytic enzyme activity or hemicellulolytic enzyme activity as well as other proteins/polypeptides useful in the degradation of the cellulosic material, *e.g.,* AA9 polypeptides, may be derived or obtained from any suitable origin, including, archaeal, bacterial, fungal, yeast, plant, or animal origin. The term "obtained" also means herein that the enzyme may have been produced recombinantly in a host organism employing methods described herein, wherein the recombinantly produced enzyme is either native or foreign to the host organism or has a modified amino acid sequence, *e.g.,* having one or more (*e.g.,* several) amino acids that are deleted, inserted and/or substituted, *i.e.,* a recombinantly produced enzyme that is a mutant and/or a fragment of a native amino acid sequence or an enzyme produced by nucleic acid shuffling processes known in the art. Encompassed within the meaning of a native enzyme are natural variants and within the meaning of a foreign enzyme are variants obtained by, *e.g.,* site-directed mutagenesis or shuffling.

Each polypeptide may be a bacterial polypeptide. For example, each polypeptide may be a Gram-positive bacterial polypeptide having enzyme activity, or a Gram-negative bacterial polypeptide having enzyme activity.

Each polypeptide may also be a fungal polypeptide, *e.g.,* a yeast polypeptide or a filamentous fungal polypeptide.

Chemically modified or protein engineered mutants of polypeptides may also be used.

One or more (*e.g.,* several) components of the enzyme composition may be a recombinant component, *i.e.,* produced by cloning of a DNA sequence encoding the single component and subsequent cell transformed with the DNA sequence and expressed in a host (see, for example, WO 91/17243 and WO 91/17244). The host may be a heterologous host (enzyme is foreign to host), but the host may under certain conditions also be a homologous host (enzyme is native to host). Monocomponent cellulolytic proteins may also be prepared by purifying such a protein from a fermentation broth.

In one embodiment, the one or more (*e.g.,* several) cellulolytic enzymes comprise a commercial cellulolytic enzyme preparation. Examples of commercial cellulolytic enzyme preparations suitable for use in the present invention include, for example, CELLIC® CTec (Novozymes A/S), CELLIC® CTec2 (Novozymes A/S), CELLIC® CTec3 (Novozymes A/S), CELLUCLAST® (Novozymes A/S), NOVOZYM® 188 (Novozymes A/S), SPEZYME® CP (Genencor Int.), ACCELLERASE® TRIO (Danisco US Inc.), FILTRASE™ NL (DSM); METHAPLUS® S/L 100 (DSM), ROHAMENT® 7069 W (Röhm GmbH), or ALTERNAFUEL® CMAX3 (Dyadic International, Inc.). The cellulolytic enzyme preparation is added in an amount effective from about 0.001 to about 5.0 wt. % of solids, *e.g.,* about 0.025 to about 4.0 wt. % of solids or about 0.005 to about 2.0 wt. % of solids.

Examples of bacterial endoglucanases that may be used in the processes of the present invention, include, but are not limited to, *Acidothermus cellulolyticus* endoglucanase (WO 91/05039; WO 93/15186; U.S. Patent No. 5,275,944; WO 96/02551; U.S. Patent No. 5,536,655; WO 00/70031; WO 05/093050), *Erwinia carotovara* endoglucanase (Saarilahti et al., 1990, Gene 90: 9-14), *Thermobifida fusca* endoglucanase III (WO 05/093050), and *Thermobifida fusca* endoglucanase V (WO 05/093050).

Examples of fungal endoglucanases that may be used in the present invention, include, but are not limited to, *Trichoderma reesei* endoglucanase I (Penttila et al., 1986, Gene 45: 253-263, *Trichoderma reesei* Cel7B endoglucanase I (GenBank:M15665), *Trichoderma reesei* endoglucanase II (Saloheimo et al., 1988, Gene 63:11-22), *Trichoderma reesei* Cel5A endoglucanase II (GenBank:M19373), *Trichoderma reesei* endoglucanase III (Okada et al., 1988, Appl. Environ. Microbiol. 64: 555-563, GenBank:AB003694), *Trichoderma reesei* endoglucanase V (Saloheimo et al., 1994, Molecular Microbiology 13: 219-228, GenBank:Z33381), *Aspergillus aculeatus* endoglucanase (Ooi et al., 1990, Nucleic Acids Research 18: 5884), *Aspergillus kawachii* endoglucanase (Sakamoto et al., 1995, Current Genetics 27: 435-439), *Fusarium oxysporum* endoglucanase (GenBank:L29381), *Humicola grisea* var. *thermoidea* endoglucanase (GenBank:AB003107), *Melanocarpus albomyces* endoglucanase (GenBank:MAL515703), *Neurospora crassa* endoglucanase (GenBank:XM_324477), *Humicola insolens* endoglucanase V, *Myceliophthora thermophila* CBS 117.65 endoglucanase, *Thermoascus aurantiacus* endoglucanase I (GenBank:AF487830) and *Trichoderma reesei* strain No. VTT-D-80133 endoglucanase (GenBank:M15665).

Examples of cellobiohydrolases useful in the present invention include, but are not limited to, *Aspergillus aculeatus* cellobiohydrolase II (WO 2011/059740), *Chaetomium thermophilum* cellobiohydrolase I, *Chaetomium thermophilum* cellobiohydrolase II, *Humicola insolens* cellobiohydrolase I, *Myceliophthora thermophila* cellobiohydrolase II (WO 2009/042871), *Penicillium occitanis* cellobiohydrolase I (GenBank:AY690482), *Talaromyces emersonii* cellobiohydrolase I (GenBank:AF439936), *Thielavia hyrcanie* cellobiohydrolase II (WO 2010/141325), *Thielavia terrestris* cellobiohydrolase II (CEL6A, WO 2006/074435), *Trichoderma reesei* cellobiohydrolase I, *Trichoderma reesei* cellobiohydrolase II, and *Trichophaea saccata* cellobiohydrolase II (WO 2010/057086).

Examples of beta-glucosidases useful in the present invention include, but are not limited to, beta-glucosidases from *Aspergillus aculeatus* (Kawaguchi et al., 1996, Gene 173: 287-288), *Aspergillus fumigatus* (WO 2005/047499), *Aspergillus niger* (Dan et al., 2000, J. Biol. Chem. 275: 4973-4980), *Aspergillus oryzae* (WO 02/095014), *Penicillium brasilianum* IBT 20888 (WO 2007/019442 and WO 2010/088387), *Thielavia terrestris* (WO 2011/035029), and *Trichophaea saccata* (WO 2007/019442).

Other useful endoglucanases, cellobiohydrolases, and beta-glucosidases are disclosed in numerous Glycosyl Hydrolase families using the classification according to Henrissat, 1991, Biochem. J. 280: 309-316, and Henrissat and Bairoch, 1996, Biochem. J. 316: 695-696.

In the processes of the present invention, any AA9 polypeptide may be used as a component of the enzyme composition.

Examples of AA9 polypeptides useful in the processes of the present invention include, but are not limited to, AA9 polypeptides from *Thielavia terrestris* (WO 2005/074647, WO 2008/148131, and WO 2011/035027), *Thermoascus aurantiacus* (WO 2005/074656 and WO 2010/065830), *Trichoderma reesei* (WO 2007/089290), *Myceliophthora thermophila* (WO 2009/085935, WO 2009/085859, WO 2009/085864, and WO 2009/085868), *Aspergillus fumigatus* (WO 2010/138754), *Penicillium pinophilum* (WO 2011/005867), *Thermoascus* sp. (WO 2011/039319), *Penicillium* sp. (WO 2011/041397), *Thermoascus crustaceous* (WO 2011/041504), *Aspergillus aculeatus* (WO 2012/125925), *Thermomyces lanuginosus* (WO 2012/113340, WO 12/129699, and WO 2012/130964), *Aurantiporus alborubescens* (WO 2012/122477), *Trichophaea saccata* (WO 2012/122477), *Penicillium thomii* (WO 2012/122477), *Talaromyces stipitatus* (WO 2012/135659), *Humicola insolens* (WO 2012/146171), *Malbranchea cinnamomea* (WO 2012/101206), *Talaromycesleycettanus* (WO 2012/101206), and *Chaetomium thermophilum* (WO 2012/101206).

In one embodiment, the AA9 polypeptide is used in the presence of a soluble activating divalent metal cation according to WO 2008/151043, *e.g.,* manganese or copper.

In another embodiment, the AA9 polypeptide is used in the presence of a dioxy compound, a bicylic compound, a heterocyclic compound, a nitrogen-containing compound, a quinone compound, a sulfur-containing compound, or a liquor obtained from a pretreated cellulosic material such as pretreated corn stover (WO 2012/021394, WO 2012/021395, WO 2012/021396, WO 2012/021399, WO 2012/021400, WO 2012/021401, WO 2012/021408, and WO 2012/021410).

In one embodiment, such a compound is added at a molar ratio of the compound to glucosyl units of cellulose of about $10^{-6}$ to about 10, *e.g.,* about $10^{-6}$ to about 7.5, about $10^{-6}$ to about 5, about $10^{-6}$ to about 2.5, about $10^{-6}$ to about 1, about $10^{-5}$ to about 1, about $10^{-5}$ to about $10^{-1}$, about $10^{-4}$ to about $10^{-1}$, about $10^{-3}$ to about $10^{-1}$, or about $10^{-3}$ to about $10^{-2}$. In another embodiment, an effective amount of such a compound is about 0.1 μM to about 1 M, *e.g.,* about 0.5 μM to about 0.75 M, about 0.75 μM to about 0.5 M, about 1 μM to about 0.25 M, about 1 μM to about 0.1 M, about 5 μM to about 50 mM, about 10 μM to about 25 mM, about 50 μM to about 25 mM, about 10 μM to about 10 mM, about 5 μM to about 5 mM, or about 0.1 mM to about 1 mM.

In one embodiment, the one or more (e.g., several) hemicellulolytic enzymes comprise a commercial hemicellulolytic enzyme preparation. Examples of commercial hemicellulolytic enzyme preparations suitable for use in the present invention include, for example, SHEARZYME™ (Novozymes A/S), CELLIC® HTec (Novozymes A/S), CELLIC® HTec2 (Novozymes A/S), CELLIC® HTec3 (Novozymes A/S), VISCOZYME® (Novozymes A/S), ULTRAFLO® (Novozymes A/S), PULPZYME® HC (Novozymes A/S), MULTIFECT® Xylanase (Genencor), ACCELLERASE® XY (Genencor), ACCELLERASE® XC (Genencor), ECOPULP® TX-200A (AB Enzymes), HSP 6000 Xylanase (DSM), DEPOL™ 333P (Biocatalysts Limit, Wales, UK), DEPOL™ 740L. (Biocatalysts Limit, Wales, UK), and DEPOL™ 762P (Biocatalysts Limit, Wales, UK), ALTERNA FUEL 100P (Dyadic), and ALTERNA FUEL 200P (Dyadic).

Examples of xylanases useful in the processes of the present invention include, but are not limited to, xylanases from *Aspergillus aculeatus* (GeneSeqP:AAR63790; WO 94/21785), *Aspergillus fumigatus* (WO 2006/078256), *Penicillium pinophilum* (WO 2011/041405), *Penicillium* sp. (WO 2010/126772), *Talaromyces lanuginosus* GH11 (WO 2012/130965), *Talaromyces thermophilus* GH11 (WO 2012/13095), *Thielavia terrestris* NRRL 8126 (WO 2009/079210), and *Trichophaea saccata* GH10 (WO 2011/057083).

Examples of beta-xylosidases useful in the processes of the present invention include, but are not limited to, beta-xylosidases from *Neurospora crassa* (SwissProt:Q7SOW4), *Trichoderma reesei* (UniProtKB/TrEMBL:Q92458), *Talaromyces emersonii* (SwissProt:Q8X212), and *Talaromyces thermophilus* GH11 (WO 2012/13095).

Examples of acetylxylan esterases useful in the processes of the present invention include, but are not limited to, acetylxylan esterases from *Aspergillus aculeatus* (WO 2010/108918), *Chaetomium globosum* (UniProt:Q2GWX4), *Chaetomium gracile* (GeneSeqP:AAB82124), *Humicola insolens* DSM 1800 (WO 2009/073709), *Hypocrea jecorina* (WO 2005/001036), *Myceliophtera thermophila* (WO 2010/014880), *Neurospora crassa* (UniProt:q7s259), *Phaeosphaeria nodorum* (UniProt:Q0UHJ1), and *Thielavia terrestris* NRRL 8126 (WO 2009/042846).

Examples of feruloyl esterases (ferulic acid esterases) useful in the processes of the present invention include, but are not limited to, feruloyl esterases form *Humicola insolens* DSM 1800 (WO 2009/076122), *Neosartorya fischeri* (UniProt:A1D9T4), *Neurospora crassa* (UniProt:Q9HGR3), *Penicillium aurantiogriseum* (WO 2009/127729), and *Thielavia terrestris* (WO 2010/053838 and WO 2010/065448).

Examples of arabinofuranosidases useful in the processes of the present invention include, but are not limited to, arabinofuranosidases from *Aspergillus niger* (GeneSeqP:AAR94170), *Humicola insolens* DSM 1800 (WO 2006/114094 and WO 2009/073383), and *M. giganteus* (WO 2006/114094).

Examples of alpha-glucuronidases useful in the processes of the present invention include, but are not limited to, alpha-glucuronidases from *Aspergillus clavatus* (UniProt:alcc12), *Aspergillus fumigatus* (SwissProt:Q4WW45), *Aspergillus niger* (UniProt:Q96WX9), *Aspergillus terreus* (SwissProt:Q0CJP9), *Humicola insolens* (WO 2010/014706), *Penicillium aurantiogriseum* (WO 2009/068565), *Talaromyces emersonii* (UniProt:Q8X211), and *Trichoderma reesei* (UniProt:Q99024).

Examples of oxidoreductases useful in the processes of the present invention include, but are not limited to, *Aspergillus lentilus* catalase, *Aspergillus fumigatus* catalase, *Aspergillus niger* catalase, *Aspergillus oryzae* catalase, *Humicola insolens* catalase, *Neurospora crassa* catalase, *Penicillium emersonii* catalase, *Scytalidium thermophilum* catalase, *Talaromyces stipitatus* catalase, *Thermoascus aurantiacus* catalase, *Coprinus cinereus* laccase, *Myceliophthora thermophila* laccase, *Polyporus pinsitus* laccase, *Pycnoporus cinnabarinus* laccase, *Rhizoctonia solani* laccase, *Streptomyces coelicolor* laccase, *Coprinus cinereus* peroxidase, Soy peroxidase, Royal palm peroxidase.

The polypeptides having enzyme activity used in the processes of the present invention may be produced by fermentation of the above-noted microbial strains on a nutrient medium containing suitable carbon and nitrogen sources and inorganic salts, using procedures known in the art (see, *e.g.,* Bennett, J.W. and LaSure, L. (eds.), More Gene Manipulations in Fungi, Academic Press, CA, 1991). Suitable media are available from commercial suppliers or may be prepared according to published compositions (*e.g.,* in catalogues of the American Type Culture Collection). Temperature ranges and other conditions suitable for growth and enzyme production are known in the art (see, *e.g.,* Bailey, J.E., and

Ollis, D.F., Biochemical Engineering Fundamentals, McGraw-Hill Book Company, NY, 1986).

The fermentation may be any method of cultivation of a cell resulting in the expression or isolation of an enzyme or protein. Fermentation may, therefore, be understood as comprising shake flask cultivation, or small-scale or large-scale fermentation (including continuous, batch, fed-batch, or solid state fermentations) in laboratory or industrial fermentors performed in a suitable medium and under conditions allowing the enzyme to be expressed or isolated. The resulting enzymes produced by the methods described above may be recovered from the fermentation medium and purified by conventional procedures.

### Producing Fermentation Products From Preconditioned Pretreated Cellulosic Material

In another embodiment, the invention relates to processes for producing a fermentation product from pretreated lignocellulose-containing material, the process comprising:

(i) preconditioning a pretreated lignocellulose-containing material, the preconditioning comprising incubating the pretreated lignocellulose-containing material with laccase and beta-glucosidase;

(i) hydrolyzing the preconditioned lignocellulose-containing material with a cellulolytic enzyme composition to obtain a hydrolysis product; and

fermenting the hydrolysis product to obtain a fermentation product, wherein preconditioning and hydrolysis are carried out sequentially.

Suitable lignocellulose-containing material and methods or processes of pretreating and preconditioning such materials according to embodiments of the invention are described more fully herein. In one embodiment the preconditioning comprises incubating the pretreated lignocellulose-containing material with a combination of a phenol oxidizing enzyme and a glycosidase. In a particular embodiment the phenol oxidizing enzyme is a laccase, such as one from *Myceliophthora thermophila* (MtL) (WO 95/33836 (SEQ ID NO: 1 herein) and the glycosidase is a beta-glucosidase or a beta-glucosidase containing enzyme preparation. In an embodiment, preconditioning step (i) is carried out in accordance with preconditioning processes described herein.

In an embodiment the fermentation product produced is an alcohol (*e.g.,* ethanol or butanol), an organic acid, a ketone, an amino acid, or a gas.

In an embodiment the fermentation product is an alcohol (*e.g.,* ethanol or butanol), an organic acid, a ketone, an amino acid, or a gas. In a preferred embodiment the fermentation product is ethanol. In an embodiment the fermentation product is recovered after fermentation in step (iii). Processes described herein may result in an increased hydrolysis yield and/or increased rate of hydrolysis, compared to when no preconditioning is done. Also, processes described herein may result in an increased fermentation yield compared to when no preconditioning is done.

### Fermentation

The fermentable sugars obtained from the hydrolyzed cellulosic material may be fermented by one or more (*e.g.,* several) fermenting microorganisms capable of fermenting the sugars directly or indirectly into a desired fermentation product.

"Fermentation" or "fermentation process" refers to any fermentation process or any process comprising a fermentation step. Fermentation processes also include fermentation processes used in the consumable alcohol industry (*e.g.,* beer and wine), dairy industry (*e.g.,* fermented dairy products), leather industry, and tobacco industry. The fermentation conditions depend on the desired fermentation product and fermenting organism and may easily be determined by one skilled in the art.

In the fermentation step, sugars, released from the cellulosic material as a result of the pretreatment and enzymatic hydrolysis steps, are fermented to a product, *e.g.,* ethanol, by a fermenting organism, such as yeast. Hydrolysis (saccharification) and fermentation may be separate or simultaneous.

Saccharification (hydrolysis) and fermentation, separate or simultaneous, include, but are not limited to, separate saccharification (hydrolysis) and fermentation (SHF); simultaneous saccharification and fermentation (SSF); simultaneous saccharification and cofermentation (SSCF); hybrid hydrolysis and fermentation (HHF); separate hydrolysis and co-fermentation (SHCF); hybrid hydrolysis and co-fermentation (HHCF); and direct microbial conversion (DMC). SHF uses separate process steps to first saccharify (hydrolyze) cellulosic material to fermentable sugars, *e.g.,* glucose, cellobiose, cellotriose, and pentose sugars, and then ferment the fermentable sugars to ethanol. In SSF, the enzymatic hydrolysis of cellulosic material and the fermentation of sugars to ethanol are combined in one step (Philippidis, G. P., 1996, Cellulose bioconversion technology, in Handbook on Bioethanol: Production and Utilization, Wyman, C. E., ed., Taylor & Francis, Washington, DC, 179-212). SSCF involves the cofermentation of multiple sugars (Sheehan, J., and Himmel,

M., 1999, Enzymes, energy and the environment: A strategic perspective on the U.S. Department of Energy's research and development activities for bioethanol, Biotechnol. Prog. 15: 817-827). HHF involves a separate hydrolysis step, and in addition a simultaneous saccharification and hydrolysis step, which may be carried out in the same reactor. The steps in an HHF process may be carried out at different temperatures, *i.e.,* high temperature enzymatic saccharification followed by SSF at a lower temperature that the fermentation strain can tolerate. DMC combines all three processes (enzyme production, hydrolysis, and fermentation) in one or more (several) steps where the same organism is used to produce the enzymes for conversion of the cellulosic material to fermentable sugars and to convert the fermentable sugars into a final product (Lynd et al., 2002, Microbial cellulose utilization: Fundamentals and biotechnology, Microbiol. Mol. Biol. Reviews 66: 506-577). It is understood herein that any method known in the art comprising pretreatment, enzymatic hydrolysis (saccharification), fermentation, or a combination thereof, may be used in the practicing methods and processes of the present invention.

Any suitable hydrolyzed cellulosic material may be used in the fermentation step in practicing the present invention. The material is generally selected based on economics, *i.e.,* costs per equivalent sugar potential, and recalcitrance to enzymatic conversion.

The term "fermentation medium" is understood herein to refer to a medium before the fermenting microorganism(s) is (are) added, such as, a medium resulting from a saccharification process, as well as a medium used in a simultaneous saccharification and fermentation process (SSF).

Suitable fermenting organisms used according of processes of the invention are described below in the "Fermenting Organism"-section below

Fermenting Organism

"Fermenting organism" or "fermenting microorganism" refers to any microorganism, including bacterial and fungal organisms, suitable for use in a desired fermentation process to produce a fermentation product. The fermenting organism may be hexose (*i.e.,* $C_6$) and/or pentose ($C_5$) fermenting organisms, or a combination thereof. Both hexose and pentose fermenting organisms are well known in the art. Suitable fermenting organisms are able to ferment, *i.e.,* convert, sugars, such as glucose, xylose, xylulose, arabinose, maltose, mannose, galactose, and/or oligosaccharides, directly or indirectly into the desired fermentation product.

Examples of bacterial and fungal fermenting organisms producing ethanol are described by Lin et al., 2006, Appl. Microbiol. Biotechnol. 69: 627-642.

Examples of fermenting microorganisms that can ferment $C_6$ sugars include bacterial and fungal organisms, such as yeast. Yeast include strains of *Candida, Kluyveromyces,* and *Saccharomyces, e.g., Candida sonorensis, Kluyveromyces marxianus,* and *Saccharomyces cerevisiae.* Preferred yeast includes strains of the *Saccharomyces* spp., preferably *Saccharomyces cerevisiae.*

Examples of fermenting organisms that can ferment $C_5$ sugars include bacterial and fungal organisms, such as yeast. Preferred $C_5$ fermenting yeast include strains of *Pichia,* preferably *Pichia stipitis,* such as *Pichia stipitis* CBS 5773; strains of *Candida,* preferably *Candida boidinii, Candida brassicae, Candida sheatae, Candida diddensii, Candida pseudotropicalis,* or *Candida utilis.* Organisms not capable of fermenting pentose sugars, such as xylose and arabinose, may be genetically modified to do so by methods known in the art.

Examples of bacteria that can efficiently ferment hexose and pentose to ethanol include, for example, *Bacillus coagulans, Clostridium acetobutylicum, Clostridium thermocellum, Clostridium phytofermentans, Geobacillus* sp., *Thermoanaerobacter saccharolyticum,* and *Zymomonas mobilis* (Philippidis, 1996, *supra).*

Other fermenting organisms include strains of *Bacillus,* such as *Bacillus coagulans*; *Candida,* such as *C. sonorensis, C. methanosorbosa, C. diddensiae, C. parapsilosis, C. naedodendra, C. blankii, C. entomophilia, C. brassicae, C. pseudotropicalis, C. boidinii, C. utilis,* and *C. scehatae; Clostridium,* such as *C. acetobutylicum, C. thermocellum,* and *C. phytofermentans; E. coli,* especially *E. coli* strains that have been genetically modified to improve the yield of ethanol; *Geobacillus* sp.; *Hansenula,* such as *Hansenula anomala*; *Klebsiella,* such as *K. oxytoca; Kluyveromyces,* such as *K. marxianus, K. lactis, K. thermotolerans,* and *K. fragilis*; *Schizosaccharomyces,* such as *S. pombe*; *Thermoanaerobacter,* such as *Thermoanaerobacter saccharolyticum*; and *Zymomonas,* such as *Zymomonas mobilis.*

Commercially available yeast suitable for ethanol production include, *e.g.,* BIO-FERM® AFT and XR, ETHANOL RED® yeast, FALI®, FERMIOL®, GERT STRAND™ (Gert Strand AB, Sweden), SUPERSTART™ and THERMOSACC® fresh yeast.

In one embodiment, the fermenting organism has been genetically modified to provide the ability to ferment pentose sugars, such as xylose utilizing, arabinose utilizing, and xylose and arabinose co-utilizing microorganisms.

The cloning of heterologous genes into various fermenting microorganisms has led to the construction of organisms capable of converting hexoses and pentoses to ethanol (cofermentation) (Chen and Ho, 1993, Appl. Biochem. Biotechnol. 39-40: 135-147; Ho et al., 1998, Appl. Environ. Microbiol. 64: 1852-1859; Kotter and Ciriacy, 1993, Appl. Microbiol. Biotechnol. 38: 776-783; Walfridsson et al., 1995, Appl. Environ. Microbiol. 61: 4184-4190; Kuyper et al., 2004,

FEMS Yeast Research 4: 655-664; Beall et al., 1991, Biotech. Bioeng. 38: 296-303; Ingram et al., 1998, Biotechnol. Bioeng. 58: 204-214; Zhang et al., 1995, Science 267: 240-243; Deanda et al., 1996, Appl. Environ. Microbiol. 62: 4465-4470; WO 03/062430).

It is well known in the art that the organisms described above may also be used to produce other substances, as described herein.

The fermenting organism is typically added to the degraded cellulosic material or hydrolysate and the fermentation is performed for about 8 to about 96 hours, such as about 24 to about 60 hours. The temperature is typically between about 26°C to about 60°C, in particular about 32°C or 50°C, and at about pH 3 to about pH 8, such as around pH 4-5, 6, or 7.

In one embodiment, the yeast and/or another microorganism are applied to the degraded cellulosic material and the fermentation is performed for about 12 to about 96 hours, such as typically 24-60 hours. In another embodiment, the temperature is preferably between about 20°C to about 60°C, *e.g.,* about 25°C to about 50°C, about 32°C to about 50°C, or about 32°C to about 50°C, and the pH is generally from about pH 3 to about pH 7, *e.g.,* about pH 4 to about pH 7. However, some fermenting organisms, *e.g.,* bacteria, have higher fermentation temperature optima. Yeast or another microorganism is preferably applied in amounts of approximately $10^5$ to $10^{12}$, preferably from approximately $10^7$ to $10^{10}$, especially approximately 2 x $10^8$ viable cell count per ml of fermentation broth. Further guidance in respect of using yeast for fermentation may be found in, *e.g.,* "The Alcohol Textbook" (Editors K. Jacques, T.P. Lyons and D.R. Kelsall, Nottingham University Press, United Kingdom 1999).

For ethanol production, following the fermentation the fermented slurry may be distilled to extract the ethanol. The ethanol obtained according to processes of the invention may be used as, e.g., fuel ethanol, drinking ethanol, *i.e.,* potable neutral spirits, or industrial ethanol.

Fermentation Stimulators

A fermentation stimulator may be used in combination with any of the processes described herein to further improve the fermentation process, and in particular, the performance of the fermenting microorganism, such as, rate enhancement and ethanol yield. A "fermentation stimulator" refers to stimulators for growth of the fermenting microorganisms, in particular, yeast. Preferred fermentation stimulators for growth include vitamins and minerals. Examples of vitamins include multivitamins, biotin, pantothenate, nicotinic acid, meso-inositol, thiamine, pyridoxine, para-aminobenzoic acid, folic acid, riboflavin, and Vitamins A, B, C, D, and E. See, for example, Alfenore et al., Improving ethanol production and viability of Saccharomyces cerevisiae by a vitamin feeding strategy during fed-batch process, Springer-Verlag (2002). Examples of minerals include minerals and mineral salts that can supply nutrients comprising P, K, Mg, S, Ca, Fe, Zn, Mn, and Cu.

Fermentation products

A fermentation product may be any substance derived from the fermentation. The fermentation product may be, without limitation, an alcohol (*e.g.,* arabinitol, n-butanol, isobutanol, ethanol, glycerol, methanol, ethylene glycol, 1,3-propanediol [propylene glycol], butanediol, glycerin, sorbitol, and xylitol); an alkane (*e.g.,* pentane, hexane, heptane, octane, nonane, decane, undecane, and dodecane), a cycloalkane (*e.g.,* cyclopentane, cyclohexane, cycloheptane, and cyclooctane), an alkene (*e.g.,* pentene, hexene, heptene, and octene); an amino acid (*e.g.,* aspartic acid, glutamic acid, glycine, lysine, serine, and threonine); a gas (*e.g.,* methane, hydrogen ($H_2$), carbon dioxide ($CO_2$), and carbon monoxide (CO)); isoprene; a ketone (*e.g.,* acetone); an organic acid (*e.g.,* acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, oxaloacetic acid, propionic acid, succinic acid, and xylonic acid); and polyketide. The fermentation product may also be protein as a high value product.

In one embodiment, the fermentation product is an alcohol. The term "alcohol" encompasses a substance that contains one or more hydroxyl moieties. The alcohol may be, but is not limited to, n-butanol, isobutanol, ethanol, methanol, arabinitol, butanediol, ethylene glycol, glycerin, glycerol, 1,3-propanediol, sorbitol, xylitol. See, for example, Gong et al., 1999, Ethanol production from renewable resources, in Advances in Biochemical Engineering/Biotechnology, Scheper, T., ed., Springer-Verlag Berlin Heidelberg, Germany, 65: 207-241; Silveira and Jonas, 2002, Appl. Microbiol. Biotechnol. 59: 400-408; Nigam and Singh, 1995, Process Biochemistry 30(2): 117-124; Ezeji et al., 2003, World Journal of Microbiology and Biotechnology 19(6): 595-603.

In another embodiment, the fermentation product is an alkane. The alkane may be an unbranched or a branched alkane. The alkane may be, but is not limited to, pentane, hexane, heptane, octane, nonane, decane, undecane, or dodecane.

In another embodiment, the fermentation product is a cycloalkane. The cycloalkane may be, but is not limited

to, cyclopentane, cyclohexane, cycloheptane, or cyclooctane.

In another embodiment, the fermentation product is an alkene. The alkene may be an unbranched or a branched alkene. The alkene may be, but is not limited to, pentene, hexene, heptene, or octene.

In another embodiment, the fermentation product is an amino acid. The organic acid may be, but is not limited to, aspartic acid, glutamic acid, glycine, lysine, serine, or threonine. See, for example, Richard and Margaritis, 2004, Biotechnology and Bioengineering 87(4): 501-515.

In another embodiment, the fermentation product is a gas. The gas may be, but is not limited to, methane, $H_2$, $CO_2$, or CO. See, for example, Kataoka et al., 1997, Water Science and Technology 36(6-7): 41-47; and Gunaseelan, 1997, Biomass and Bioenergy 13(1-2): 83-114.

In another embodiment, the fermentation product is isoprene.

In another embodiment, the fermentation product is a ketone. The term "ketone" encompasses a substance that contains one or more ketone moieties. The ketone may be, but is not limited to, acetone.

In another embodiment, the fermentation product is an organic acid. The organic acid may be, but is not limited to, acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, propionic acid, succinic acid, or xylonic acid. See, for example, Chen and Lee, 1997, Appl. Biochem. Biotechnol. 63-65: 435-448.

In another embodiment, the fermentation product is polyketide.

Recovery

The fermentation product(s) may be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation. Ethanol with a purity of up to about 96 vol. % may be obtained, which may be used as, for example, fuel ethanol, drinking ethanol, i.e., potable neutral spirits, or industrial ethanol.

**Enzymes**

Below sections describe polypeptides, enzymes and enzyme preparations that may be used according to the processes of the invention.

Phenol Oxidizing Enzymes

A phenol oxidizing enzyme present or added during preconditioning methods or processes according to embodiments of the invention may be any phenol oxidizing enzyme. The phenol oxidizing enzyme may be of any origin, but preferably of fungal or bacterial origin.

The phenol oxidizing enzyme(s) may belong to any of the following EC classes including, but not limited to, Laccase (EC 1.10.3.2), Catechol oxidase (EC 1.10.3.1), o-Aminophenol oxidase (1.10.3.4); and Monophenol monooxygenase (1.14.18.1). In one embodiment, laccases are preferred.

Laccases

According to an embodiment of the invention a laccase may be present or added during preconditioning. Laccases (EC 1.10.3.2.) are multi-copper-containing enzymes that catalyze the oxidation of phenolic compounds. Laccases are produced by plants, bacteria and also a wide variety of fungi, including Ascomycetes such as *Aspergillus, Neurospora,* and *Podospora*; Deuteromycete including *Botrytis,* and Basidiomycetes such as *Collybia, Fomes, Lentinus, Pleurotus, Trametes,* and perfect forms of *Rhizoctonia.* A number of fungal laccases have been isolated. For example, Choi et al. (Mol. Plant-Microbe Interactions 5: 119-128, 1992) describe the molecular characterization and cloning of the gene encoding the laccase of the chestnut blight fungus, *Cryphonectria parasitica.* Kojima et al. (J. Biol. Chem. 265: 15224-15230, 1990; JP 2-238885) provide a description of two allelic forms of the laccase of the white-rot basidiomycete *Coriolus hirsutus.* Germann and Lerch (Experientia 41: 801, 1985; PNAS USA 83: 8854-8858, 1986) have reported the cloning and partial sequencing of the *Neurospora crassa* laccase gene. Saloheimo et al. (J. Gen. Microbiol. 137: 1537-1544, 1985; WO 92/01046) have disclosed a structural analysis of the laccase gene from the fungus *Phlebia radiata.*

Especially contemplated laccases include those derived from a strain of *Polyporus,* preferably *Polyporus pinsitus*; *Melanocarpus,* preferably *Melanocarpus albomyces*; *Myceliophtora,* preferably *Myceliophtora thermophila*; *Coprinus,* preferably *Coprinus cinereus*; *Rhizoctonia,* preferably *Rhizoctonia solani* or *Rhizoctonia praticola*; *Scytalidium,* preferably *Scytalidium thermophilum*; *Pyricularia,* preferably *Pyricularia oryzae.*

In an embodiment the laccase is derived from the tree *Rhus vernicifera* (Yoshida, 1883, Chemistry of Lacquer (Urushi) part 1. J. Chem. Soc. 43, 472-486).

In another embodiment the laccase is derived from *Polyporus pinsitus, e.g.,* the one described in WO 96/00290 (Novozymes).

Jönsson et al., 1998, Appl. Microbiol. Biotechnol. 49, 691-697, also disclose a suitable laccase derived from *Polyporus versicolar.*

Other laccases include the one derived from *Pyricularia oryzae* concerned in, *e.g.,* Muralikrishna et al., 1995, Appl. Environ. Microbiol. 61(12):4374-4377) or the laccase disclosed in Abstract of Papers American Chemical Society vol. 209, no. 1-2, 1995 derived from a *Scytalidium thermophilum.*

The laccase may also be one derived from *Coprinus cinereus, e.g.,* the one concerned in Schneider et al., 1999, Enzyme and Microbial Technology 25: 502-508.

Other suitable laccases include those derived from *Rhizoctonia solani* concerned in Waleithner et al., 1996, Curr. Genet. 29: 395-403, or derived from *Melanocarpus albomyces* concerned in Kiiskinen et al., 2004, Microbiology 150: 3065-3074.

Suitable bacterial laccase include those derived from *Streptomyces coelicolor, e.g.,* disclosed by Machczynski et al., 2004, Protein Science 13: 2388-2397.

In a preferred embodiment the laccase is derived from *Myceliopthora thermophila, e.g.,* the laccase described in WO 95/33836 as SEQ ID NO: 2 (SEQ ID NO: 1 herein). In various embodiments the laccase is provided in liquid, granular or powdered form.

Contemplated laccases also include those comprising an amino acid sequence having at least 60%, at least 70% at least 80%, at least 85%, at least 90%, at least 95% identity, at least 97%, at least 98%, at least 99% sequence identity to SEQ ID NO: 2 of described in WO 95/33836.

<u>Glucoamylases</u>

According to an embodiment of the invention a glucoamylase may be present or added during preconditioning or hydrolysis. A glucoamylase (glucan 1,4-$\alpha$-glucosidase, EC 3.2.1.3) may be derived from any suitable source, e.g., derived from a microorganism or a plant. Preferred glucoamylases are of fungal or bacterial origin, selected from the group consisting of *Aspergillus* glucoamylases, in particular *Aspergillus niger* G1 or G2 glucoamylase (Boel et al., 1984, EMBO J. 3(5): 1097-1102), or variants thereof, such as those disclosed in WO 92/00381, WO 00/04136 and WO 01/04273 (from Novozymes, Denmark); the *A. awamori* glucoamylase disclosed in WO 84/02921, *Aspergillus oryzae* glucoamylase (Hata et al., 1991, Agric. Biol. Chem. 55(4): 941-949), or variants or fragments thereof. Other *Aspergillus* glucoamylase variants include variants with enhanced thermal stability: G137A and G139A (Chen et al., 1996, Prot. Eng. 9: 499-505); D257E and D293E/Q (Chen et al., 1995, Prot. Eng. 8: 575-582); N182 (Chen et al., 1994, Biochem. J. 301: 275-281); disulphide bonds, A246C (Fierobe et al., 1996, Biochemistry 35: 8698-8704; and introduction of Pro residues in positions A435 and S436 (Li et al., 1997, Protein Eng. 10: 1199-1204.

Other glucoamylases include *Athelia rolfsii* (previously denoted *Corticium rolfsii*) glucoamylase (see U.S. Patent No. 4,727,026 and Nagasaka et al., 1998, Appl. Microbiol. Biotechnol. 50: 323-330), *Talaromyces* glucoamylases, in particular derived from *Talaromyces duponti, Talaromyces emersonii* (WO 99/28448), *Talaromyces leycettanus* (U.S. Patent No. Re. 32,153), and *Talaromyces thermophilus* (U.S. Patent No. 4,587,215).

Bacterial glucoamylases include glucoamylases from *Clostridium,* in particular *C. thermoamylolyticum* (EP 135138) and *C. thermohydrosulfuricum* (WO 86/01831), *Trametes cingulata, Pachykytospora papyracea,* and *Leucopaxillus giganteus,* all disclosed in WO 2006/069289; or *Peniophora rufomarginata* disclosed in PCT/US2007/066618; or a mixture thereof. A hybrid glucoamylase may be used in the present invention. Examples of hybrid glucoamylases are disclosed in WO 2005/045018. Specific examples include the hybrid glucoamylase disclosed in Tables 1 and 4 of Example 1.

A glucoamylase may be one having a high degree of sequence identity to any of above mentioned glucoamylases, i.e., at least 60%, at least 70%, at least 75%, at least 80%, at least 85%, at least 90%, at least 95%, at least 96%, at least 97%, at least 98%, at least 99% or even 100% identity to the mature enzyme sequences described herein.

A glucoamylase may further comprise a blend of glucoamylases and/or additional amylases and may further comprise variants or fragments of glucoamylases. In one embodiment the glucoamylase is a blend comprising *Talaromyces emersonii* glucoamylase, disclosed as SEQ ID NO: 34 in WO99/28448, *Trametes cingulata* glucoamylase, disclosed as SEQ ID NO: 2 in WO 06/69289, and *Rhizomucorpusillus* alpha-amylase with *Aspergillus niger* glucoamylase linker and starch binding domain (SBD), disclosed as V039 in Table 5 in WO 2006/069290 as side activity (activity ratio in AGU:AGU:FAU-F is about 20:5:1).

Glucoamylase activity may be measured in AGU (Glucoamylase Unit). 1 AFU is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes.

An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

Commercially available glucoamylase compositions include AMG 200L; AMG 300L; SAN™ SUPER, SAN™ EXTRA L, SPIRIZYME® PLUS, SPIRIZYME® FUEL, SPIRIZYME® B4U, SPIRIZYME® ULTRA™, SPIRIZYME® EXCEL, SPIRIZYME® ACHIEVE and AMG™ E (from Novozymes A/S, Denmark); OPTIDEX® 300, GC480™ and GC147™ (from Danisco US Inc); AMIGASE™ and AMIGASE™ PLUS (from DSM); G-ZYME® G900, G-ZYME® and G990 ZR (from Danisco US Inc.).

Glucoamylases are preferably added in a concentration between 0.01 and 20 mg EP/g cellulose, such as 0.1-1 mg EP/g cellulose.

Alpha-Amylases

According to an embodiment of the invention an alpha-amylase may be present or added during preconditioning or hydrolysis. According to an embodiment of the invention any alpha-amylase may be used, such as of fungal, bacterial or plant origin. In a preferred embodiment the alpha-amylase is an acid alpha-amylase, *e.g.,* acid fungal or acid bacterial alpha-amylase.

In an embodiment the alpha-amylase is an acid alpha-amylase. The term "acid alpha-amylase" means an alpha-amylase (EC 3.2.1.1) which added in an effective amount has activity optimum at a pH in the range of 3 to 7, preferably from 3.5 to 6, or more preferably from 4-5. In an embodiment the alpha-amylase is a fungal alpha-amylase, such as an acid fungal alpha-amylase.

Fungal alpha-amylases include alpha-amylases derived from a strain of *Aspergillus,* such as, *Aspergillus kawachii, Aspergillus niger* and *Aspergillus oryzae* alpha-amylases.

A preferred acid fungal alpha-amylase is an alpha-amylase which exhibits a high identity, *i.e.,* at least 70%, at least 75%, at least 80%, at least 85%, at least 90%, at least 95%, at least 96%, at least 97%, at least 98%, at least 99% or even 100% identity to the mature part of the amino acid sequence shown in SEQ ID NO: 10 in WO 96/23874.

Another preferred acid alpha-amylase is derived from a strain of *Aspergillus niger.* In a preferred embodiment the acid fungal alpha-amylase is an *Aspergillus niger* alpha-amylase disclosed as "AMYA_ASPNG" in the Swiss-prot/TeEMBL database under the primary accession no. P56271 and described in WO 89/01969 (Example 3). A commercially available acid fungal alpha-amylase derived from *Aspergillus niger* is SP288 (available from Novozymes A/S, Denmark).

Other wild-type alpha-amylases include those derived from a strain of *Meripilus* and *Rhizomucor,* preferably a strain of *Meripilus giganteus* or *Rhizomucor pusillus* (WO 2004/055178).

In a preferred embodiment the alpha-amylase is derived from *Aspergillus kawachii* (Kaneko et al., 1996, J. Ferment. Bioeng. 81: 292-298, "Molecular-cloning and determination of the nucleotide-sequence of a gene encoding an acid-stable alpha-amylase from Aspergillus kawachii"; and further as EMBL: #AB008370).

The fungal alpha-amylase may also be a wild-type enzyme comprising a starch-binding domain (SBD) and an alpha-amylase catalytic domain, or a variant thereof.

In an embodiment the fungal acid alpha-amylase is a hybrid alpha-amylase. Examples of fungal hybrid alpha-amylases include the ones disclosed in WO 2005/003311, U.S. Patent No. 7,883,883 (Novozymes), and WO 2006/069290 (Novozymes). A hybrid alpha-amylase may comprise an alpha-amylase catalytic domain (CD) and a carbohydrate-binding domain/module (CBM), such as a starch binding domain (SBD), and optionally a linker.

Examples of hybrid alpha-amylases include those disclosed in Tables 1 to 5 of the examples in WO 2006/069290 including the variant with the catalytic domain JA118 and *Athelia rolfsii* SBD (SEQ ID NO: 100 in WO 2006/069290), *Rhizomucor pusillus* alpha-amylase with *Athelia rolfsii* AMG linker and SBD (SEQ ID NO: 101 in WO 2006/069290), *Rhizomucor pusillus* alpha-amylase with *Aspergillus niger* glucoamylase linker and SBD (which is disclosed as V039 in Table 5 in WO 2006/069290), and *Meripilus giganteus* alpha-amylase with *Athelia rolfsii* glucoamylase linker and SBD (SEQ ID NO: 102 in WO 2006/069290). Other hybrid alpha-amylases are listed in Tables 3, 4, 5, and 6 in Example 4 in WO 2006/069290.

Other examples of hybrid alpha-amylases include those disclosed in U.S. Patent 7,883,883, including those disclosed in Table 3 in col. 26, such as *Aspergillus niger* alpha-amylase with *Aspergillus kawachii* linker and starch binding domain.

Other alpha-amylases exhibit a high degree of sequence identity to any of above mentioned alpha-amylases, i.e., at least 70%, at least 75%, at least 80%, at least 85%, at least 90%, at least 95%, at least 96%, at least 97%, at least 98%, at least 99% or even 100% identity to the mature enzyme sequences disclosed above.

Acid alpha-amylase activity may be measured in AFAU (Acid Fungal Alpha-amylase Units), which are determined relative to an enzyme standard. 1 AFAU is defined as the amount of enzyme which degrades 5.260 mg starch

dry matter per hour under the below mentioned standard conditions.

Acid alpha-amylase, an endo-alpha-amylase (1,4-alpha-D-glucan-glucanohydrolase, E.C. 3.2.1.1) hydrolyzes alpha-1,4-glucosidic bonds in the inner regions of the starch molecule to form dextrins and oligosaccharides with different chain lengths. The intensity of color formed with iodine is directly proportional to the concentration of starch. Amylase activity is determined using reverse colorimetry as a reduction in the concentration of starch under the specified analytical conditions.

$$\text{STARCH + IODINE} \xrightarrow[40°,\ pH\ 2.5]{\text{ALPHA - AMYLASE}} \text{DEXTRINS + OLIGOSACCHARIDES}$$

$\lambda = 590$ nm

blue/violet t = 23 sec. decoloration

The reaction conditions are substrate: Soluble starch, approx. 0.17 g/L, buffer: Citrate, approx. 0.03 M, 0.03g/L iodine ($I_2$), 1.85 mM $CaCl_2$, pH 2.50 ± 0.05, incubation temperature 40°C, reaction time 23 seconds, wavelength 590 nm, enzyme concentration 0.025 AFAU/mL, enzyme working range 0.01-0.04 AFAU/mL.

FAU-F Fungal Alpha-Amylase Units (Fungamyl) is measured relative to an enzyme standard of a declared strength. The reaction conditions are 37°C, pH 7.15, wavelength: 405nm, reaction time 5 minutes, measuring time 2 minutes.

Commercial compositions comprising alpha-amylase include MYCOLASE®(DSM), BAN™, TERMAMYL® SC, FUNGAMYL®, LIQUOZYME® X, LIQUOZYME® SC and SAN™ SUPER, SAN™ EXTRA L (Novozymes A/S) and CLARASE™ L-40,000, DEX-LO™, SPEZYME® FRED, SPEZYME® AA, SPEZYME® DELTAAA, GC358, GC980, and SPEZYME® RSL (Danisco), and the acid fungal alpha-amylase sold under the trade name SP288 (available from Novozymes A/S, Denmark).

Alpha-amylase is preferably added in concentrations between 0.001 and 20 mg EP/g cellulose, such as 0.01-1 mg EP/g cellulose.

## Hemicellulases

According to an embodiment of the invention a hemicellulase may be present or added during preconditioning or hydrolysis (i.e., saccharification). The hemicellulase may be any hemicellulase. The hemicellulase may be in the form of a hemicellulolytic enzyme preparation. The hemicellulase may be of any origin, but preferably of fungal or bacterial origin.

The term "hemicellulase" or "hemicellulolytic enzyme" means one or more (several) enzymes that hydrolyze a hemicellulosic material. See, for example, Shallom and Shoham, 2003, Microbial hemicellulases. Current Opinion In Microbiology, 6(3): 219-228. Hemicellulases are key components in the degradation of plant biomass. Examples of hemicellulases include, but are not limited to, an acetylmannan esterase, an acetyxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, may be assigned into GH and CE families marked by numbers. Some families, with overall similar fold, may be further grouped into clans, marked alphabetically (e.g., GH-A). A most informative and updated classification of these and other carbohydrate active enzymes is available on the Carbohydrate-Active Enzymes (CAZy) database. Hemicellulolytic enzyme activities may be measured according to Ghose and Bisaria, 1987, Pure & Appl. Chem. 59: 1739-1752.

In an embodiment the hemicellulase present or added during preconditioning and/or saccharification is a hemicellulolytic enzyme preparation. In an embodiment the hemicellulolytic enzyme preparation is cellulolytic enzyme preparation from *Trichoderma reesei,* further comprising a xylanase and/or a beta-xylosidase. In a preferred embodiment the hemicellulolytic enzyme preparation is cellulolytic enzyme preparation from *Trichoderma reesei,* further comprising *Aspergillus fumigatus* xylanase (Xyl III in WO 2006/078256, SEQ ID NO: 6 herein) and *Aspergillus fumigatus* beta-xylosidase (WO 2011/057140, SEQ ID NO: 7 herein).

The hemicellulase or hemicellulolytic enzyme preparation may preferably be added in concentrations between 0.01 and 20 mg EP/g cellulose, such as 0.1-1 mg EP/g cellulose.

## Xylanases

In a preferred embodiment the hemicellulase is a xylanase or the hemicellulolytic enzyme preparation comprises a xylanase. The term "xylanase" means a 1,4-beta-D-xylan-xylohydrolase (E.C. 3.2.1.8) that catalyzes the endohydrolysis

of 1,4-beta-D-xylosidic linkages in xylans. For purposes of the present invention, xylanase activity is determined with 0.2% AZCL-arabinoxylan as substrate in 0.01% TRITON® X-100 and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

Examples of specifically contemplated xylanases include GH10 xylanases, such as one derived from a strain of the genus *Aspergillus,* such as a strain from *Aspergillus fumigatus,* such as the one disclosed as Xyl III in WO 2006/078256, or *Aspergillus aculeatus,* such as the one disclosed as Xyl II in WO 94/21785 (SEQ ID NO: 5 therein).

The xylanase may be comprised in a cellulolytic enzyme preparation which further includes a xylanase. In one embodiment hemicellulase is a cellulolytic enzyme preparation further comprising a xylanase, preferably a GH10 xylanase, such as one derived from a strain of the genus *Aspergillus,* such as a strain from *Aspergillus fumigatus,* such as the one disclosed as Xyl III in WO 2006/078256, or *Aspergillus aculeatus,* such as the one disclosed as Xyl II in WO 94/21785.

Contemplated xylanases also include those comprising an amino acid sequence having at least 60%, at least 70%, at least 80%, at least 85%, at least 90%, at least 95% identity, at least 97%, at least 98%, at least 99% identity to the *Aspergillus fumigatus* xylanase in WO 2006/078256, or the *Aspergillus aculeatus* xylanase disclosed as Xyl II in WO 94/21785.

Beta-xvlosidases

In an embodiment the hemicellulase used in methods or processes of the invention is a beta-xylosidase, or the hemicellulolytic enzyme preparation comprises a beta-xylosidase. The term "beta-xylosidase" means a beta-D-xyloside xylohydrolase (E.C. 3.2.1.37) that catalyzes the exo-hydrolysis of short beta (1→4)-xylooligosaccharides, to remove successive D-xylose residues from the non-reducing termini. For purposes of the present invention, one unit of beta-xylosidase is defined as 1.0 μmole of p-nitrophenolate anion produced per minute at 40°C, pH 5 from 1 mM *p*-nitrophenyl-beta-D-xyloside as substrate in 100 mM sodium citrate containing 0.01% TWEEN® 20.

Examples of specifically contemplated beta-xylosidase include those derived from a strain of the genus *Aspergillus,* such as a strain of *Aspergillus fumigatus,* such as the one disclosed in WO 2013/028928 (Example 16 and 17), or derived from a strain of *Trichoderma,* such as a strain of *Trichoderma reesei,* such as the mature polypeptide of SEQ ID NO: 58 in WO 2011/057140.

The beta-xylosidase used during preconditioning may be comprised in a cellulolytic enzyme preparation. In one embodiment the hemicellulase is a cellulolytic enzyme preparation further comprising a beta-xylosidase, such as one derived from a strain of the genus *Aspergillus,* such as a strain of *Aspergillus fumigatus* (*e.g.,* one disclosed in WO 2011/057140), such as one disclosed in WO 2013/028928 (Examples 16 and 17), or derived from a strain of *Trichoderma,* such as a strain of *Trichoderma reesei,* such as the mature polypeptide of SEQ ID NO: 58 in WO 2011/057140.

Contemplated beta-xylosidases also include those comprising an amino acid sequence having at least 60%, at least 70% at least 80%, at least 85%, at least 90%, at least 95% identity, at least 97%, at least 98%, at least 99% identity to the *Aspergillus fumigatus* beta-xylosidase disclosed as SEQ ID NO: 206 in WO 2011/057140 or any of the beta-xylosidases mentioned herein.

The hemicellulase used for preconditioning is or may comprise a commercial hemicellulase product. Examples of commercial hemicellulase products include, for example, SHEARZYME™ (Novozymes A/S), CELLIC® HTec (Novozymes A/S), CELLIC® HTec2 (Novozymes A/S), CELLIC® HTec3 (Novozymes), VISCOZYME® (Novozymes A/S), ULTRAFLO® (Novozymes A/S), PULPZYME® HC (NovozymesA/S), MULTIFECT® Xylanase (Danisco US Inc), ECOPULP® TX-200A (Roal Oy), HSP 6000 Xylanase (DSM), DEPOL® 333P (Biocatalysts Limit, Wales, UK), DEPOL™ 740L. (Biocatalysts Limit, Wales, UK), and DEPOL™ 762P (Biocatalysts Limit, UK).

Beta-Glucosidase

According to an embodiment of the invention a beta-glucosidase may be present or added during preconditioning or hydrolysis (i.e., saccharification).

In an embodiment the beta-glucosidase is an enzyme preparation with beta-glucosidase activity. Where the preparation exhibits beta-glucosidase activity, such activity may be the primary activity of the composition, or may be a lesser activity of the composition. In one embodiment the beta-glucosidase is comprised in an *Aspergillus niger* enzyme preparation comprising 57% glucoamylase, 27% beta-glucosidase and 16% alpha-amylase (protein content basis), where the preparation exhibits beta-glucosidase activity.

Further, a cellulolytic enzyme preparation used according to the invention may in one embodiment comprise one or more beta-glucosidases. The beta-glucosidase may in one embodiment be one derived from a strain of the genus *Aspergillus,* such as *Aspergillus niger* or *Aspergillus oryzae,* such as the one disclosed in WO 2002/095014 or the fusion protein having beta-glucosidase activity disclosed in WO 2008/057637, or *Aspergillus fumigatus,* such as such as one

disclosed in WO 2005/047499 or an *Aspergillus fumigatus* beta-glucosidase variant, such as one disclosed in WO 2012/044915, such as one with the following substitutions: F100D, S283G, N456E, F512Y.

In another embodiment the beta-glucosidase is derived from a strain of the genus *Penicillium,* such as a strain of the *Penicillium brasilianum* disclosed in WO 2007/019442, or a strain of the genus *Trichoderma,* such as a strain of *Trichoderma reesei.*

In an embodiment beta-glucosidase is an *Aspergillus fumigatus* beta-glucosidase or homolog thereof selected from the group consisting of:

(i) a beta-glucosidase comprising the mature polypeptide of SEQ ID NO: 2 of WO 2005/047499;

(ii) a beta-glucosidase comprising an amino acid sequence having at least 70%, e.g., at least 75%, at least 80%, at least 85%, at least 90%, at least 91%, at least 92%, at least 93%, at least 94%, at least 95%, at least 96%, at least 97%, at least 98%, or at least 99% sequence identity to the mature polypeptide of SEQ ID NO: 2 of WO 2005/047499.

In an embodiment the beta-glucosidase is a variant comprises a substitution at one or more (several) positions corresponding to positions 100, 283, 456, and 512 of the mature polypeptide of SEQ ID NO: 2 of WO 2005/047499, wherein the variant has beta-glucosidase activity.

In an embodiment the parent beta-glucosidase of the variant is (a) a polypeptide comprising the mature polypeptide of SEQ ID NO: 2 of WO 2005/047499; (b) a polypeptide having at least 80% sequence identity to the mature polypeptide of SEQ ID NO: 2 of WO 2005/047499 or (c) a fragment of the mature polypeptide of SEQ ID NO: 2 of WO 2005/047499, which has beta-glucosidase activity.

In an embodiment the beta-glucosidase variant has at least 80%, e.g., at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, but less than 100% sequence identity to the amino acid sequence of the parent beta-glucosidase.

In an embodiment the beta-glucosidase variant has at least 80%, e.g., at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, but less than 100% sequence identity to the mature polypeptide of SEQ ID NO: 2 of WO 2005/047499.

In an embodiment the beta-glucosidase is from a strain of *Aspergillus,* such as a strain of *Aspergillus fumigatus,* such as *Aspergillus fumigatus* beta-glucosidase (SEQ ID NO: 2 of WO 2005/047499), which comprises one or more substitutions selected from the group consisting of L89M, G91L, F100D, I140V, I186V, S283G, N456E, and F512Y; such as a variant thereof with the following substitutions:

- F100D + S283G + N456E + F512Y;
- L89M + G91L + I186V + I140V;
- I186V + L89M + G91L + I140V + F100D + S283G + N456E + F512Y.

In an embodiment the number of substitutions is between 1 and 10, such 1 and 8, such as 1 and 6, such as 1 and 4, such as 1, 2, 3, 4, 5, 6, 7, 8, 9 or 10 substitutions.

In an embodiment the variant comprises a substitution at a position corresponding to position 100, a substitution at a position corresponding to position 283, a substitution at a position corresponding to position 456, and/or a substitution at a position corresponding to position 512.

In a preferred embodiment the beta-glucosidase variant comprises the following substitutions: Phe100Asp, Ser283Gly, Asn456Glu, Phe512Tyr in SEQ ID NO: 2 of WO 2005/047499 (described in WO 2012/044915).

Polypeptide having cellulolytic enhancing activity

The cellulolytic enzyme preparation used according to an embodiment of the invention may comprise one or more AA9 (GH61) polypeptides having cellulolytic enhancing activity. In one embodiment the enzyme composition comprises an AA9 (GH61) polypeptide having cellulolytic enhancing activity, such as one derived from the genus *Thermoascus,* such as a strain of *Thermoascus aurantiacus,* such as the one described in WO 2005/074656 as SEQ ID NO: 2; or one derived from the genus *Thielavia,* such as a strain of *Thielavia terrestris,* such as the one described in WO 2005/074647 as SEQ ID NO: 7 and SEQ ID NO: 8; or one derived from a strain of *Aspergillus,* such as a strain of *Aspergillus fumigatus,* such as the one described in WO 2010/138754 as SEQ ID NO: 2; or one derived from a strain derived from *Penicillium,* such as a strain of *Penicillium emersonii,* such as the one disclosed as SEQ ID NO: 2 of WO 2011/041397.

In an embodiment the *Penicillium* sp. AA9 polypeptide having cellulolytic enhancing activity or a homolog thereof is selected from the group consisting of:

(i) an AA9 polypeptide having cellulolytic enhancing activity comprising the mature polypeptide of SEQ ID NO: 2 of WO 2011/041397;

(ii) an AA9 polypeptide having cellulolytic enhancing activity comprising an amino acid sequence having at least 70%, e.g., at least 75%, at least 80%, at least 85%, at least 90%, at least 91%, at least 92%, at least 93%, at least 94%, at least 95%, at least 96%, at least 97%, at least 98%, or at least 99% sequence identity to the mature polypeptide of SEQ ID NO: 2 of WO 2011/041397.

Cellobiohydrolase I

The cellulolytic enzyme preparation used according to an embodiment of the invention may comprise one or more CBH I (cellobiohydrolase I). In one embodiment the cellulolytic composition comprises a cellobiohydrolase I (CBH I), such as one derived from a strain of the genus *Aspergillus,* such as a strain of *Aspergillus fumigatus,* such as the Cel7A CBHI disclosed as SEQ ID NO: 6 in WO 2011/057140, or a strain of the genus *Trichoderma,* such as a strain of
*Trichoderma reesei.*
In an embodiment the *Aspergillus fumigatus* cellobiohydrolase I or homolog thereof is selected from the group consisting of:

(i) a cellobiohydrolase I comprising the mature polypeptide of SEQ ID NO: 6 in WO 2011/057140;
(ii) a cellobiohydrolase I comprising an amino acid sequence having at least 60%, at least 70%, e.g., at least 75%, at least 80%, at least 85%, at least 90%, at least 91%, at least 92%, at least 93%, at least 94%, at least 95%, at least 96%, at least 97%, at least 98%, or at least 99% sequence identity to the mature polypeptide of SEQ ID NO: 6 in WO 2011/057140.

Cellobiohydrolase II

The cellulolytic enzyme preparation used according to an embodiment of the invention may comprise one or more CBH II (cellobiohydrolase II). In one embodiment the cellobiohydrolase II (CBHII), such as one derived from a strain of the genus *Aspergillus,* such as a strain of *Aspergillus fumigatus,* such as one described in WO2011/057140 or a strain of the genus *Trichoderma,* such as *Trichoderma reesei,* or a strain of the genus *Thielavia,* such as a strain of *Thielavia terrestris,* such as cellobiohydrolase II CEL6A from *Thielavia terrestris.*
In an embodiment the *Aspergillus fumigatus* cellobiohydrolase II or homolog thereof is selected from the group consisting of:

(i) a cellobiohydrolase II comprising the mature polypeptide of a CBHII described in WO2011/057140;
(ii) a cellobiohydrolase II comprising an amino acid sequence having at least 60%, at least 70%, e.g., at least 75%, at least 80%, at least 85%, at least 90%, at least 91%, at least 92%, at least 93%, at least 94%, at least 95%, at least 96%, at least 97%, at least 98%, or at least 99% identity to the mature polypeptide of a CBHII described in WO2011/057140.

Endoglucanase

The term "endoglucanase" means an endo-1,4-(1,3;1,4)-beta-D-glucan 4-glucanohydrolase (E.C. 3.2.1.4), which catalyzes endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity may be determined by measuring reduction in substrate viscosity or increase in reducing ends determined by a reducing sugar assay (Zhang et al., 2006, Biotechnology Advances 24: 452-481). For purposes of the present invention, endoglucanase activity is determined using carboxymethyl cellulose (CMC) as substrate according to the procedure of Ghose, 1987, Pure and Appl. Chem. 59: 257-268, at pH 5, 40°C.

Cellulolytic Enzyme Preparation

According to an embodiment of the invention a cellulolytic enzyme preparation is present or added during hydrolysis (i.e., saccharification). A cellulolytic enzyme preparation is a preparation containing one or more (e.g., several)

enzymes that hydrolyze cellulosic material. Such enzymes include endoglucanase, cellobiohydrolase, beta-glucosidase, or combinations thereof.

The two basic approaches for measuring cellulolytic activity include: (1) measuring the total cellulolytic activity, and (2) measuring the individual cellulolytic activities (endoglucanases, cellobiohydrolases, and beta-glucosidases) as reviewed in Zhang et al., Outlook for cellulase improvement: Screening and selection strategies, 2006, Biotechnology Advances 24: 452-481. Total cellulolytic activity is usually measured using insoluble substrates, including Whatman №1 filter paper, microcrystalline cellulose, bacterial cellulose, algal cellulose, cotton, pretreated lignocellulose, etc. The most common total cellulolytic activity assay is the filter paper assay using Whatman №1 filter paper as the substrate. The assay was established by the International Union of Pure and Applied Chemistry (IUPAC) (Ghose, 1987, Measurement of cellulase activities, Pure Appl. Chem. 59: 257-68).

For purposes of the present invention, cellulolytic enzyme activity for, e.g., a cellulolytic enzyme preparation, may be determined by measuring the increase in hydrolysis of a cellulosic material by cellulolytic enzyme(s) under the following conditions: 0.25-50 mg of cellulolytic enzyme protein/g of cellulose in PCS (or other pretreated cellulosic material) for 3-7 days at a suitable temperature, e.g., 50°C, 55°C, 60°C, or 65°C, compared to a control hydrolysis without addition of cellulolytic enzyme protein. Typical conditions are 1 ml reactions, washed or unwashed PCS, 5% insoluble solids, 50 mM sodium acetate pH 5, 1 mM $MnSO_4$, 50°C, 55°C, 60°C, or 65°C, 72 hours, sugar analysis by AMINEX® HPX-87H column (Bio-Rad Laboratories, Inc., Hercules, CA, USA).

As mentioned above a cellulolytic enzyme preparation used for hydrolysis (i.e., saccharification) in processes of the invention typically comprises one or more endoglucanases, cellubiohydrolases and/or beta-glucosidases.

In an embodiment the cellulolytic enzyme preparation is derived from a strain of *Trichoderma,* such as a strain of *Trichoderma reesei*; a strain of *Humicola,* such as a strain of *Humicola insolens,* and/or a strain of *Chrysosporium,* such as a strain of *Chrysosporium lucknowense.* In a preferred embodiment the cellulolytic enzyme preparation is derived from a strain of *Trichoderma reesei.*

The cellulolytic enzyme preparation may further comprise one or more of the following polypeptides, such as enzymes: AA9 polypeptide having cellulolytic enhancing activity, beta-glucosidase, xylanase, beta-xylosidase, CBHI, CBHII, or a mixture of two, three, four, five or six thereof.

The further polypeptide(s) (*e.g.*, AA9 polypeptide) and/or enzyme(s) (*e.g.*, beta-glucosidase, xylanase, beta-xylosidase, CBH I and/or CBH II may be foreign to the cellulolytic enzyme preparation producing organism (*e.g.*, *Trichoderma reesei*).

In an embodiment the cellulolytic enzyme preparation comprises a AA9 polypeptide having cellulolytic enhancing activity and a beta-glucosidase.

In another embodiment the cellulolytic enzyme preparation comprises a AA9 polypeptide having cellulolytic enhancing activity, a beta-glucosidase, and a CBHI.

In another embodiment the cellulolytic enzyme preparation comprises a AA9 polypeptide having cellulolytic enhancing activity, a beta-glucosidase, a CBHI and a CBHII.

Other enzymes, such as endoglucanases, may also be comprises in the cellulolytic enzyme preparation.

As mentioned above the cellulolytic enzyme preparation may comprise a number of difference polypeptides, including enzymes.

In an embodiment the cellulolytic enzyme preparation comprises a *Trichoderma reesei* cellulolytic preparation, further comprising *Thermoascus aurantiacus* AA9 (GH61A) polypeptide having cellulolytic enhancing activity (WO 2005/074656), and *Aspergillus oryzae* beta-glucosidase fusion protein (WO 2008/057637).

In another embodiment the cellulolytic enzyme preparation comprises a *Trichoderma* reesei cellulolytic enzyme preparation, further comprising *Thermoascus aurantiacus* AA9 (GH61A) polypeptide having cellulolytic enhancing activity (SEQ ID NO: 2 in WO 2005/074656), and *Aspergillus fumigatus* beta-glucosidase (SEQ ID NO: 2 of WO 2005/047499).

In another embodiment the cellulolytic enzyme preparation comprises a *Trichoderma* reesei cellulolytic enzyme preparation further comprising *Penicillium emersonii* AA9 (GH61A) polypeptide having cellulolytic enhancing activity disclosed in WO 2011/041397, and *Aspergillus fumigatus* beta-glucosidase (SEQ ID NO: 2 of WO 2005/047499).

In another embodiment the cellulolytic enzyme preparation comprises a *Trichoderma* reesei cellulolytic enzyme preparation further comprising *Penicillium emersonii* AA9 (GH61A) polypeptide having cellulolytic enhancing activity disclosed in WO 2011/041397, and *Aspergillus fumigatus* beta-glucosidase (SEQ ID NO: 2 of WO 2005/047499) or variant disclosed in WO 2012/044915, the following substitutions: F100D, S283G, N456E, F512Y.

In an embodiment the cellulolytic enzyme preparation is derived from *Trichoderma reesei* further comprising a AA9 (GH61A) polypeptide having cellulolytic enhancing activity derived from a strain of *Penicillium emersonii* (SEQ ID NO: 2 in WO 2011/041397), *Aspergillus fumigatus* beta-glucosidase (SEQ ID NO: 2 in WO 2005/047499) variant F100D, S283G, N456E, F512Y) disclosed in WO 2012/044915; *Aspergillus fumigatus* Cel7A CBH1 disclosed as SEQ ID NO: 6 in WO2011/057140 and *Aspergillus fumigatus* CBH II disclosed as SEQ ID NO: 18 in WO 2011/057140.

In a preferred embodiment the cellulolytic enzyme preparation from *Trichoderma reesei,* further comprises a hemicellulase or hemicellulolytic enzyme preparation, such as an *Aspergillus fumigatus* xylanase (WO 2006/078256)

and *Aspergillus fumigatus* beta-xylosidase (WO 2011/057140).

In an embodiment the cellulolytic enzyme preparation also comprises a xylanase (*e.g.*, derived from *Aspergillus aculeatus* or *Aspergillus fumigatus*) and/or a beta-xylosidase (*e.g.*, derived from *Aspergillus fumigatus*).

In an embodiment the cellulolytic enzyme preparation comprises a *Trichoderma reesei* cellulolytic preparation, further comprising *Thermoascus aurantiacus* AA9 (GH61A) polypeptide having cellulolytic enhancing activity (WO 2005/074656), *Aspergillus oryzae* beta-glucosidase fusion protein (WO 2008/057637), and *Aspergillus aculeatus* xylanase (Xyl II in WO 94/21785).

In another embodiment the cellulolytic enzyme preparation comprises a *Trichoderma* reesei cellulolytic preparation, further comprising *Thermoascus aurantiacus* AA9 (GH61A) polypeptide having cellulolytic enhancing activity (SEQ ID NO: 2 in WO 2005/074656), *Aspergillus fumigatus* beta-glucosidase (SEQ ID NO: 2 of WO 2005/047499) and *Aspergillus aculeatus* xylanase (Xyl II disclosed in WO 94/21785).

In another embodiment the cellulolytic enzyme preparation comprises a *Trichoderma* reesei cellulolytic preparation further comprising *Penicillium emersonii* AA9 (GH61A) polypeptide having cellulolytic enhancing activity disclosed in WO 2011/041397, *Aspergillus fumigatus* beta-glucosidase (SEQ ID NO: 2 of WO 2005/047499) and *Aspergillus fumigatus* xylanase (Xyl III in WO 2006/078256).

In another embodiment the cellulolytic enzyme preparation comprises a *Trichoderma* reesei cellulolytic preparation further comprising *Penicillium emersonii* AA9 (GH61A) polypeptide having cellulolytic enhancing activity disclosed in WO 2011/041397, *Aspergillus fumigatus* beta-glucosidase (SEQ ID NO: 2 of WO 2005/047499), *Aspergillus fumigatus* xylanase (Xyl III in WO 2006/078256), and Cel7A CBH I from *Aspergillus fumigatus* disclosed as SEQ ID NO: 2 in WO 2011/057140.

In another embodiment the cellulolytic enzyme preparation comprises a *Trichoderma* reesei cellulolytic preparation further comprising *Penicillium emersonii* AA9 (GH61A) polypeptide having cellulolytic enhancing activity disclosed in WO 2011/041397, *Aspergillus fumigatus* beta-glucosidase (SEQ ID NO: 2 of WO 2005/047499), *Aspergillus fumigatus* xylanase (Xyl III in WO 2006/078256), Cel7A CBH I from *Aspergillus fumigatus* disclosed as SEQ ID NO: 2 in WO 2011/057140, and CBH II derived from *Aspergillus fumigatus* disclosed in WO 2013/028928.

In another embodiment the cellulolytic enzyme preparation comprises a *Trichoderma* reesei cellulolytic preparation further comprising *Penicillium emersonii* AA9 (GH61A) polypeptide having cellulolytic enhancing activity disclosed in WO 2011/041397, *Aspergillus fumigatus* beta-glucosidase (SEQ ID NO: 2 of WO 2005/047499) or variant with the following substitutions: F100D, S283G, N456E, F512Y; *Aspergillus fumigatus* xylanase (Xyl III in WO 2006/078256), Cel7A CBH I from *Aspergillus fumigatus* disclosed as SEQ ID NO: 2 in WO 2011/057140, and CBH II derived from *Aspergillus fumigatus* disclosed in WO 2013/028928.

All cellulolytic enzyme preparations disclosed in WO 2013/028928 are also contemplated.

The cellulolytic enzyme preparation comprises or may further comprise one or more (several) proteins selected from the group consisting of a cellulase, an AA9 polypeptide having cellulolytic enhancing activity, a hemicellulase, an expansin, an esterase, a laccase, a ligninolytic enzyme, a pectinase, a peroxidase, a protease, and a swollenin.

In an embodiment the cellulolytic enzyme preparation is or comprises a commercial cellulolytic enzyme preparation.

Examples of commercial cellulolytic enzyme preparations suitable for use in the present invention include, for example, CELLIC® CTec (Novozymes A/S), CELLIC® CTec2 (Novozymes A/S), CELLIC® Ctec3 (Novozymes A/S), CELLUCLAST® (Novozymes A/S), CELLUZYME™ (Novozymes A/S), CEREFLO® (Novo Nordisk A/S), and ULTRAFLO® (Novozymes A/S), ACCELLERASE® (Danisco US Inc.), LAMINEX® (Danisco US Inc.), SPEZYME® CP (Danisco US Inc.), ROHAMENT® 7069 W (Röhm GmbH), FIBREZYME® LDI (Dyadic International, Inc.), FIBREZYME® LBR (Dyadic International, Inc.), or VISCOSTAR™ 150L (Dyadic International, Inc.).

The cellulolytic enzyme preparation may be present or added during hydrolysis (i.e., saccharification) in amounts effective from about 0.001 to about 5.0 wt % of solids (TS), more preferably from about 0.025 to about 4.0 wt % of solids, and most preferably from about 0.005 to about 2.0 wt % of solids (TS).

The present invention is further described by the following examples that should not be construed as limiting the scope of the invention.

The following are referred to in the examples:

<u>Mt Laccase:</u> Laccase derived from *Myceliophthora thermophila* disclosed in WO 95/33836 as SEQ ID NO: 2 (SEQ ID NO: 1 herein) and available from Novozymes A/S, Denmark in liquid form (MtL).

<u>Gr</u> Laccase: Laccase derived from *Myceliophthora thermophila* disclosed in WO 95/33836 as SEQ ID NO: 2 (SEQ ID NO: 1 herein) and available from Novozymes A/S, Denmark in granular form (GrL), comprising laccase in an amount of about 1%, in combination with inert ingredients and a laccase stabilizer.

<u>Beta-glucosidase preparation 188 ("BG188"):</u> *Aspergillus niger* enzyme preparation comprising about 57% glucoamylase, about 27% beta-glucosidase and about 16% alpha-amylase (protein content basis), exhibiting beta-glucosidase activity.

Cellulolytic Enzyme Preparation C3 ("C3"): Cellulolytic enzyme preparation derived from *Trichoderma reesei* further comprising AA9 (GH61A) polypeptide having cellulolytic enhancing activity derived from a strain of *Penicillium emersonii* (SEQ ID NO: 2 in WO 2011/041397, SEQ ID NO: 3 herein), *Aspergillus fumigatus* beta-glucosidase (SEQ ID NO: 2 in WO 2005/047499, SEQ ID NO: 2 herein) variant F100D, S283G, N456E, F512Y) disclosed in WO 2012/044915; *Aspergillus fumigatus* Cel7A CBH1 disclosed as SEQ ID NO: 6 in WO2011/057140 (SEQ ID NO: 4 herein) and *Aspergillus fumigatus* CBH II disclosed as SEQ ID NO: 18 in WO 2011/057140 (SEQ ID NO: 5 herein). Further, Cellulolytic Enzyme Preparation C3 further comprises about 10% Hemicellulolytic Enzyme Preparation H3.

Hemicellulolytic Enzyme Preparation H3 ("H3"): Cellulolytic enzyme preparation from *Trichoderma reesei,* further comprising *Aspergillus fumigatus* xylanase (WO 2006/078256, SEQ ID NO: 6 herein) and *Aspergillus fumigatus* beta-xylosidase (WO 2011/057140, SEQ ID NO: 7 herein).

Glucoamylase E ("GAE"): Glucoamylase blend comprising *Talaromyces emersonii* glucoamylase (SEQ ID NO: 34 in WO 99/28448 (SEQ ID NO: 8 herein)), *Trametes cingulata* glucoamylase (SEQ ID NO: 2 in WO 06/69289 (SEQ ID NO: 9 herein), and *Rhizomucorpusillus* alpha-amylase with *Aspergillus niger* glucoamylase linker and SBD (V039 in Table 5 in WO 2006/069290) (activity ratio in AGU:AGU:FAU-F is about 20:5: 1).

The invention described and claimed herein is not to be limited in scope by the specific aspects or embodiments herein disclosed, since such are intended as illustrations of several aspects or embodiments of the invention. Any equivalent aspects or embodiments are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

## EXAMPLES

## EXAMPLE 1

### ENZYMATIC PRECONDITIONING TO INCREASE GLUCOSE YIELD

Low severity dilute acid pretreated (3% $H_2SO_4$ (w/v), 160°C, for 5 minutes) unwashed dry fractionated corn fiber (uwPCF) was used. The pH of the pretreated uwPCF was adjusted with 50% NaOH to 5.2. The total solids were 17%.
The pH adjusted uwPCF was placed in 8 kettles (R1-R8). Each kettle containing pH adjusted uwPCF was brought to 50°C in water bath and the agitation speed was increased to 450rpm. All kettles were run at pH 5.0, 50°C and 450rpm for 3 hours. Enzymatic preconditioning (EPC) enzymes were added to all kettles except kettle R1, as set forth in Table 1 below, and the EPC process ran for 3 hours. Kettle R1 was run as a control at pH 5, 50°C, 450rpm for 6 hours without addition of EPC enzymes. Kettle R5 had EPC enzyme laccase added after 3 hours and 2 hours later the agitation speed was reduced to 250rpm and EPC enzyme beta-glucosidase (BG188) was added, versus adding the 2 EPC enzymes at the same time and co-incubating for the full 3 hours.
3 hours after addition of the EPC enzymes (or the first EPC enzyme in kettle R5), at a total of 6 tank hours, agitation was reduced on all kettles to 250rpm and Cellulolytic Enzyme Preparation C3 was added to all kettles R1-R8 to start hydrolysis.
24 hours after addition of Cellulolytic Enzyme Preparation C3 to each of kettles R1-R8 (30 hours tank time), Glucoamylase E (GAE) was added to each kettle. Hydrolysis was run for 6 days after addition of GAE. At the end of 6 days, 10gram aliquots were extracted to 30mL tubes and overdosed with Cellulolytic Enzyme Preparation C3 or GAE to evaluate the cellulose conversion holistically. The overdosed tubes were hydrolyzed for 24 hours.

**Table 1**: Enzyme & Dosing Scheme (mgEP/g-Cellulose unless otherwise indicated)

| Sample ID | Kettle No. | Preconditioning enzymes | | | | | Hydrolysis enzymes | |
|---|---|---|---|---|---|---|---|---|
| | | Mt Laccase | C3 | Gr Laccase | H3 | BG188 | C3 | GAE |
| PC550 | R1 | 0 | 0 | 0 | 0 | 0 | 2.00 | .0005% v/v |
| L-OR only | R2 | .015 | 0 | 0 | 0 | 0 | 2.00 | .0005% v/v |

(continued)

| Sample ID | Kettle No. | Preconditioning enzymes | | | | | Hydrolysis enzymes | |
|---|---|---|---|---|---|---|---|---|
| | | Mt Laccase | C3 | Gr Laccase | H3 | BG188 | C3 | GAE |
| L-OR+H3 | R3 | .015 | 0 | 0 | .25 | 0 | 1.75 | .0005% v/v |
| L-OR+BG188 | R4 | .015 | 0 | 0 | 0 | .20 | 1.80 | .0005% v/v |
| L-OR+BG188 (2) | R5 | .015 | 0 | 0 | 0 | .20 | 1.80 | .0005% v/v |
| S-OR only | R6 | 0 | 0 | .015 | 0 | 0 | 2.00 | .0005% v/v |
| S-OR+H3 | R7 | 0 | 0 | .015 | .25 | 0 | 1.75 | .0005% v/v |
| S-OR+C3 | R8 | 0 | .25 | .015 | 0 | 0 | 1.75 | .0005% v/v |

Hydrolysis samples were analyzed for glucose, xylose, cellobiose, xylitol, glycerol, acetic acid, and ethanol. Glucose was measured using an Agilent HPLC system with an analytical BIO-RAD Aminex HPX-87H column and a BIO-RAD Cation H refill guard column.

Time course of glucose content is illustrated in Figure 1. Compared with control (Kettle R1; PC550), EPC with laccase and beta-glucosidase (Kettle R4; L-OR+188) significantly improved enzymatic hydrolysis rate and final glucose concentration.

## EXAMPLE 2

### ENZYMATIC PRECONDITIONING WITH LACCASE AND BG188

Dilute acid pretreated unwashed dry fractionated corn fiber was obtained as described in Example 1. A volume of the pH adjusted uwPCF was added into a kettle reactor in order to reach a final TS of 10%. 2.5 ml of 1 g/L penicillin and make up water were added into the kettle reactor and mixed well.

Enzymatic preconditioning was initiated by addition of 0.015 mg EP/g cell of Mt Laccase and 0.2 mg EP/g cell of BG188 into the well mixed kettle reactor. Preconditioning was conducted at 50°C for 6 hours at a mixing speed of 250 rpm. As a control, the well mixed whole slurry was also preconditioned at 50°C for 6 hours at a mixing speed of 250 rpm, but without any enzyme addition. After preconditioning, the pH was checked and adjusted, if needed.

1.8 mg EP/g cell of C3 was added to initiate hydrolysis and, 24 hours after addition, 0.0005% (v/v) Glucoamylase E (GAE) was added. To the control reactor, 2 mg EP/g cell of C3 was added, followed 24 hours later by 0.01% (v/v) GAE. The total solids loading was 10% and hydrolysis time was 120 hours. Hydrolysis samples were analyzed using an Agilent HPLC system with an analytical BIO-RAD Aminex HPX-87H column. Total glucose (g/L) was analyzed at 72.9g/L in the experimental reactor and 66.5g/L in the control reactor.

## EXAMPLE 3

### ENZYMATIC PRECONDITIONING WITH LACCASE AND C3

Dilute acid pretreated unwashed dry fractionated corn fiber was obtained as described in Example 1. A volume of the pH adjusted uwPCF was added into a kettle reactor in order to reach a final TS of 17%. 2.5 ml of 1 g/L penicillin and make up water were added into the kettle reactor and mixed well.

Enzymatic preconditioning was initiated by addition of 0.015 mg EP/g cell of granular laccase (GrL) and 0.25 mg EP/g cell C3 into the well mixed kettle reactor. Preconditioning was conducted at 50°C for 6 hours at a mixing speed of 250 rpm. As a control, the well mixed whole slurry was also preconditioned at 50°C for 6 hours at a mixing speed of 250 rpm, but without any enzyme addition. After preconditioning, the pH was checked and adjusted, if needed.

1.75 mg EP/g cell of C3 was added to initiate hydrolysis and, 24 hours after addition, 0.0005% (v/v) Glucoamylase E (GAE) was added. To the control reactor, 2 mg EP/g cell of C3 was added, followed 24 hours later by 0.01% (v/v) GAE. The total solids loading was 20% and hydrolysis time was 120 hours. Hydrolysis samples were analyzed using an Agilent HPLC system with an analytical BIO-RAD Aminex HPX-87H column. Total glucose (g/L) was analyzed at 71.0 g/L in the experimental reactor and 66.5 g/L in the control reactor.

## EXAMPLE 4

### ENZYMATIC PRECONDITIONING WITH LACCASE AND GA

Dilute acid pretreated unwashed dry fractionated corn fiber was obtained as described in Example 1. A volume of the pH adjusted uwPCF was added into a kettle reactor in order to reach a final TS of 17%. 2.5 ml of 1 g/L penicillin and make up water were added into the kettle reactor and mixed well.

Enzymatic preconditioning was initiated by addition of 0.015 mg EP/g cell of Mt Laccase and 0.0005% (v/v) of Glucoamylase E (GAE) into the well mixed kettle reactor. Preconditioning was conducted at 50°C for 6 hours at a mixing speed of 250 rpm. As a control, the well mixed whole slurry was also preconditioned at 50°C for 6 hours at a mixing speed of 250 rpm, but without any enzyme addition. After preconditioning, the pH was checked and adjusted, if needed.

2.0 mg EP/g cell of C3 was added to initiate hydrolysis. To the control reactor, 2 mg EP/g cell of C3 was added, followed 24 hours later by 0.01% (v/v) GAE. The total solids loading was 17.3% and hydrolysis time was 96 hours. Hydrolysis samples were analyzed using an Agilent HPLC system with an analytical BIO-RAD Aminex HPX-87H column. Total glucose (g/L) was analyzed at 64.3g/L in the experimental reactor and 63.9g/L in the control reactor.

## EXAMPLE 5

### ENZYMATIC PRECONDITIONING WITH LACCASE

Low severity dilute acid pretreated (3% $H_2SO_4$ (w/v), 160°C, for 5 minutes) unwashed dry fractionated corn fiber (uwPCF) was used. The pH of the pretreated uwPCF was adjusted with 50% NaOH to 5.2. The total solids were measured as 10%.

The pH adjusted uwPCF was liquated to rotisserie tube reactors and 0.1 ml of 1 g/L penicillin and make up water were added into the reactors and mixed well. Enzymes were added to the tubes, for evaluation as preconditioners, as follows:

5ug Mt Laccase/g cel

10ug Mt Laccase/g cel

15ug Mt Laccase/g cel

25ug Mt Laccase/g cel

15ug Gr Laccase/g cel

A control tube (PC550) of liquated substrate only, with no laccase added, served as a control. All tubes were preconditioned, with or without enzymes, for 16 hours in the rotisserie method.

16 hours after addition of the enzymes as set forth above, Cellulolytic Enzyme Preparation C3 (2 mgEP/g-cel) was added to the enzyme tubes to start hydrolysis. No C3 was added to the PC550 control.

24 hours after addition C3 to each of the enzyme tubes, 0.005% (v/v) of glucoamylase (Glucoamylase E) was added (0.01 % w/v) to each tube, including the PC550 control. Hydrolysis continued to run after addition of GAE. The total process time was 6 days.

Hydrolysis samples were analyzed for glucose, xylose, cellobiose, xylitol, glycerol, acetic acid, and ethanol. Glucose was measured using an Agilent HPLC system with an analytical BIO-RAD Aminex HPX-87H column and a BIO-RAD Cation H refill guard column. Results at 3 days and 6 days are provided in Figure 2. The EPC with all laccases is seen to outperform the control GA only.

## Claims

1. A process for producing a hydrolysis product from pretreated lignocellulose-containing material, the process comprising:

   preconditioning a pretreated lignocellulose-containing material, the preconditioning comprising incubating the pretreated lignocellulose-containing material with laccase and beta-glucosidase; and
   hydrolyzing the preconditioned lignocellulose-containing material with a cellulolytic enzyme composition to obtain a hydrolysis product, wherein preconditioning and hydrolysis are carried out sequentially.

2. The process of claim 1, wherein the pretreated lignocellulose-containing material is dilute acid pretreated corn fiber.

3. The process of claim 2, wherein the corn fiber is pretreated dry fractionated corn fiber.

4. The process of any of claims 2 or 3, wherein the corn fiber is unwashed.

5. The process of any of claims 1-4, wherein the beta-glucosidase is comprised in an enzyme preparation.

6. The process of claim 5, wherein the enzyme preparation further comprises glucoamylase and alpha-amylase.

7. The process of any of claims 1-6, wherein the hydrolyzing further comprises addition of a glucoamylase enzyme.

8. The process of any of claims 1-7, wherein the hydrolysis is performed for at least 5 days.

9. A process for producing a fermentation product from pretreated lignocellulose-containing material, the process comprising:

   preconditioning a pretreated lignocellulose-containing material, the preconditioning comprising incubating the pretreated lignocellulose-containing material with laccase and beta-glucosidase;
   hydrolyzing the preconditioned lignocellulose-containing material with a cellulolytic enzyme composition to obtain a hydrolysis product; and
   fermenting the hydrolysis product to obtain a fermentation product, wherein preconditioning and hydrolysis are carried out sequentially.

10. The process of claim 9, wherein the pretreated lignocellulose-containing material is dilute acid pretreated corn fiber.

11. The process of claim 10, wherein the corn fiber is pretreated dry fractionated corn fiber.

12. The process of any of claims 10 or 11, wherein the corn fiber is unwashed.

13. The process of any of claims 9-12, wherein the beta-glucosidase is comprised in an enzyme preparation.

14. The process of claim 13, wherein the enzyme preparation further comprises glucoamylase and alpha-amylase.

15. The process of any of claims 9-14, wherein the fermentation product is an alcohol, preferably ethanol or butanol.

## Patentansprüche

1. Verfahren zum Herstellen eines Hydolyseprodukts aus vorbehandeltem lignocellulosehaltigen Material, wobei das Verfahren umfasst:

   Vorkonditionieren eines vorbehandelten lignocellulosehaltigen Materials, wobei das Vorkonditionieren Inkubieren des vorbehandelten lignocellulosehaltigen Materials mit Laccase und beta-Glucosidase umfasst; und
   Hydolysieren des vorbehandelten lignocellulosehaltigen Materials mit einer cellulolytischen Enzymzusammensetzung, um ein Hydrolyseprodukt zu erhalten, wobei Vorkonditionierung und Hydrolyse der Reihe nach ausgeführt werden.

**2.** Verfahren nach Anspruch 1, wobei das vorbehandelte lignocellulosehaltige Material mit verdünnter Säure vorbehandelte Maisfaser ist.

**3.** Verfahren nach Anspruch 2, wobei die Maisfaser vorbehandelte trockenfraktionierte Maisfaser ist.

**4.** Verfahren nach einem beliebigen der Ansprüche 2 oder 3, wobei die Maisfaser ungewaschen ist.

**5.** Verfahren nach einem beliebigen der Ansprüche 1-4, wobei die beta-Glucosidase in einer Enzymzubereitung umfasst ist.

**6.** Verfahren nach Anspruch 5, wobei die Enzymzubereitung weiterhin Glucoamylase und alpha-Amylase umfasst.

**7.** Verfahren nach einem beliebigen der Ansprüche 1-6, wobei das Hydrolysieren weiterhin die Zugabe eines Glucoamylaseenzyms umfasst.

**8.** Verfahren nach einem beliebigen der Ansprüche 1-7, wobei die Hydrolyse für mindestens 5 Tage durchgeführt wird.

**9.** Verfahren zum Herstellen eines Fermentationsprodukts aus vorbehandeltem lignocellulosehaltigem Material, wobei das Verfahren umfasst:

Vorkonditionieren eines vorbehandelten lignocellulosehaltigen Materials, wobei das Vorkonditionieren Inkubieren des vorbehandelten lignocellulosehaltigen Materials mit Laccase und beta-Glucosidase umfasst;
Hydolysieren des vorbehandelten lignocellulosehaltigen Materials mit einer cellulolytischen Enzymzusammensetzung, um ein Hydrolyseprodukt zu erhalten; und
Fermentieren des Hydrolyseprodukts, um ein Fermentationsprodukt zu erhalten, wobei Vorkonditionieren und Hydrolyse der Reihe nach ausgeführt werden.

**10.** Verfahren nach Anspruch 9, wobei das vorbehandelte lignocellulosehaltige Material mit verdünnter Säure vorbehandelte Maisfaser ist.

**11.** Verfahren nach Anspruch 10, wobei die Maisfaser vorbehandelte trockenfraktionierte Maisfaser ist.

**12.** Verfahren nach einem beliebigen der Ansprüche 10 oder 11, wobei die Maisfaser ungewaschen ist.

**13.** Verfahren nach einem beliebigen der Ansprüche 9-12, wobei die beta-Glucosidase in einer Enzymzubereitung umfasst ist.

**14.** Verfahren nach Anspruch 13, wobei die Enzymzubereitung weiterhin Glucoamylase und alpha-Amylase umfasst.

**15.** Verfahren nach einem beliebigen der Ansprüche 9-14, wobei das Fermentationsprodukt ein Alkohol, bevorzugt Ethanol oder Butanol ist.

## Revendications

**1.** Procédé de production d'un produit d'hydrolyse à partir d'une matière contenant de la lignocellulose prétraitée, le procédé comprenant :

le préconditionnement d'une matière contenant de la lignocellulose prétraitée, le préconditionnement comprenant l'incubation de la matière contenant de la lignocellulose prétraitée avec une laccase et une bêta-glucosidase ; et
l'hydrolyse de la matière contenant de la lignocellulose préconditionnée avec une composition d'enzyme cellulolytique pour obtenir un produit d'hydrolyse, dans laquelle le préconditionnement et l'hydrolyse sont effectués séquentiellement.

**2.** Procédé selon la revendication 1, dans lequel la matière contenant de la lignocellulose prétraitée est une fibre de maïs prétraitée à l'acide dilué.

3. Procédé selon la revendication 2, dans lequel la fibre de maïs est de la fibre de maïs fractionnée sèche prétraitée.

4. Procédé selon l'une quelconque des revendications 2 ou 3, dans lequel la fibre de maïs est non lavée.

5. Procédé selon l'une quelconque des revendications 1 à 4, dans lequel la bêta-glucosidase est comprise dans une préparation enzymatique.

6. Procédé selon la revendication 5, dans lequel la préparation enzymatique comprend en outre une glucoamylase et une alpha-amylase.

7. Procédé selon l'une quelconque des revendications 1 à 6, dans lequel l'hydrolyse comprend en outre l'ajout d'une enzyme glucoamylase.

8. Procédé selon l'une quelconque des revendications 1 à 7, dans lequel l'hydrolyse est effectuée pendant au moins 5 jours.

9. Procédé de production d'un produit de fermentation à partir d'une matière contenant de la lignocellulose prétraitée, le procédé comprenant :

   le préconditionnement d'une matière contenant de la lignocellulose prétraitée, le préconditionnement comprenant l'incubation de la matière contenant de la lignocellulose prétraitée avec une laccase et une bêta-glucosidase ;
   l'hydrolyse de la matière contenant de la lignocellulose préconditionnée avec une composition d'enzyme cellulolytique pour obtenir un produit d'hydrolyse ; et
   la fermentation du produit d'hydrolyse pour obtenir un produit de fermentation, dans laquelle le préconditionnement et l'hydrolyse sont effectués séquentiellement.

10. Procédé selon la revendication 9, dans lequel la matière contenant de la lignocellulose prétraitée est une fibre de maïs prétraitée à l'acide dilué.

11. Procédé selon la revendication 10, dans lequel la fibre de maïs est de la fibre de maïs sèche fractionnée prétraitée.

12. Procédé selon l'une quelconque des revendications 10 ou 11, dans lequel la fibre de maïs est non lavée.

13. Procédé selon l'une quelconque des revendications 9 à 12, dans lequel la bêta-glucosidase est comprise dans une préparation enzymatique.

14. Procédé selon la revendication 13, dans lequel la préparation enzymatique comprend en outre une glucoamylase et une alpha-amylase.

15. Procédé selon l'une quelconque des revendications 9 à 14, dans lequel le produit de fermentation est un alcool, de préférence l'éthanol ou le butanol.

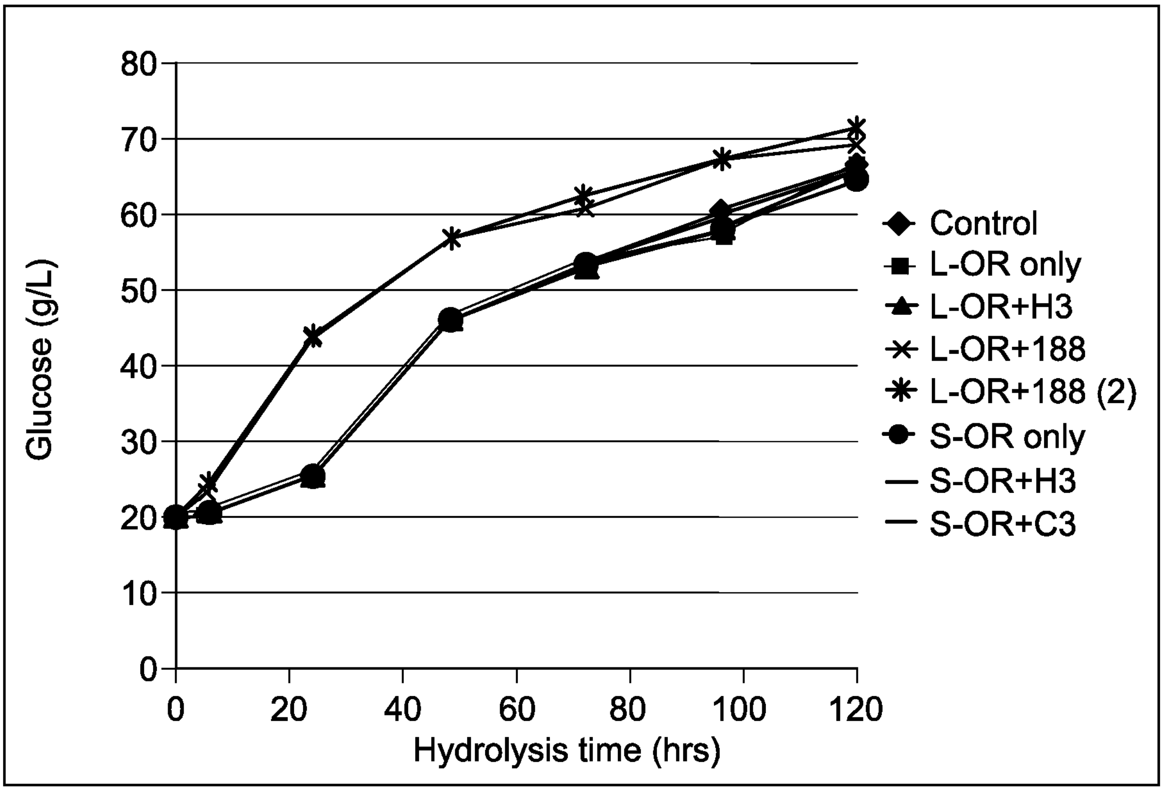

80
70
60
50
40
30
20
10
0
Glucose (g/L)
0
20
40
60
80
100
120
Hydrolysis time (hrs)
Control
L-OR only
L-OR+H3
L-OR+188
L-OR+188 (2)
S-OR only
S-OR+H3
S-OR+C3


FIG. 1

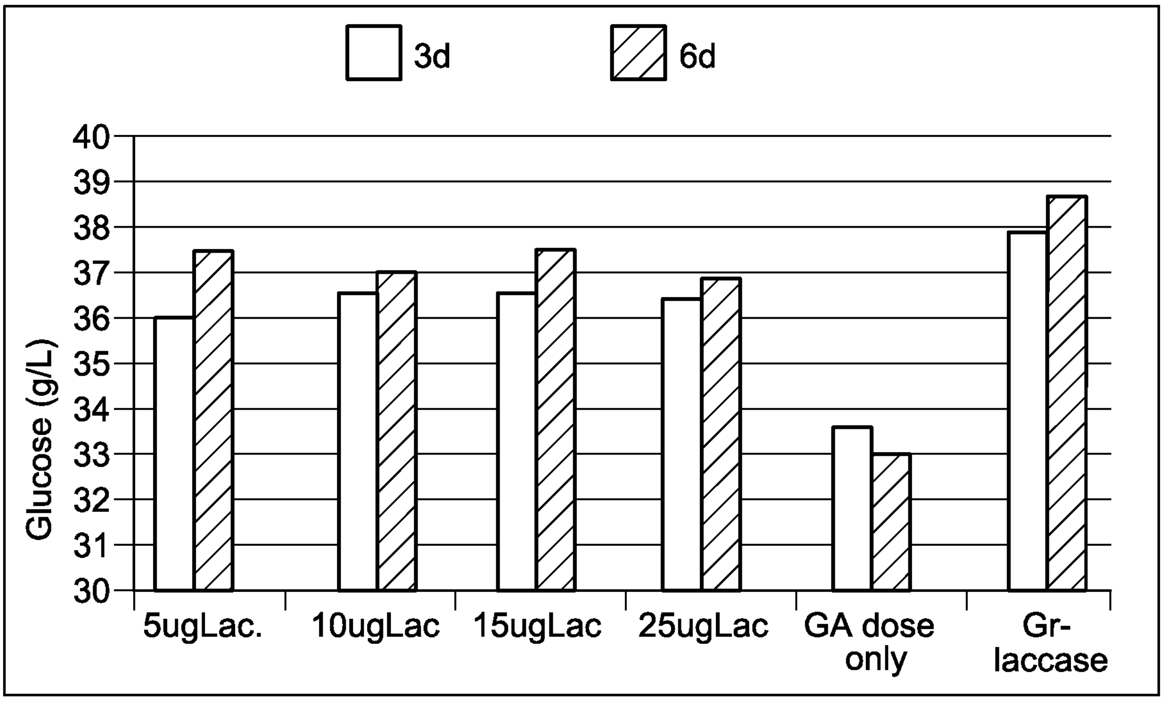

3d
6d
40
39
38
37
36
35
34
33
32
31
30
Glucose (g/L)
5ugLac.
10ugLac
15ugLac
25ugLac
GA dose only
Gr-laccase


FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008134259 A
- WO 02095014 A
- WO 2013028928 A
- US 20020164730 A
- WO 2006110891 A
- WO 2006110899 A
- WO 2006110900 A
- WO 2006110901 A
- WO 2006032282 A
- WO 9533836 A
- WO 2005047499 A
- WO 2012044915 A
- WO 2005074656 A
- WO 2011041397 A
- WO 94021785 A
- WO 2006078256 A
- WO 2011057140 A
- WO 9117243 A
- WO 9117244 A
- WO 9105039 A
- WO 9315186 A
- US 5275944 A
- WO 9602551 A
- US 5536655 A
- WO 0070031 A
- WO 05093050 A
- WO 2011059740 A
- WO 2009042871 A
- WO 2010141325 A
- WO 2006074435 A
- WO 2010057086 A
- WO 2007019442 A
- WO 2010088387 A
- WO 2011035029 A
- WO 2005074647 A
- WO 2008148131 A
- WO 2011035027 A
- WO 2010065830 A
- WO 2007089290 A
- WO 2009085935 A
- WO 2009085859 A
- WO 2009085864 A
- WO 2009085868 A
- WO 2010138754 A
- WO 2011005867 A
- WO 2011039319 A
- WO 2011041504 A
- WO 2012125925 A
- WO 2012113340 A
- WO 12129699 A
- WO 2012130964 A
- WO 2012122477 A
- WO 2012135659 A
- WO 2012146171 A
- WO 2012101206 A
- WO 2008151043 A
- WO 2012021394 A
- WO 2012021395 A
- WO 2012021396 A
- WO 2012021399 A
- WO 2012021400 A
- WO 2012021401 A
- WO 2012021408 A
- WO 2012021410 A
- WO 9421785 A
- WO 2011041405 A
- WO 2010126772 A
- WO 2012130965 A
- WO 201213095 A
- WO 2009079210 A
- WO 2011057083 A
- WO 2010108918 A
- WO 2009073709 A
- WO 2005001036 A
- WO 2010014880 A
- WO 2009042846 A
- WO 2009076122 A
- WO 2009127729 A
- WO 2010053838 A
- WO 2010065448 A
- WO 2006114094 A
- WO 2009073383 A
- WO 2010014706 A
- WO 2009068565 A

- WO 03062430 A
- JP 2238885 A
- WO 9201046 A
- WO 9600290 A
- WO 9200381 A
- WO 0004136 A
- WO 0104273 A
- WO 8402921 A
- US 4727026 A
- WO 9928448 A
- US RE32153 E
- US 4587215 A
- EP 135138 A
- WO 8601831 A
- WO 2006069289 A
- US 2007066618 W
- WO 2005045018 A
- WO 0669289 A
- WO 2006069290 A
- WO 9623874 A
- WO 8901969 A
- WO 2004055178 A
- WO 2005003311 A
- US 7883883 B
- WO 2002095014 A
- WO 2008057637 A
- US 61824082 B

**Non-patent literature cited in the description**

- **VENTURI et al.** Extracellular beta-D-glucosidase from Chaetomium thermophilum var. coprophilum: production, purification and some biochemical properties. *J. Basic Microbiol.,* 2002, vol. 42, 55-66
- **TEERI.** Crystalline cellulose degradation: New insight into the function of cellobiohydrolases. *Trends in Biotechnology,* 1997, vol. 15, 160-167
- **TEERI et al.** Trichoderma reesei cellobiohydrolases: why so efficient on crystalline cellulose?. *Biochem. Soc. Trans.,* 1998, vol. 26, 173-178
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **TOMME et al.** *Eur. J. Biochem.,* 1988, vol. 170, 575-581
- **ZHANG et al.** Outlook for cellulase improvement: Screening and selection strategies. *Biotechnology Advances,* 2006, vol. 24, 452-481
- **GHOSE.** Measurement of cellulase activities. *Pure Appl. Chem.,* 1987, vol. 59, 257-68
- **SHALLOM ; SHOHAM.** *Current Opinion In Microbiology,* 2003, vol. 6 (3), 219-228
- **GHOSE ; BISARIA.** *Pure & Appl. Chem.,* 1987, vol. 59, 1739-1752
- **NIELSEN et al.** *Protein Engineering,* 1997, vol. 10, 1-6
- **QUINLAN et al.** *Proc. Natl. Acad. Sci. USA,* 2011, vol. 208, 15079-15084
- **PHILLIPS et al.** *ACS Chem. Biol.,* 2011, vol. 6, 1399-1406
- **LIN.** *Structure,* 2012, vol. 20, 1051-1061
- **HENRISSAT.** *Biochem. J.,* 1991, vol. 280, 309-316
- **HENRISSAT ; BAIROCH.** *Biochem. J.,* 1996, vol. 316, AA9
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **WISELOGEL et al.** Handbook on Bioethanol. Taylor & Francis, 1995, 105-118
- **WYMAN.** *Bioresource Technology,* 1994, vol. 50, 3-16
- **LYND.** *Applied Biochemistry and Biotechnology,* 1990, vol. 24/25, 695-719
- Recent Progress in Bioconversion of Lignocellulosics. **MOSIER et al.** Advances in Biochemical Engineering/Biotechnology. Springer-Verlag, 1999, vol. 65, 23-40
- **CHANDRA et al.** *Adv. Biochem. Engin./Biotechnol.,* 2007, vol. 108, 67-93
- **GALBE ; ZACCHI.** *Adv. Biochem. Engin./Biotechnol.,* 2007, vol. 108, 41-65
- **HENDRIKS ; ZEEMAN.** *Bioresource Technology,* 2009, vol. 100, 10-18
- **MOSIER et al.** *Bioresource Technology,* 2005, vol. 96, 673-686
- **TAHERZADEH ; KARIMI.** *Int. J. Mol. Sci.,* 2008, vol. 9, 1621-1651
- **YANG ; WYMAN.** *Biofuels Bioproducts and Biorefining-Biofpr.,* 2008, vol. 2, 26-40
- **DUFF ; MURRAY.** *Bioresource Technology,* 1996, vol. 855, 1-33
- **GALBE ; ZACCHI.** *Appl. Microbiol. Biotechnol.,* 2002, vol. 59, 618-628
- **BALLESTEROS et al.** *Appl. Biochem. Biotechnol.,* 2006, vol. 129-132, 496-508
- **VARGA et al.** *Appl. Biochem. Biotechnol.,* 2004, vol. 113-116, 509-523
- **SASSNER et al.** *Enzyme Microb. Technol.,* 2006, vol. 39, 756-762
- **SCHELL et al.** *Bioresource Technology,* 2004, vol. 91, 179-188

- **LEE et al.** *Adv. Biochem. Eng. Biotechnol.,* 1999, vol. 65, 93-115
- **WYMAN et al.** *Bioresource Technology,* 2005, vol. 96, 1959-1966
- **SCHMIDT ; THOMSEN.** *Bioresource Technology,* 1998, vol. 64, 139-151
- **PALONEN et al.** *Appl. Biochem. Biotechnol.,* 2004, vol. 117, 1-17
- **VARGA et al.** *Biotechnol. Bioeng.,* 2004, vol. 88, 567-574
- **MARTIN et al.** *J. Chem. Technol. Biotechnol.,* 2006, vol. 81, 1669-1677
- **GOLLAPALLI et al.** *Appl. Biochem. Biotechnol.,* 2002, vol. 98, 23-35
- **CHUNDAWAT et al.** *Biotechnol. Bioeng.,* 2007, vol. 96, 219-231
- **ALIZADEH et al.** *Appl. Biochem. Biotechnol.,* 2005, vol. 121, 1133-1141
- **TEYMOURI et al.** *Bioresource Technology,* 2005, vol. 96, 2014-2018
- **PAN.** *Biotechnol. Bioeng.,* 2005, vol. 90, 473-481
- **PAN et al.** *Biotechnol. Bioeng.,* 2006, vol. 94, 851-861
- **KURABI et al.** *Appl. Biochem. Biotechnol.,* 2005, vol. 121, 219-230
- **SCHELL et al.** *Appl. Biochem. Biotechnol.,* 2003, vol. 105-108, 69-85
- Pretreatment of biomass. **HSU, T.-A.** Handbook on Bioethanol: Production and Utilization. Taylor & Francis, 1996, 179-212
- **GHOSH ; SINGH.** *Adv. Appl. Microbiol.,* 1993, vol. 39, 295-333
- Pretreating lignocellulosic biomass: a review. **MCMILLAN, J. D.** Enzymatic Conversion of Biomass for Fuels Production. American Chemical Society, 1994
- Ethanol production from renewable resources. **GONG, C. S. ; CAO, N. J. ; DU, J. ; TSAO, G. T.** Advances in Biochemical Engineering/Biotechnology. Springer-Verlag Berlin Heidelberg, 1999, vol. 65, 207-241
- **OLSSON ; HAHN-HAGERDAL.** *Enz. Microb. Tech.,* 1996, vol. 18, 312-331
- **VALLANDER ; ERIKSSON.** *Adv. Biochem. Eng./Biotechnol.,* 1990, vol. 42, 63-95
- **LUO et al.** *Biomass and Bioenergy,* 2002, vol. 22, 125-138
- **SAARILAHTI et al.** *Gene,* 1990, vol. 90, 9-14
- **PENTTILA et al.** *Gene,* 1986, vol. 45, 253-263
- **SALOHEIMO et al.** *Gene,* 1988, vol. 63, 11-22
- **OKADA et al.** *Appl. Environ. Microbiol.,* 1988, vol. 64, 555-563
- **SALOHEIMO et al.** *Molecular Microbiology,* 1994, vol. 13, 219-228
- **OOI et al.** *Nucleic Acids Research,* 1990, vol. 18, 5884
- **SAKAMOTO et al.** *Current Genetics,* 1995, vol. 27, 435-439
- **KAWAGUCHI et al.** *Gene,* 1996, vol. 173, 287-288
- **DAN et al.** *J. Biol. Chem.,* 2000, vol. 275, 4973-4980
- **HENRISSAT ; BAIROCH.** *Biochem. J.,* 1996, vol. 316, 695-696
- More Gene Manipulations in Fungi. Academic Press, 1991
- **BAILEY, J.E. ; OLLIS, D.F.** Biochemical Engineering Fundamentals. McGraw-Hill Book Company, 1986
- Cellulose bioconversion technology. **PHILIPPIDIS, G. P.** Handbook on Bioethanol: Production and Utilization. Taylor & Francis, 1996, 179-212
- **SHEEHAN, J. ; HIMMEL, M.** Enzymes, energy and the environment: A strategic perspective on the U.S. Department of Energy's research and development activities for bioethanol. *Biotechnol. Prog.,* 1999, vol. 15, 817-827
- **LYND et al.** Microbial cellulose utilization: Fundamentals and biotechnology. *Microbiol. Mol. Biol. Reviews,* 2002, vol. 66, 506-577
- **LIN et al.** *Appl. Microbiol. Biotechnol.,* 2006, vol. 69, 627-642
- **CHEN ; HO.** *Appl. Biochem. Biotechnol.,* 1993, vol. 39-40, 135-147
- **HO et al.** *Appl. Environ. Microbiol.,* 1998, vol. 64, 1852-1859
- **KOTTER ; CIRIACY.** *Appl. Microbiol. Biotechnol.,* 1993, vol. 38, 776-783
- **WALFRIDSSON et al.** *Appl. Environ. Microbiol.,* 1995, vol. 61, 4184-4190
- **KUYPER et al.** *FEMS Yeast Research,* 2004, vol. 4, 655-664
- **BEALL et al.** *Biotech. Bioeng.,* 1991, vol. 38, 296-303
- **INGRAM et al.** *Biotechnol. Bioeng.,* 1998, vol. 58, 204-214
- **ZHANG et al.** *Science,* 1995, vol. 267, 240-243
- **DEANDA et al.** *Appl. Environ. Microbiol.,* 1996, vol. 62, 4465-4470
- The Alcohol Textbook. Nottingham University Press, 1999
- **ALFENORE et al.** Improving ethanol production and viability of Saccharomyces cerevisiae by a vitamin feeding strategy during fed-batch process. Springer-Verlag, 2002
- Ethanol production from renewable resources. **GONG et al.** Advances in Biochemical Engineering/Biotechnology. Springer-Verlag Berlin Heidelberg, 1999, vol. 65, 207-241
- **SILVEIRA ; JONAS.** *Appl. Microbiol. Biotechnol.,* 2002, vol. 59, 400-408
- **NIGAM ; SINGH.** *Process Biochemistry,* 1995, vol. 30 (2), 117-124

- **EZEJI et al.** *World Journal of Microbiology and Biotechnology,* 2003, vol. 19 (6), 595-603
- **RICHARD ; MARGARITIS.** *Biotechnology and Bioengineering,* 2004, vol. 87 (4), 501-515
- **KATAOKA et al.** *Water Science and Technology,* 1997, vol. 36 (6-7), 41-47
- **GUNASEELAN.** *Biomass and Bioenergy,* 1997, vol. 13 (1-2), 83-114
- **CHEN ; LEE.** *Appl. Biochem. Biotechnol.,* 1997, vol. 63-65, 435-448
- **CHOI et al.** *Mol. Plant-Microbe Interactions,* 1992, vol. 5, 119-128
- **KOJIMA et al.** *J. Biol. Chem.,* 1990, vol. 265, 15224-15230
- **GERMANN ; LERCH.** *Experientia,* 1985, vol. 41, 801
- *PNAS USA,* 1986, vol. 83, 8854-8858
- **SALOHEIMO et al.** *J. Gen. Microbiol.,* 1985, vol. 137, 1537-1544
- **YOSHIDA.** Chemistry of Lacquer (Urushi). *J. Chem. Soc.,* vol. 43, 472-486
- **JÖNSSON et al.** *Appl. Microbiol. Biotechnol.,* 1998, vol. 49, 691-697
- **MURALIKRISHNA et al.** *Appl. Environ. Microbiol.,* 1995, vol. 61 (12), 4374-4377
- *Abstract of Papers American Chemical Society,* 1995, vol. 209 (1-2
- **SCHNEIDER et al.** *Enzyme and Microbial Technology,* 1999, vol. 25, 502-508
- **WALEITHNER et al.** *Curr. Genet.,* 1996, vol. 29, 395-403
- **KIISKINEN et al.** *Microbiology,* 2004, vol. 150, 3065-3074
- **MACHCZYNSKI et al.** *Protein Science,* 2004, vol. 13, 2388-2397
- **BOEL et al.** *EMBO J.,* 1984, vol. 3 (5), 1097-1102
- **HATA et al.** *Agric. Biol. Chem.,* 1991, vol. 55 (4), 941-949
- **CHEN et al.** *Prot. Eng.,* 1996, vol. 9, 499-505
- **CHEN et al.** *Prot. Eng.,* 1995, vol. 8, 575-582
- **CHEN et al.** *Biochem. J.,* 1994, vol. 301, 275-281
- **FIEROBE et al.** *Biochemistry,* 1996, vol. 35, 8698-8704
- **LI et al.** *Protein Eng.,* 1997, vol. 10, 1199-1204
- **NAGASAKA et al.** *Appl. Microbiol. Biotechnol.,* 1998, vol. 50, 323-330
- **KANEKO et al.** Molecular-cloning and determination of the nucleotide-sequence of a gene encoding an acid-stable alpha-amylase from Aspergillus kawachii. *J. Ferment. Bioeng.,* 1996, vol. 81, 292-298
- **SHALLOM ; SHOHAM.** Microbial hemicellulases. *Current Opinion In Microbiology,* 2003, vol. 6 (3), 219-228
- **ZHANG et al.** *Biotechnology Advances,* 2006, vol. 24, 452-481
- **GHOSE.** *Pure and Appl. Chem.,* 1987, vol. 59, 257-268